(19) Europäisches Patentamt
European Patent Office
Office européen des brevets

(11) EP 3 449 013 B1

(12) EUROPEAN PATENT SPECIFICATION

(45) Date of publication and mention of the grant of the patent:
**09.06.2021 Bulletin 2021/23**

(21) Application number: **17723533.0**

(22) Date of filing: **19.04.2017**

(51) Int Cl.:
***C12Q 1/6816*** *(2018.01)* ***G01N 33/543*** *(2006.01)*

(86) International application number:
**PCT/JP2017/015755**

(87) International publication number:
**WO 2017/188095 (02.11.2017 Gazette 2017/44)**

(54) **TESTING DEVICE AND METHOD FOR PRODUCING SAME, TESTING METHOD, AND TESTING KIT AND TRANSFER MEDIUM FOR PRODUCING TESTING DEVICE**

PRÜFVORRICHTUNG UND VERFAHREN ZUR HERSTELLUNG DAVON, PRÜFVERFAHREN UND PRÜFKIT UND ÜBERTRAGUNGSMEDIUM ZUR HERSTELLUNG EINER PRÜFVORRICHTUNG

DISPOSITIF D’ESSAI ET SON PROCÉDÉ DE FABRICATION, PROCÉDÉ D’ESSAI ET KIT D’ESSAI ET SUPPORT DE TRANSFERT POUR FABRIQUER LE DISPOSITIF D’ESSAI

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.04.2016 JP 2016087281**
**25.04.2016 JP 2016087285**
**05.09.2016 JP 2016172718**
**26.10.2016 JP 2016209720**

(43) Date of publication of application:
**06.03.2019 Bulletin 2019/10**

(73) Proprietor: **Ricoh Company, Ltd.**
**Tokyo 143-8555 (JP)**

(72) Inventors:
- **YAMOTO, Rie**
**Tokyo 143-8555 (JP)**
- **KOBAYASHI, Rie**
**Tokyo 143-8555 (JP)**

(74) Representative: **Fairbairn, Angus Chisholm et al**
**Marks & Clerk LLP**
**15 Fetter Lane**
**London EC4A 1BW (GB)**

(56) References cited:
**WO-A1-03/025573**
**WO-A1-2014/171891**
**WO-A1-2015/041373**
**WO-A1-2015/129924**
**JP-A- 2001 157 598**

- **MUHAMMAD SAJID ET AL: "Designs, formats and applications of lateral flow assay: A literature review", JOURNAL OF SAUDI CHEMICAL SOCIETY, vol. 19, no. 6, 1 November 2015 (2015-11-01), pages 689-705, XP55323878, AMSTERDAM, NL ISSN: 1319-6103, DOI: 10.1016/j.jscs.2014.09.001**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 449 013 B1

## Description

[Technical Field]

The present invention relates to a testing device and a method for producing the same, a testing method, and a testing kit and a transfer medium for producing a testing device.

[Background Art]

Hitherto, testing devices in which flow paths for flowing analytes are formed have been used in order to test analytes such as blood, DNAs, foods, and beverages.
Among the testing devices, lateral flow chromatographic devices for nucleic acid detection capable of detecting target nucleic acids can accurately diagnose, for example, infectious diseases, hereditary diseases such as tumors, and predispositions by checking presence or absence of nucleic acids (DNAs and RNAs) attributable to viruses or bacteria or presence or absence of nucleic acids attributable to mutant genes related to specific diseases or predispositions. Lateral flow chromatographic devices for nucleic acid detection are also used for food inspections for detecting allergens or specific foods and environmental surveys for detecting microorganisms existing in the environment.
For example, there are proposed lateral flow chromatographic devices for nucleic acid detection capable of detecting a target nucleic acid using: a detecting portion coated with a capture nucleic acid including a sequence complementary with the target nucleic acid; and a label body bindable with the target nucleic acid (see, e.g., PTLs 1 and 2).

[Citation List]

[Patent Literature]

[PTL 1]
Japanese Unexamined Patent Application Publication No. 2001-157598
[PTL 2]
Japanese Translation of PCT International Application Publication No. JP-T-2005-503556
[PTL 3]
WO2015/129924 discloses a testing device with a resin layer with antibodies provided over the flow path member.


[Summary of Invention]

[Technical Problem]

The present invention has an object to provide a target nucleic acid testing device capable of performing a measurement at a high sensitivity and obtaining clear judgement lines.

[Solution to Problem]

The invention is defined by the scope of the appended claims.
According to one aspect of the present invention, a testing device includes a porous flow path member constituting a flow path through which a testing target liquid is flowed, a testing target liquid dropping portion provided on the flow path member, a labeling portion configured to apply a label to a target nucleic acid when the target nucleic acid is contained in the testing target liquid dropped onto the testing target liquid dropping portion, and a detecting portion configured to detect the target nucleic acid labeled at the labeling portion. The testing device includes a shaped body on the flow path member at the detecting portion. A capture nucleic acid including a sequence bindable and complementary with the target nucleic acid is immobilized by covalent binding to a surface of the shaped body between the shaped body and the flow path member.

[Advantageous Effects of Invention]

The present invention can provide a target nucleic acid testing device capable of performing a measurement at a high sensitivity and obtaining clear judgement lines.

[Brief Description of Drawings]

[FIG. 1]
FIG. 1 is a top view illustrating an example of a testing device of the present invention.
[FIG. 2]
FIG. 2 is a schematic cross-sectional view of the testing device of FIG. 1 taken along a line A-A.
[FIG. 3]
FIG. 3 is a partially enlarged cross-sectional view depicting a part at which a first detecting portion and a flow path member contact each other when a nucleic acid is used as a label body.
[FIG. 4]
FIG. 4 is a partially enlarged cross-sectional view depicting a part at which a first detecting portion and a flow path member contact each other when an antibody is used as a label body.
[FIG. 5]
FIG. 5 is a partially enlarged view depicting a part at which a second detecting portion and a flow path member contact each other.
[FIG. 6]
FIG. 6 is a conceptual diagram of a membrane of an existing testing device.
[FIG. 7]
FIG. 7 is a cross-sectional view illustrating an example of a layer configuration of a transfer medium used for a testing device of the present invention.
[FIG. 8]
FIG. 8 is a conceptual diagram illustrating an example of a testing kit of the present invention.
[FIG. 9]
FIG. 9 is a top view illustrating an example of a testing device of Comparative Example 1 or Comparative Example 101.
[FIG. 10]
FIG. 10 is a schematic cross-sectional view of the testing device of FIG. 9 taken along a line B-B.

[Description of Embodiments]

(Testing device)

A testing device of the present invention includes a porous flow path member constituting a flow path through which a testing target liquid is flowed, a testing target liquid dropping portion provided on the flow path member, a labeling portion configured to apply a label to a target nucleic acid when the target nucleic acid is contained in the testing target liquid dropped onto the testing target liquid dropping portion, and a detecting portion configured to detect the target nucleic acid labeled at the labeling portion. The testing device includes a shaped body on the flow path member at the detecting portion. A capture nucleic acid including a sequence bindable and complementary with the target nucleic acid is immobilized by covalent binding to a surface of the shaped body between the shaped body and the flow path member. The testing device further includes other members as needed.

The testing device of the present invention is based on the following finding from existing lateral flow chromatographic devices for nucleic acid detection. That is, in the existing lateral flow chromatographic devices for nucleic acid detection, because reagents such as a capture nucleic acid and reagents such as a labeling indicator and a detecting indicator are immobilized to fibers in the flow path member, the flow path member that is formed of a material optionally selected for improving an analyte spreading speed may have an excessively strong interaction with the reagents such as the labeled nucleic acid and the labeling indicator and may not be able to spread the reagents, or may have an excessively weak interaction with the reagents such as a nucleic acid and the detecting indicator and may not be able to immobilize the analyte that is captured. The testing device of the present invention is also based on a finding that although a capture nucleic acid is present diffusively in a hydrophilic porous material forming the flow path member because a liquid in which the capture nucleic acid is dissolved is coated directly over the flow path member during formation of judgement lines such as a test line and a control line, the capture nucleic acid has a small molecular weight and may be diffused at a high speed to blur the judgement lines such as the test line and the control line or cause color unevenness in the judgement lines to make particularly the contours of the judgement lines unclear, or a color developed by labeling particles such as gold colloid particles bound with the capture nucleic acid present in the hydrophilic porous material cannot actually be sensed due to light scattering, i.e., most of the capture nucleic acid is not used effectively.

<Flow path member>

The flow path member of the testing device is not particularly limited and may be appropriately selected so long as the flow path member is a porous member capable of flowing the testing target liquid through the flow path member. Examples of the flow path member include a hydrophilic porous material.

The flow path member formed of the hydrophilic porous material contains voids. The flow path is formed when the testing target liquid flows through the voids. It is preferable that cells be present in the hydrophilic porous material, and that the cells be linked together to form a continuous cell.

The continuous cell is distinguished from independent cells that are not linked together.

The continuous cell has a function of sucking in a liquid by a capillary action or letting a gas pass through the continuous cell because the continuous cell has small holes in the walls between the cells.

The flow path member needs no external actuating device such as a pump because the flow path member is configured to deliver the testing target liquid by utilizing a capillary action through the voids.

A spreading speed in the flow path member is not particularly limited and may be appropriately selected depending on the intended purpose.

The hydrophilic porous material is not particularly limited and may be appropriately selected depending on the intended purpose. However, a material having hydrophilicity and a high voidage is preferable for use. The hydrophilic porous material refers to a porous material that is easily permeable by an aqueous solution.

The hydrophilic porous material is referred to as being easily permeable when in a water permeability evaluation test in which 0.01 mL of pure water is dropped onto a surface of a plate-shaped test piece of the hydrophilic porous material dried at 120 degrees C for 1 hour, the whole of 0.01 mL of the pure water permeates the test piece in 10 minutes.

The voidage of the hydrophilic porous material is not particularly limited, may be appropriately selected depending on the intended purpose, and is preferably 40% or greater but 90% or less and more preferably 65% or greater but 80% or less. When the voidage of the hydrophilic porous material is 90% or less, the hydrophilic porous material can maintain the strength as the flow path member. When the voidage of the hydrophilic porous material is 40% or greater, permeability of the testing target liquid is not influenced. The voidage of the hydrophilic porous material can be calculated according to a calculation formula 1 below based on a basis weight ($g/m^2$) and an average thickness (micrometer) of the hydrophilic porous material and a specific gravity of the component of the hydrophilic porous material.

<Calculation formula 1>

$$\text{Voidage (\%)} = \{1 - [\text{basis weight } (g/m^2)/\text{average thickness (micrometer)}/\text{specific gravity of the component}]\} \times 100$$

The hydrophilic porous material is not particularly limited and may be appropriately selected depending on the intended purpose. Examples of the hydrophilic porous material include filter paper, plain paper, wood free paper, watercolor painting paper, Kent paper, synthetic paper, synthetic resin films, special-purpose paper with a coat layer, fabrics, textile goods, films, inorganic substrates, and glass.

Examples of the fabrics include artificial fiber such as rayon, bemberg, acetate, nylon, polyester, and vinylon, natural fiber such as cotton and silk, blended fabrics of these fabrics, and non-woven fabrics of these fabrics.

Among these hydrophilic porous materials, filter paper is preferable because filter paper has a high voidage and a favorable hydrophilicity. When the testing device is used as a lateral flow chromatographic device for nucleic acid detection, the filter paper is preferable as a static bed of paper chromatography.

The shape of the hydrophilic porous material is not particularly limited and may be appropriately selected depending on the intended purpose. However, a sheet shape is preferable.

The average thickness of the hydrophilic porous material is not particularly limited, may be appropriately selected depending on the intended purpose, and is preferably 0.01 mm or greater but 0.3 mm or less. When the average thickness of the hydrophilic porous material is 0.01 mm or greater, the hydrophilic porous material can maintain the strength as the flow path member. When the average thickness of the hydrophilic porous material is 0.3 mm or less, the amount of the testing target liquid needed can be saved. In the present invention, the thickness can be defined as the length of an article in a direction perpendicular to a contact plane at which a base material and the flow path member contact each other.

<Testing target liquid dropping portion>

The testing target liquid dropping portion is not particularly limited and may be appropriately selected depending

on the intended purpose, so long as the testing target liquid dropping portion is formed at a place onto which the testing target liquid is dropped on the flow path member and is capable of supplying the testing target liquid to the flow path. The testing target liquid dropping portion may be selected from known materials.

<Labeling portion>

The labeling portion is a portion configured to apply a label to a target nucleic acid when the target nucleic acid is contained in the testing target liquid dropped onto the testing target liquid dropping portion.

It is preferable that a label body contained in the labeling portion contain a single-stranded nucleic acid fragment complementary with the target nucleic acid, and that the target nucleic acid be labeled by hybridization between the target nucleic acid and the label body.

It is preferable that the label body contained in the labeling portion contain an antibody having bindability with the target nucleic acid or with a compound or a protein bound with the target nucleic acid, and that the target nucleic acid be labeled by an antibody-antigen reaction between the target nucleic acid and the label body.

-Label body-

The label body is not particularly limited and may be appropriately selected depending on the intended purpose so long as the label body can bind with the target nucleic acid. Examples of the label body include a nucleic acid including a sequence complementary with the target nucleic acid, and an antibody against a compound labeled with the target nucleic acid in the testing target liquid or against a protein forming a complex with the target nucleic acid in the testing target liquid. When the nucleic acid is used, the base sequence and length of the nucleic acid may be selected depending on the intended purpose. When the antibody is used, an antibody against an antigen may be selected depending on the intended purpose. It is preferable that the nucleic acid and the antibody contain a label. A preferable example is a gold colloid-labeled nucleic acid.

Particles for labeling the nucleic acid and the antibody are not particularly limited to gold colloid but may be appropriately selected depending on the intended purpose. Examples of the particles include metal colloids, enzymatically labeling particles containing an enzyme, coloring particles containing a pigment, fluorescent particles containing a fluorescent substance, and magnetic body-encapsulating particles containing a magnetic body. The kind of the nucleic acid is not particularly limited so long as the nucleic acid is single-stranded. Examples of the nucleic acid include deoxyribonucleic acid (DNA), ribonucleic acid (RNA), and peptide nucleic acid (PNA). It is common to use a nucleic acid having a base length of from 5 bases through 60 bases. A nucleic acid having a base length of from 10 bases through 40 bases is preferable.

The base sequence of the nucleic acid is not particularly limited so long as the base sequence is complementary with the target nucleic acid. However, a sequence different from the capture nucleic acid immobilized to the detecting portion is preferable.

The nucleic acid may be bound with a labeling substance such as gold colloid directly via a functional group, or by means of bindability of avidin-biotin, streptavidin-biotin, neutravidin-biotin, antibiotin antibody-biotin, hapten-antihapten antibody, and digoxigenin-antidigoxigenin antibody. The antibody may be bound with the labeling substance such as gold colloid by adsorption or may be bound via a functional group.

When the nucleic acid is used as the label body, a site of the target nucleic acid to be bound with the capture nucleic acid and a site of the target nucleic acid to be bound with the label body may be appropriately selected depending on the intended purpose such as specificity and a detecting temperature. It is preferable that a length from one end of the site to be bound with the capture nucleic acid to one end of the site to be bound with the label body be 50 bases or less, more preferably 20 bases or less, and yet more preferably 10 bases or less.

<Detecting portion>

The detecting portion is a portion configured to detect the target nucleic acid labeled at the labeling portion.

A shaped body is provided on the flow path member at the detecting portion. A capture nucleic acid including a sequence bindable and complementary with the target nucleic acid is immobilized by covalent binding to a surface of the shaped body between the shaped body and the flow path member.

It is preferable that a shaped body be provided on the flow path member at the detecting portion, and that a capture nucleic acid including a sequence bindable and complementary with the target nucleic acid be bound by a linker to the surface of the shaped body between the shaped body and the flow path member.

A plurality of detecting portions may be disposed on the flow path member, and capture nucleic acids immobilized to the plurality of detecting portions may have different sequences.

A shaped body is provided on the flow path member at the detecting portion. A capture nucleic acid including

a sequence bindable and complementary with the target nucleic acid is immobilized by covalent binding to the surface of the shaped body between the shaped body and the flow path member.

-Shaped body-

As the shaped body, a capture nucleic acid including a sequence bindable and complementary with the target nucleic acid is immobilized by covalent binding to a surface of the shaped body between the shaped body and the flow path member.

As the shaped body, it is preferable that a capture nucleic acid including a sequence bindable and complementary with the target nucleic acid be bound by a linker with the surface of the shaped body between the shaped body and the flow path member.

As the shaped body, it is preferable that a capture nucleic acid including: a sequence bindable and complementary with the target nucleic acid; and a spacer be immobilized to the surface of the shaped body between the shaped body and the flow path member.

The shaped body is not particularly limited and may be appropriately selected depending on the intended purpose so long as the shaped body is a shaped body formed of a resin. It is preferable that the capture nucleic acid be immobilized by covalent binding to a surface of the shaped body at a side facing the flow path member. It is also preferable that the capture nucleic acid be bound by a linker to the surface of the shaped body at the side facing the flow path member.

With the capture nucleic acid immobilized to the surface of the shaped body at the side facing the flow path member, it is possible to control affinity between the shaped body and the testing target liquid and between the capture nucleic acid and the target nucleic acid. As the method for adjusting the affinity, there is a method of, for example, changing the kind of the resin constituting the shaped body or the composition ratio of resins constituting the shaped body depending on the target nucleic acid concerned.

--Binding by linker--

The linker is a molecule to be bound for immobilizing the capture nucleic acid to the shaped body.

Binding (cross-linking) by the linker is not particularly limited and may be appropriately selected depending on the intended purpose so long as the capture nucleic acid can be immobilized to the shaped body via the linker. Examples of the binding by the linker include covalent binding, coordination binding, metal binding, ionic binding, hydrogen binding, and van der Waals binding. Among these kinds of binding, covalent binding is preferable in terms of bonding strength.

-- Resin--

The resin constituting the shaped body is not particularly limited and may be appropriately selected depending on the intended purpose so long as the resin has a functional group having bindability with the capture nucleic acid. However, a water-insoluble resin is preferable. When the water-insoluble resin is used, dissolution of the water-insoluble resin in the testing target liquid can be prevented. This makes it possible to prevent the flow path from being clogged and judgement lines such as a control line and a test line from being blurred.

It is preferable that the shaped body and the capture nucleic acid have functional groups having bindability with each other.

Examples of the functional group of the shaped body having bindability with the capture nucleic acid include carboxyl group, acid anhydride, active ester group, aldehyde group, isocyanato group, isothiocyanato group, tosyl group, pyridyl disulfide group, bromoacetyl group, hydroxyl group, amino group, epoxy group, thiol group, maleimide group, vinyl sulfone group, aminooxyacetyl group, diazo group, carbodiimide group, vinyl group, nitro group, sulfone group, succinimide group, hydrazide group, azido group, phosphoric acid group, azlactone group, nitrile group, amide group, imino group, nitrene group, acetyl group, sulfonyl chloride group, acyl azide group, anhydride group, fluorobenzene group, carbonate group, imide ester group, epoxide group, and fluorophenyl ester group. One of these functional groups may be used alone or two or more of these functional groups may be used in combination.

The active ester group refers to an ester group having a high reactivity. Specific examples of the active ester group include p-nitrophenyl ester group, N-hydroxysuccinimide ester group, N-hydroxysulfosuccinimide ester group, succinic acid imide ester group, phthalic acid imide ester group, and 5-norbornene-2,3-dicarboxyimide ester group. One of these active ester groups may be used alone or two or more of these active ester groups may be used in combination.

Among the functional groups, carboxyl group, acid anhydride, active ester group, aldehyde group, isocyanato group, isothiocyanato group, tosyl group, pyridyl disulfide group, bromoacetyl group, hydroxyl group, amino group, epoxy group, maleimide group, and thiol group are preferable, and carboxyl group, amino group, hydroxyl group, active ester group, and maleimide group are particularly preferable.

The shaped body needs only to have the functional group on at least the surface of the shaped body facing the flow path member. It is possible to use the shaped body to which the functional group is introduced by a known surface treatment method. In this case, examples of the resin include thermoplastic resins and thermosetting resins. Among these resins, the thermoplastic resins are preferable in terms of production efficiency.

Examples of the thermoplastic resins include: straight-chain polyolefins such as polystyrene (PS) resins, polyethylene (PE) resins, and polypropylene (PP) resins, cyclic polyolefins, ethylene-vinyl acetate (EVA) copolymerized resins, acrylonitrile-styrene (AS) copolymerized resins, acrylic acid ester polymerized (acrylic) resins, methacrylic acid ester polymerized (acrylic) resins, methyl methacrylate (PMMA) resins, polyamide (PA) resins, and polycarbonate (PC) resins; ester resins such as polyethylene terephthalate (PET) resins and polybutylene terephthalate (PBT) resins; and cellulose acetate (CA) resins, cycloolefin (CO)-based resins, imine resins, ethyleneimine resins, and epoxy resins. One of these thermoplastic resins may be used alone or two or more of these thermoplastic resins may be used in combination.

Examples of compounds to constitute the shaped body other than the resins include: natural waxes such as a beeswax, a carnauba wax, a cetaceum, a Japan tallow, a candellila wax, a rice bran wax, and a montan wax; synthetic waxes such as a paraffin wax, a microcrystalline wax, an oxide wax, ozokerite, ceresin, an ester wax, a polyethylene wax, and a polyethylene oxide wax; higher fatty acids such as margaric acid, lauric acid, myristic acid, palmitic acid, stearic acid, furoic acid, and behenic acid; higher alcohols such as stearic alcohol and behenyl alcohol; esters such as fatty acid ester of sorbitan; and amides such as stearamide and oleamide. One of these compounds may be used alone or two or more of these compounds may be used in combination.

Among the resins and the compounds other than the resins, acrylic resins, polystyrene resins, polyolefin resins, ester resins, epoxy resins, ethyleneimine resins, carnauba wax, and polyethylene wax are preferable in terms of the purpose of use.

The method for surface treatment of the shaped body is not particularly limited and may be appropriately selected depending on the intended purpose. For example, for introduction of the carboxyl group or the hydroxyl group to the surface of the shaped body, methods such as plasma treatment, corona discharge treatment, flame treatment, and ultraviolet irradiation treatment may be used. Among these methods, plasma treatment and corona discharge treatment under an oxygen atmosphere are preferable in terms of a high reaction efficiency. For introduction of the amino group to the surface of the shaped body, for example, methods such as plasma treatment and aminoalkylsilane treatment under a nitrogen atmosphere may be used. Of these methods, plasma treatment under nitrogen atmosphere is preferable in terms of ease of treatment and uniformity.

It is preferable that the shaped body be a non-porous body. The non-porous body refers to a non-porous structure substantially free of voids, and a structure opposite to a porous material such as a membrane that contains voids provided for promoting absorption of a liquid. Hence, for example, a material that contains only few cells that have been incidentally mixed in the material during a production process and do not contribute to promotion of the liquid absorbing action is encompassed within the non-porous body.

Next, characteristics of the shaped body used in the present invention when the shaped body is a non-porous body will be described.

Hitherto, judgement lines such as a test line and a control line have been formed by directly coating a liquid in which the capture nucleic acid is dissolved over the flow path member formed of a hydrophilic porous material. Hence, the capture nucleic acid is quickly diffused inside the porous material concentrically along with permeation of the liquid because the capture nucleic acid is typically a single-stranded nucleic acid including about 5 bases through about 60 bases and has a small molecular weight. This tends to blur the judgement lines such as a test line and a control line or cause color unevenness in the judgement lines, and particularly make the contours of the lines unclear. Moreover, a color developed by labeling particles such as gold colloid particles bound with the capture nucleic acid present in the porous material cannot actually be sensed due to light scattering. This means that most of the capture nucleic acid is not used effectively.

Generally, color developing particles that can be sensed from a porous material are particles that are present at and above the depth of about 5 micrometers from the surface of the porous material. In order to immobilize the capture nucleic acid needed for testing to the region at and above the depth of 5 micrometers, there is a need for coating the capture nucleic acid in a large amount considering diffusion of the capture nucleic acid in the direction of thickness. That is, the amount of the capture nucleic acid to be coated increases in proportion to the thickness of the porous material.

Meanwhile, in the present invention, when a resin shaped body formed of a non-porous body containing many hydrophobic groups is used for immobilizing the capture nucleic acid, the capture nucleic acid is immobilized to only the surface of the shaped body without entering the inside of the resin shaped body. A color is developed when labeling particles bind with the capture nucleic acid immobilized to the surface of the shaped body. The color can be sensed through the shaped body formed of the non-porous body that does not scatter light. This significantly improves the efficiency of utilization of the color developed by the labeling particles. Because there are no wasteful color developing particles in the direction of thickness, there is an advantage that the amount of the capture nucleic acid to be coated can be significantly saved. For example, when it is assumed that the thickness of the flow path member formed of a

hydrophilic porous material is 100 micrometers and color development from a region at and above the depth of 5 micrometers from the surface of the flow path member can only be utilized, the amount of the capture nucleic acid used for obtaining the same color developing intensity can be reduced to 1/20 in the present invention.

Hence, in the present invention, when the shaped body formed of a non-porous body containing many hydrophobic groups is used for immobilizing the capture nucleic acid, the efficiency of utilization of the color developed by the labeling particles can be improved significantly, and the amount of the capture nucleic acid to be coated can be reduced from the amount hitherto used because there are no wasteful color developing particles in the direction of thickness.

It is preferable that the shaped body be immobilized over the flow path member. The method for immobilizing the shaped body is not particularly limited so long as the method immobilizes the shaped body in a state that enables the capture nucleic acid and the testing target liquid to contact each other during testing. Examples of the method include a method for thermally transferring the constituent resin of the shaped body onto the flow path member with, for example, a thermal transfer printer, a method for applying a pressure to the constituent resin of the shaped body and transferring the resin with, for example, a dot impact printer, and a method for pasting the constituent resin of the shaped body over the flow path member with, for example, a tape, an adhesive, and a tackifier.

-Linker-

The linker needs only to contain a functional group having reactivity with the functional group present on the front surface of the shaped body at one end of the linker, and a functional group having reactivity with a functional group introduced into the capture nucleic acid at another end of the linker. The strength of the linker may be appropriately selected depending on the intended purpose. The linker may be a linker that contains a single functional group and has reactivity for continuously reacting with the functional group present on the front surface of the shaped body and with a functional group introduced into the capture nucleic acid.

A spacer may be inserted between the 2 functional groups of the linker. The distance between the functional groups is preferably at least 3 angstroms (0.3 nm), more preferably from 3 angstroms (0.3 nm) through 35 angstroms (3.5 nm), and yet more preferably from 3 angstroms (0.3 nm) through 25 angstroms (2.5 nm).

When the distance between the functional groups is long, it is preferable that the spacer be a water-soluble resin so as not to inhibit binding between the target nucleic acid and the capture nucleic acid.

The functional groups having bindability with the shaped body and the capture nucleic acid are not particularly limited and may be appropriately selected depending on the intended purpose. Examples of the functional groups include carboxyl group, acid anhydride, active ester group, aldehyde group, isocyanato group, isothiocyanato group, tosyl group, pyridyl disulfide group, bromoacetyl group, hydroxyl group, amino group, epoxy group, thiol group, maleimide group, vinylsulfone group, aminooxyacetyl group, diazo group, carbodiimide group, vinyl group, nitro group, sulfone group, succinimide group, hydrazide group, azide group, phosphoric acid group, azlactone group, nitrile group, amide group, imino group, nitrene group, and acetyl group. One of these functional groups may be used alone or two or more of these functional groups may be used in combination.

The active ester group refers to an ester group having a high reactivity. Specific examples of the active ester group include p-nitrophenyl ester group, N-hydroxysuccinimide ester group, succinic acid imide ester group, phthalic acid imide ester group, and 5-norbornene-2,3-dicarboxyimide ester group.

Among the functional groups, carboxyl group, acid anhydride, active ester group, aldehyde group, isocyanato group, isothiocyanato group, tosyl group, pyridyl disulfide group, bromoacetyl group, hydroxyl group, amino group, epoxy group, maleimide group, and thiol group are preferable, and carboxyl group, amino group, active ester group, and maleimide group are particularly preferable.

Examples of the water-soluble resins include polyethylene glycol (PEG), single-stranded DNA, polynucleotide formed of RNA or PNA, and polypeptide.

It is preferable that the linker contain an N-hydroxysuccinic acid imide ester group at one end and be bound with an amino group on the surface of the shaped body by amide binding.

It is preferable that the linker contain a maleimide group at one end and be bound with a thiol group introduced at a 5' end or a 3' end of the capture nucleic acid by thioether binding.

It is preferable that an atom be present between the N-hydoxysuccinic acid imide ester group and the maleimide group of the linker, and that the N-hydoxysuccinic acid imide ester group and the maleimide group be separated by at least 3 angstroms (0.3 nm).

A structural formula of GMBS, which is an example of the linker containing the N-hydoxysuccinic acid imide ester group and the maleimide group, is presented below.

[Chem. 1]

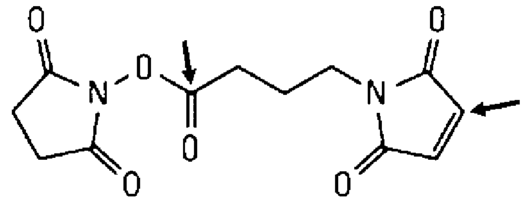

In the structural formula, the distance between a carbon atom in the N-hydroxysuccinic acid imide ester group indicated by an arrow and a carbon atom in the maleimide group indicated by an arrow is at least 3 angstroms (0.3 nm) because of the chemical bond distance between the N-hydroxysuccinic acid imide ester group and the maleimide group in the linker.

It is preferable that the distance between the N-hydroxysuccinic acid imide ester group and the maleimide group in the linker be from 3 angstroms (0.3 nm) through 35 angstroms (3.5 nm) and more preferably from 3 angstroms (0.3 nm) through 25 angstroms (2.5 nm). Experimentally, it is preferable that the distance between the N-hydoxysuccinic acid imide ester group and the maleimide group be at least 7.3 angstroms (0.73 nm), more preferably from 7.3 angstroms (0.73 nm) through 32.5 angstroms (3.25 nm), and yet more preferably from 7.3 angstroms (0.73 nm) through 24.6 angstroms (2.46 nm).

It is preferable that the linker contain polyethylene glycol (PEG) between the N-hydoxysuccinic acid imide ester group and the maleimide group.

-Spacer-

The spacer is a molecule to be inserted in-between to secure a space (distance). The kind of the molecule constituting the spacer is not particularly limited and may be appropriately selected depending on the intended purpose. It is preferable that the spacer in the capture nucleic acid be inserted to be positioned between the shaped body and the sequence bindable and complementary with the target nucleic acid.

When the spacer is positioned between the shaped body and the sequence bindable and complementary with the target nucleic acid, the efficicency of the capture nucleic acid's binding with the target nucleic acid and the label body is improved, and sequence specificity is also improved.

It is preferable that the spacer be formed of an alkyl group, or of an alkyl group and a phosphoric acid group. Examples of such a spacer include one represented by a structural formula below.

[Chem. 2]

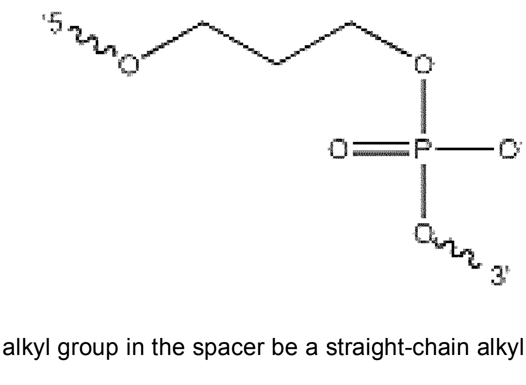

It is preferable that the alkyl group in the spacer be a straight-chain alkyl group containing from 2 through 12 carbon atoms.

It is preferable that the spacer be formed of an alkyl group, a tetrahydrofuran group, and a phosphoric acid group. Examples of such a spacer include one represented by a structural formula below.

[Chem. 3]

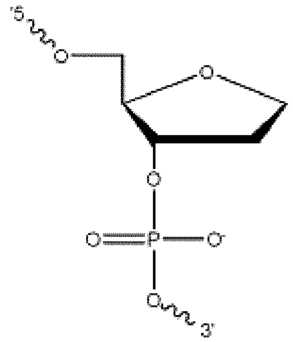

It is preferable that the number of atoms in the main chain included in the spacer be from 10 through 40.
The spacer is preferably one represented by general formula I below.

<General formula I>

[Chem. 4]

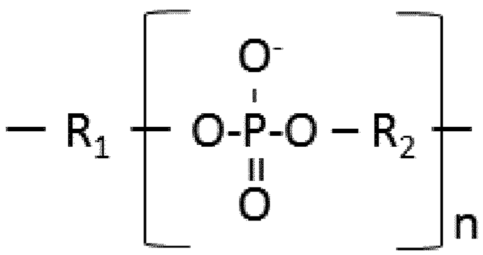

In general formula I, $R_1$ represents a substituted or unsubstituted alkylene group, $R_2$ represents a substituted or unsubstituted alkylene group, n represents an integer, and the substituted alkylene group represented by $R_2$ is an alkylene group having a cyclic structure.
It is preferable that the number of carbon atoms in the unsubstituted alkylene group represented by $R_1$ and $R_2$ be from 2 through 24, and that the number of carbon atoms in the substituted alkylene group represented by $R_2$ be 4.
n is an integer of preferably 20 or lower and more preferably from 0 through 5.
The spacer is not particularly limited and may be appropriately selected depending on the intended purpose so long as the spacer contains an appropriate number of atoms and has water-solubility. Nucleic acids such as DNAs are commonly synthesized by solid-phase synthesis. For example, DNAs are synthesized on a porous solid phase called CPG. With a 3’ end of a DNA strand immobilized to CPG, DNAs are elongated by coupling bases one by one in a 5’ direction. Each coupling step is performed by sequentially binding a nucleoside, which is a DNA monomer derivatized with a phosphoramidite. A reactive phosphoramidite is bound with a 3’-OH group of the nucleoside, and a dimethoxytrityl group, which is a protecting group, is bound with a 5’-OH group of the nucleoside. Removal of the dimethoxytrityl group, coupling of a nucleoside containing an intended base, and capping of an unreacted moiety are repeated. This allows phosphoramidites and 5’-OH groups of the nucleosides to react with each other to be elongated. There are commercially available spacer molecules (monomers) derivatized with phosphoramidites that are reactive in the same manner as in the elongation reaction in the solid-phase synthesis of nucleic acids. These spacer molecules are suitable for the spacer in terms of versatility. Preferable examples include: linkers formed of a straight-chain alkyl group and a phosphoric acid group, such as a C2 linker containing 2 carbon atoms, a C3 linker containing 3 carbon atoms, a C4 linker containing 4 carbon atoms, a C6 linker containing 6 carbon atoms, a C9 linker containing 9 carbon atoms, and a C12 linker containing 12 carbon atoms; and a spacer 9 and a spacer 18 that are formed of polyethylene glycol and a phosphoric acid group. Preferable examples further include 1,2-dideoxyribose (d spacer) and deoxy-D-ribose (r spacer) that are formed of a tetrahydrofuran group and a phosphporic acid group. Particularly, the C2 linker, the C3 linker, the C4 linker, the d spacer,

and the r spacer are preferable, and the C3 linker and the d spacer are more preferable. These spacers may be used alone or in combination.

<C3 linker>

[Chem. 5]

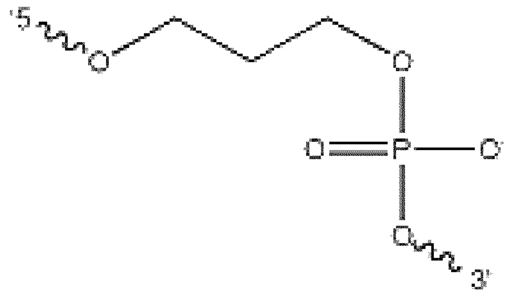

<d spacer>

[Chem. 6]

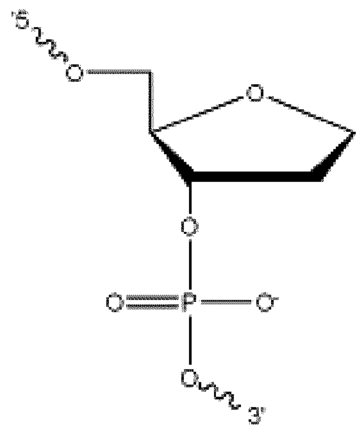

The molecular length of the spacer is not particularly limited and may be appropriately selected and adjusted depending on the intended purpose. Nucleic acids are synthesized by solid-phase synthesis. Hence, when the capture nucleic acid contains an introduced functional group, it is common to insert a spacer formed of an alkyl group between the functional group and the nucleic acid. A chemical formula below represents an example in which the capture nucleic acid containing a carboxyl group as the functional group is bound with the shaped body.

[Chem. 7]

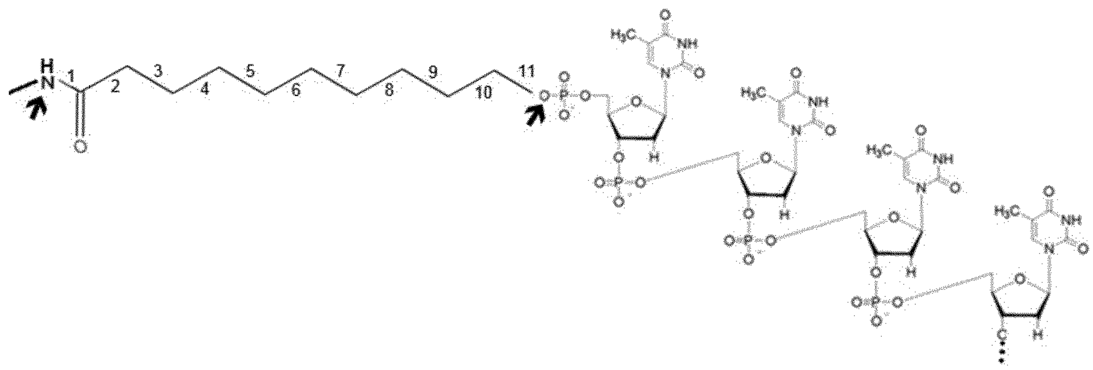

In the present invention, the molecular length of the spacer is the number of atoms included in the main chain extending from the functional group possessed by the shaped body and indicated by an arrow in the chemical formula above to an O atom of the first phosphporic acid group of the nucleic acid moiety in the capture nucleic acid indicated by an arrow in the chemical formula above. In the chemical formula above, the spacer has a molecular length corresponding to the number of atoms of 11. Insertion of, for example, the C3 linker or the d spacer in the chemical formula above is between the alkyl group and the first phosphoric acid group of the nucleic acid moiety. The molecular length of the spacer needs only to be a length corresponding to the number of atoms of 1 or greater. The number of atoms in the main chain is preferably from 4 through 100, and particularly preferably from 7 through 60. Experimentally, the number of atoms in the main chain is preferably from 7 through 40, and particularly preferably from 13 through 40.

-Capture nucleic acid-

It is preferable that the capture nucleic acid contain a functional group covalently bindable with the functional group present on the surface of the shaped body. The capture nucleic acid is not particularly limited so long as the capture nucleic acid is bindable with at least any one of the target nucleic acid and the label body. The base sequence and length of the capture nucleic acid may be selected depending on the intended purpose.

It is preferable that the capture nucleic acid be single-stranded, terminally modified with the functional group, and hybridizable with the target nucleic acid.

The kind of the capture nucleic acid is not particularly limited and may be appropriately selected depending on the intended purpose. Examples of the kind of the capture nucleic acid include deoxyribonucleic acid (DNA), ribonucleic acid (RNA), and peptide nucleic acid (PNA). It is common to use a nucleic acid having a base length of from 5 bases through 60 bases. A nucleic acid having a base length of from 10 bases through 40 bases is preferable.

The functional group of the capture nucleic acid covalently bindable with the functional group present on the surface of the shaped body is not particularly limited and may be appropriately selected depending on the intended purpose. Examples of the functional group of the capture nucleic acid include carboxyl group, acid anhydride, active ester group, aldehyde group, isocyanato group, isothiocyanato group, tosyl group, pyridyl disulfide group, bromoacetyl group, hydroxyl group, amino group, epoxy group, thiol group, maleimide group, vinylsulfone group, aminooxyacetyl group, diazo group, carbodiimide group, vinyl group, nitro group, sulfone group, succinimide group, hydrazide group, azide group, phosphoric acid group, azlactone group, nitrile group, amide group, imino group, nitrene group, acetyl group, sulfonyl chloride group, acyl azide group, anhydride group, fluorobenzene group, carbonate group, imide ester group, epoxide group, and fluorophenyl ester group. One of these functional groups may be used alone or two or more of these functional groups may be used in combination.

The active ester group refers to an ester group having a high reactivity. Specific examples of the active ester group include p-nitrophenyl ester group, N-hydroxysuccinimide ester group, N-hydroxysulfosuccinimide ester group, succinic acid imide ester group, phthalic acid imide ester group, and 5-norbornene-2,3-dicarboxyimide ester group.

Among the functional groups of the capture nucleic acid, carboxyl group, acid anhydride, active ester group, aldehyde group, isocyanato group, isothiocyanato group, tosyl group, pyridyl disulfide group, bromoacetyl group, hydroxyl group, amino group, epoxy group, maleimide group, and thiol group are preferable, and carboxyl group, amino group, thiol group, active ester group, and maleimide group are particularly preferable.

The position to which the functional group is introduced may be at an end of the molecular chain of the nucleic acid or in the molecular chain of the nucleic acid. However, for efficient binding with the target nucleic acid, it is preferable that the functional group be introduced at an end of the molecular chain. A 5' end or a 3' end may be selected depending on the intended purpose.

The method for immobilizing the capture nucleic acid to the shaped body is not particularly limited and may be appropriately selected depending on the intended purpose. Examples of the method include a method of directly covalently binding the functional group present on the surface of the shaped body with the functional group possessed by the capture nucleic acid, and a method of introducing a compound having an appropriate chain length between the shaped body and the capture nucleic acid as a medium (spacer).

When the shaped body containing a carboxyl group on the surface is used, it is possible to bind the capture nucleic acid with the shaped body by forming an amide bond through a reaction between an amino group introduced into the molecule of the capture nucleic acid and the carboxyl group on the surface of the shaped body in the presence of a dehydration condensation agent such as a water-soluble carbodiimide such as 1-ethyl-3-(3-dimethylaminopropyl)carbodiimide (EDC). When the shaped body containing an aldehyde group on the surface is used, for example, there is a method of allowing an amino group introduced into the molecule of the capture nucleic acid to undergo a reaction with the aldehyde group to form a Schiff base, and allowing a reducing agent such as sodium cyanoborohydride to undergo a reaction with the Schiff base to form a stable covalent bond.

Specifically, for immobilizing the capture nucleic acid to the surface of the shaped body, a method of coating a liquid in which the capture nucleic acid is dissolved or dispersed is preferable. pH of the liquid in which the capture nucleic acid is dissolved or dispersed is not particularly limited, and the liquid may be set to pH suitable for the immobilization reaction. However, when the capture nucleic acid is a RNA, pH is preferably lower than 9.0 because the RNA is hydrolyzed in alkaline conditions. After the capture nucleic acid is immobilized, the surface to which the capture nucleic acid is immobilized may be washed with water containing a surfactant or a buffer solution, so unnecessary components can be removed. When a functional group reactive with the capture nucleic acid remains on the surface to which the capture nucleic acid is immobilized, it is preferable to perform a treatment for deactivating the functional group remaining on the surface by means of an alkali compound or a compound containing a primary amino group.

--Covalent binding--

The kind of the covalent binding is not particularly limited and may be appropriately selected depending on the intended purpose. Examples of the kind of the covalent binding include amide binding, ester binding, thiourea binding, thioether binding, ether binding, imine binding, and disulfide binding. One of these kinds of covalent binding may be used alone or two or more of these kinds of covalent binding may be used in combination.

The imine binding refers to R1-CH=N-R2. R1 and R2 represent different alkyl groups. R1 and R2 may be the same.

Whether the capture nucleic acid is covalently bound with the surface of the shaped body can be confirmed by, for example, a FT-IR ATR method.

<Other members>

The other members are not particularly limited and may be appropriately selected depending on the intended purpose. Examples of the other members include a label body supplying portion, a label body spreading member, a base material, and an absorbing member.

-Label body supplying portion-

The label body supplying portion is not particularly limited and may be appropriately selected depending on the intended purpose, so long as the label body supplying portion is capable of supplying a label body to the flow path member. Examples of the label body supplying portion include a structure configured to drop the label body onto the flow path using a separate device and a separate tool, and a structure configured to supply the label body using a label body spreading member laminated on the flow path and including the label body. Of these structures, the structure including the label body spreading member is preferable.

-Label body spreading member-

The label body spreading member is not particularly limited and may be selected from known materials depending on the intended purpose, so long as the label body spreading member is capable of supporting the label body in a state in which the label body can be spread. Examples of the label body spreading member include cellulose filter paper, glass fiber, and non-woven fabrics.

The method for making the label body spreading member support the label body is not particularly limited and may be appropriately selected depending on the intended purpose. Examples of the method include a method of producing the label body spreading member by impregnating the label body spreading member with the label body in a predetermined amount and drying the label body spreading member.

Particularly, there is a need that the label body spreading member be capable of letting the label body easily

leach from the label body spreading member when the label body spreading member is permeated by the testing target liquid and letting the label body move together with the testing target liquid. Therefore, glass fiber that typically has a weak adsorption force to the label body is preferable. As the label body spreading member, for example, glass fiber supporting the label body is disposed on the flow path member at a position upstream from the detecting portion in a state that the label body spreading member can spread the label body. In this way, only dropping the testing target liquid onto, for example, the dropping portion described below enables the testing to be performed in a manner that when the target nucleic acid is contained in the testing target liquid, the label body spreading member of the label body supplying portion binds the target nucleic acid with the label body and spreads the target nucleic acid together with the label body toward a second diffusion immobilizing portion. This can simplify the operation of the testing device.

-Base material-

The base material may be, for example, of any structure, any material, and any shape that may be selected depending on the intended purpose. The structure of the base material is not particularly limited and may be appropriately selected depending on the intended purpose. Examples of the structure of the base material include a structure obtained by laminating the flow path member over the top surface of the base material.

The constituent material of the base material is not particularly limited and may be appropriately selected depending on the intended purpose. Examples of the constituent material of the base material include organic, inorganic, and metal materials. It is preferable that at least one surface of the base material be coated with a hydrophobic resin, although this is non-limiting. When the testing device is used as a sensor chip, it is preferable to use a synthetic resin that is light-weight, flexible, and inexpensive as the base material. Furthermore, a constituent material having a high durability such as a plastic sheet can be selected as the base material. Therefore, the durability of the testing device is improved as a result.

Examples of the constituent material of the base material include polyvinyl chloride, polyethylene terephthalate, polypropylene, polystyrene, polyvinyl acetate, polycarbonate, polyacetal, modified polyphenyl ether, polybutylene terephthalate, and ABS resins. One of these constituent materials may be used alone or two or more of these constituent materials may be used in combination. Among these constituent materials, the base material formed of polyethylene terephthalate is particularly preferable for use because polyethylene terephthalate is low-price and highly versatile.

The shape of the base material is not particularly limited and may be appropriately selected depending on the intended purpose. However, a sheet shape is preferable.

The average thickness of the base material is not particularly limited, may be appropriately selected depending on the intended purpose, and is preferably 0.01 mm or greater but 0.5 mm or less. When the average thickness of the base material is 0.01 mm or greater, the base material has an adequate strength as a base material. When the average thickness of the base material is 0.5 mm or less, the base material has flexibility and is suitable for a sensor.

The average thickness of the base material may be an average of thicknesses measured with a micrometer at a total of 15 positions of a measuring target, namely, for example, 5 positions in the longer direction × 3 positions in the width direction that are at approximately equal intervals.

-Absorbing member-

The absorbing member is not particularly limited and may be selected from known materials so long as the absorbing member absorbs the liquid in the testing target liquid. For example, when the liquid is water, examples of the absorbing member include fiber such as paper and cloth, polymer compounds containing a carboxyl group or a salt of a carboxyl group, partially cross-linked products of polymer compounds containing a carboxyl group or a salt of a carboxyl group, and partially cross-linked products of polysaccharides.

Here, the testing device of the present invention will be described in detail with reference to the drawings. In the present invention, there are 2 detecting portions, to which different nucleic acids are immobilized. Therefore, one of the detecting portions will be referred to as "first detecting portion 50a", and the other of the detecting portions will be referred to as "second detecting portion 50b".

FIG. 1 is a top view illustrating an example of the testing device of the present invention. FIG. 2 is a schematic cross-sectional view taken alone a line A-A of FIG. 1. FIG. 3 is a partially enlarged cross-sectional view depicting a portion at which the first detecting portion and the flow path member contact each other when a nucleic acid is used as the label body. FIG. 4 is a partially enlarged cross-sectional view depicting a portion at which the first detecting portion and the flow path member contact each other when an antibody is used as the label body. FIG. 5 is a partially enlarged view depicting a portion at which the second detecting portion and the flow path member contact each other.

As illustrated in FIG. 1 and FIG. 2, the testing device 10 of the present invention includes a porous flow path member 30 in which there is formed the flow path through which the hydrophilic testing target liquid such as blood, spinal fluid, urine, or an extract liquid for testing (e.g., a liquid containing an analyte collected with an analyte collecting unit

such as a stick) is flowed, and a label body (nucleic acid) supplying portion 40, a first detecting portion 50a, and a second detecting portion 50b that are provided over the flow path member 30. As illustrated in FIG. 3 to FIG. 5, a first capture nucleic acid 17 and a second capture nucleic acid 18 that are reactive with at least any one of a target nucleic acid 14 contained in a testing target liquid 12 and the label body (nucleic acid) are immobilized to the surfaces of the first detecting portion 50a and the second detecting portion 50b facing the flow path member 30. The first capture nucleic acid 17 binds with the target nucleic acid 14, and the second capture nucleic acid 18 binds with the label body (nucleic acid) 16. This makes it possible to adjust the strength of covalent binding between the shaped body and the capture nucleic acid separately at each of the plurality of detecting portions, to make it easier to control immobilization of the capture nucleic acid even when the flow path member 30 is appropriately selected depending on the intended purpose.

In the following description, a case where the testing target liquid is a hydrophilic liquid such as blood, spinal fluid, urine, or an extract liquid for testing (e.g., a liquid containing an analyte collected with an analyte collecting unit such as a stick) will be described.

As illustrated in FIG. 1 and FIG. 2, a case where in the testing device 10, the flow path member 30 is provided over the base material 20, and an absorbing member 70 is provided over the base material 20 and the flow path member 30 at one end of the base material 20 and the flow path member 30 will be described. However, the testing device 10 of the present invention is not limited to this embodiment. What is meant when it is said that something is provided over the flow path member 30 is that that something is provided to contact the flow path member regardless of whether that something is above or below the flow path member when the testing device 10 is set in place. When an arbitrary detecting portion of the first detecting portion 50a and the second detecting portion 50b is to be referred to, the arbitrary detecting portion will be denoted as detecting portion 50. The capture nucleic acids need only to be immobilized by covalent binding.

As illustrated in FIG. 1 to FIG. 5, the first detecting portion 50a is used as a test line for judging presence or absence of the target nucleic acid 14, and the second detecting portion 50b is used as a control line for indicating that the label body (nucleic acid) 16 has arrived.

As illustrated in FIG. 1 and FIG. 2, the label body (nucleic acid) supplying portion 40 is disposed to contact the flow path member 30. As described above, the label body (nucleic acid) supplying portion 40 supports the label body (nucleic acid) 16 at a position upstream from the detecting portions 50 in a state capable of spreading the label body (nucleic acid) 16. As illustrated in FIG. 2, the label body (nucleic acid) supplying portion 40 supports the label body (nucleic acid) 16 on a surface of the label body (nucleic acid) supplying portion 40 at the flow path member 30 side.

As illustrated in FIG. 1 and FIG. 2, the first detecting portion 50a is disposed to contact the flow path member 30. As illustrated in FIG. 3 and FIG. 4, the first detecting portion 50a contains a functional group having bindability with the first capture nucleic acid 17 on a surface of the first detecting portion 50a. The first capture nucleic acid 17 contains a functional group bindable with the functional group present on the surface of the first detecting portion 50a. By the functional group, the first capture nucleic acid 17 forms a covalent bond 52 on the surface of the first detecting portion 50a facing the flow path member 30 and is immobilized to the surface. When the gap formed between the facing surfaces of the flow path member 30 and the first detecting portion 50a is filled with the testing target liquid 12, the first capture nucleic acid 17 captures the target nucleic acid 14 that is in a state of being bound with the label body (nucleic acid) 16. As a result, the target nucleic acid 14 and the label body (nucleic acid) 16 are immobilized to develop a color. Hence, the first detecting portion 50a can be used as a test line for judging presence or absence of the target nucleic acid 14. In FIG. 4, the reference sign 19 denotes a label body (antibody). In FIG. 4, the first capture nucleic acid 17 captures the target nucleic acid 14 that is in a state of being bound with the label body (antibody) 19.

In order to prevent inhibition of binding between the target nucleic acid and the capture nucleic acid, the constituent resin of the shaped body forming the first detecting portion 50a is a water-insoluble resin. In the present invention, water insolubility refers to a substantial water insolubility. Here, a resin is referred to as being substantially water-insoluble when the resin has undergone a mass change in an amount of 1% by mass or less when the resin has been immersed in a large amount of water at 25 degrees C for 24 hours and then sufficiently dried by a method such as vacuum drying. The reason why such a resin is substantially water-insoluble is that the mass change in an amount of 1% by mass or less may be attributed to mass reduction due to leaching of a by-product (e.g., a monomer component) contained in the resin product into the water.

As illustrated in FIG. 1 and FIG. 2, the second detecting portion 50b is disposed to contact the flow path member 30 at a position downstream from the first detecting portion 50a. As illustrated in FIG. 5, the second detecting portion 50b contains a functional group having bindability with the second capture nucleic acid 18 on a surface of the second detecting portion 50b. The second capture nucleic acid 18 contains a functional group bindable with the functional group present on the surface of the second detecting portion 50b. By the functional group, the second capture nucleic acid 18 forms a covalent bond 52 on the surface of the second detecting portion 50b facing the flow path member 30 and is immobilized to the surface. When the gap formed between the facing surfaces of the flow path member 30 and the second detecting portion 50b is filled with the testing target liquid 12, the second capture nucleic acid 18 captures the label body (nucleic acid) 16. As a result, the label body (nucleic acid) 16 is immobilized to develop a color. Hence, the second detecting portion 50b can be used as a control line for indicating that the label body (nucleic acid) 16 has arrived.

In order to prevent inhibition of binding between the label body and the capture nucleic acid, the constituent resin of the shaped body forming the second detecting portion 50b is a water-insoluble resin, like the first detecting portion 50a.

As illustrated in FIG. 1 and FIG. 2, a dropping portion 80 is disposed on an upstream end of the base material 20 to cover the label body (nucleic acid) supplying portion 40.

The absorbing member 70 is disposed on a downstream end of the base material 20 oppositely to the dropping portion 80 to overlap the flow path member 30.

The testing device of the present invention is not limited to a testing device utilizing nucleic acid hybridization and an antibody-antigen reaction. For example, the testing device may be configured to test a specific component contained in the testing target liquid by using as the reagent, a reagent that changes hues in response to a structural change.

As can be known from the conceptual diagram of a membrane of an existing testing device presented in FIG. 6, in the existing testing device, a capture nucleic acid 17 is immobilized to fiber F2 constituting the membrane. Hence, the capture nucleic acid 17 that can be immobilized to the membrane has been limited to a capture nucleic acid having a strong bindability with the fiber F2. That is, in the existing testing device, there have been limitations on usable fiber F2 and usable capture nucleic acid 17 for a design reason. However, in the testing device of the present invention, the shaped bodies and the reagents such as the capture nucleic acids are immobilized to each other by covalent binding at the detecting portions. This is advantageous because it is possible to control the strength of covalent binding between the shaped bodies and the capture nucleic acids and affinity between the shaped bodies and the testing target liquid.

(Transfer medium for producing testing device)

A transfer medium for producing a testing device of the present invention is a medium for producing the testing device of the present invention. The transfer medium for producing a testing device is not particularly limited and may be appropriately selected depending on the intended purpose so long as the transfer medium can form the detecting portion by being transferred onto the flow path. The transfer medium includes a support, a release layer, and a reagent immobilized layer, and may include other layers as needed.

<Support>

The support may be, for example, of any shape, any structure, any size, and any material that are not particularly limited and may be appropriately selected depending on the intended purpose.

The structure of the support is not particularly limited and may be appropriately selected depending on the intended purpose. Examples of the structure of the support include a single-layer structure and a laminated structure.

The size of the support is not particularly limited and may be appropriately selected depending on, for example, the size of the testing device.

The material of the support is not particularly limited and may be appropriately selected depending on the intended purpose. Examples of the material of the support include polyesters such as polyethylene terephthalate (PET) and polyethylene naphthalate (PEN), polycarbonate, polyimide resins (PI), polyamide, polyethylene, polypropylene, polyvinyl chloride, polyvinylidene chloride, polystyrene, styrene-acrylonitrile copolymers, and cellulose acetate. One of these materials may be used alone or two or more of these materials may be used in combination. Among these materials, polyethylene terephthalate (PET) and polyethylene naphthalate (PEN) are particularly preferable.

It is preferable to apply a surface activation treatment to the surface of the support in order to improve close adhesiveness with the layer to be provided on the support. Examples of the surface activation treatment include a glow discharge treatment and a corona discharge treatment.

The support may be kept on, for example, the base material or flow path member side after the reagent immobilized layer described below is transferred onto the base material or the flow path member. Alternatively, the support, etc. may be peeled and removed by means of the release layer described below after the reagent immobilized layer is transferred.

The support is not particularly limited and may be an appropriately synthesized product or a commercially available product.

The average thickness of the support is not particularly limited, may be appropriately selected depending on the intended purpose, and is preferably 3 micrometers or greater but 50 micrometers or less.

<Release layer>

The release layer has a function of improving releasability between the support and the reagent immobilized layer during transfer. The release layer has a function of thermally fusing to become a low-viscosity liquid when heated with a heating/pressing unit such a thermal head and facilitating separation of the reagent immobilized layer at about

the interface between the heated portion and a non-heated portion. The release layer contains a wax and a binder resin, and further contains other components appropriately selected as needed.

The wax is not particularly limited and may be appropriately selected depending on the intended purpose. Examples of the wax include: natural waxes such as a beeswax, a carnauba wax, a cetaceum, a Japan tallow, a candellila wax, a rice bran wax, and a montan wax; synthetic waxes such as a paraffin wax, a microcrystalline wax, an oxide wax, ozokerite, ceresin, an ester wax, a polyethylene wax, and a polyethylene oxide wax; higher fatty acids such as margaric acid, lauric acid, myristic acid, palmitic acid, stearic acid, furoic acid, and behenic acid; higher alcohols such as stearic alcohol and behenyl alcohol; esters such as fatty acid ester of sorbitan; and amides such as stearamide and oleamide. One of these waxes may be used alone or two or more of these waxes may be used in combination. Among these waxes, a carnauba wax and a polyethylene wax are preferable because these waxes have excellent releasability.

The binder resin is not particularly limited and may be appropriately selected depending on the intended purpose. Examples of the binder resin include ethylene-vinyl acetate copolymers, partially saponified ethylene-vinyl acetate copolymers, ethylene-vinyl alcohol copolymers, ethylene-sodium methacrylate copolymers, polyamide, polyester, polyurethane, polyvinyl alcohol, methyl cellulose, carboxymethyl cellulose, starch, polyacrylic acid, isobutylene-maleic acid copolymers, styrene-maleic acid copolymers, polyacrylamide, polyvinyl acetal, polyvinyl chloride, polyvinylidene chloride, isoprene rubbers, styrenebutadiene copolymers, ethylene-propylene copolymers, butyl rubber, and acrylonitrile-butadiene copolymers. One of these binder resins may be used alone or two or more of these binder resins may be used in combination.

The method for forming the release layer is not particularly limited and may be appropriately selected depending on the intended purpose. Examples of the method include a hot-melt coating method, and a method of coating a coating liquid obtained by dispersing the wax and the binder resin in a solvent.

The average thickness of the release layer is not particularly limited, may be appropriately selected depending on the intended purpose, and is preferably 0.5 micrometers or greater but 50 micrometers or less.

The amount of the release layer accumulated is not particularly limited, may be appropriately selected depending on the intended purpose, and is preferably 0.5 g/m$^2$ or greater but 50 g/m$^2$ or less.

<Reagent immobilized layer>

The material, etc. of the reagent immobilized layer are not particularly limited and may be appropriately selected depending on the intended purpose, so long as the reagent immobilized layer contains either one of the constituent resins of the shaped bodies forming the detecting portions of the testing device.

The method for forming the reagent immobilized layer is not particularly limited and may be appropriately selected depending on the intended purpose. For example, the reagent immobilized layer can be formed by a hot-melt coating method, and a method of coating the support or the release layer with a reagent immobilized layer coating liquid obtained by dispersing the constituent resin of the detecting portion in a solvent by a common coating method such as a gravure coater, a wire bar coater, and a roll coater, and drying the coating liquid.

The average thickness of the reagent immobilized layer is not particularly limited, may be appropriately selected depending on the intended purpose, and is preferably 200 nm or greater but 50 micrometers or less. When the average thickness of the reagent immobilized layer is 200 nm or greater, the shaped body will have a good durability and will not be at the risk of being broken by rub or a shock. When the average thickness of the reagent immobilized layer is 50 micrometers or less, heat from a thermal head is conducted uniformly through the reagent immobilized layer, resulting in a good sharpness.

The amount of the reagent immobilized layer coating liquid accumulated on the reagent immobilized layer is not particularly limited, may be appropriately selected depending on the intended purpose, and is preferably, 0.2 g/m$^2$ or greater but 50 g/m$^2$ or less. When the amount of the reagent immobilized layer coating liquid accumulated is 0.2g/m$^2$ or greater, the amount accumulated is sufficient and the detecting portion will not have any lacking portion. When the amount of the reagent immobilized layer coating liquid accumulated is 50 g/m$^2$ or less, drying will not take time and the detecting portion will not have unevenness.

After the reagent immobilized layer coating liquid is dried and the reagent immobilized layer is formed, a surface of the reagent immobilized layer is coated with a liquid in which the capture nucleic acid is dissolved or dispersed, to form a uniform coating film. After the capture nucleic acid is immobilized, the surface to which the capture nucleic acid is immobilized is washed with water containing a surfactant or a buffer solution, to remove unnecessary components. When a functional group reactive with the capture nucleic acid remains on the surface to which the capture nucleic acid is immobilized, it is preferable to perform a treatment for deactivating the functional group remaining on the surface by means of an alkali compound or a compound containing a primary amino group. Through the process described above, the capture nucleic acid can be immobilized to the surface of the reagent immobilized layer. It is preferable to coat the coating film to have a uniform thickness. The drying method is not particularly limited and may be through-flow drying, vacuum drying, natural drying, and freeze drying. However, drying at a reduced pressure or in vacuum is preferable. It

is preferable to perform drying at a drying temperature in a room temperature range of from 20 degrees C through 50 degrees C for a drying time of from 30 minutes through 24 hours. When the drying temperature is higher than 20 degrees C, the time taken for drying is saved and productivity is improved as a result. When the drying temperature is lower than 50 degrees C, there is no risk of the reagent being denatured by heat. When the drying time is longer than 30 minutes, drying is sufficient. When the drying time is shorter than 24 hours, productivity is improved and there is no need of considering discoloration of the resin.

<Other layers>

The other layers are not particularly limited and may be appropriately selected depending on the intended purpose. Examples of the other layers include a back layer, an undercoat layer, and a protective film.

-Back layer-

The back layer is not particularly limited and may be appropriately selected depending on the intended purpose. It is preferable that the transfer medium be provided with the back layer over a surface of the support opposite to a surface of the support provided with the release layer. During transfer, heat is directly applied to the opposite surface by, for example, a thermal head in a manner to conform to the shape of the shaped body to form the detecting portion. Hence, it is preferable that the back layer have resistance to a high heat and resistance to rub with, for example, a thermal head. The back layer contains a binder resin, and further contains other components as needed.

The binder resin is not particularly limited and may be appropriately selected depending on the intended purpose. Examples of the binder resin include silicone-modified urethane resins, silicone-modified acrylic resins, silicone resins, silicone rubbers, fluororesins, polyimide resins, epoxy resins, phenol resins, melamine resins, and nitrocellulose. One of these binder resins may be used alone or two or more of these binder resins may be used in combination.

The other components are not particularly limited and may be appropriately selected depending on the intended purpose. Examples of the other components include inorganic particles of, for example, talc, silica, and organopolysiloxane, and a lubricant.

The method for forming the back layer is not particularly limited and may be appropriately selected depending on the intended purpose. Examples of the method include common coating methods such as a gravure coater, a wire bar coater, and a roll coater.

The average thickness of the back layer is not particularly limited, may be appropriately selected depending on the intended purpose, and is preferably 0.01 micrometers or greater but 1.0 micrometer or less.

-Undercoat layer-

The undercoat layer may be provided between the support and the release layer, or between the release layer and the reagent immobilized layer. The undercoat layer contains a resin, and further contains other components as needed. The resin is not particularly limited and may be appropriately selected depending on the intended purpose. The various resins used in the reagent immobilized layer and the release layer may be used.

-Protective film-

It is preferable to provide a protective film over the reagent immobilized layer in order to protect the reagent immobilized layer from contamination or damages during storage. The material of the protective film is not particularly limited and may be appropriately selected depending on the intended purpose so long as the material can be easily peeled from the reagent immobilized layer. Examples of the material of the protective film include silicone paper, polyolefin sheets such as polypropylene, and polytetrafluoroethylene sheet. The average thickness of the protective film is not particularly limited, may be appropriately selected depending on the intended purpose, and is preferably 5 micrometers or greater but 100 micrometers or less and more preferably 10 micrometers or greater but 30 micrometers or less.

Hitherto, when a resin shaped body to which the capture nucleic acid is immobilized is produced in the form of a transfer medium for producing a testing device, there is a need of storing the transfer medium by winding the transfer medium around a core into a roll form in a multi-laminated state. If the force of binding between the capture nucleic acid and the resin shaped body is weak such as when the capture nucleic acid is physisorbed to the resin shaped body, it is likely that the reagent will come off to the back of the transfer medium (the back being the side opposite to the surface over which the reagent is immobilized). This raises a problem that the storage stability is low.

Meanwhile, in the present invention, the capture nucleic acid is immobilized by covalent binding. This makes it harder for the reagent to come off to the back of the transfer medium even when the transfer medium is laminated. This is advantageous in that the storage stability is high.

(Method for producing testing device)

A method for producing a testing device of the present invention includes a step of bringing the reagent immobilized layer of the transfer medium and the flow path member into contact with each other to transfer the reagent immobilized layer onto the flow path member, and further includes other steps as needed.
The step of transferring the reagent immobilized layer onto the flow path member is not particularly limited and may be appropriately selected depending on the intended purpose. Examples of the step includes a step using a method of thermally transferring the reagent immobilized layer onto the flow path member.
Transferring of the reagent immobilized layer, which is the method for producing a testing device, will be described in detail with reference to a drawing.
FIG. 7 is a cross-sectional view illustrating a layered state of a transfer medium used for a testing device. Transferring of the reagent immobilized layer is not particularly limited by, for example, the structure of the flow path so long as the reagent immobilized layer is transferred onto the flow path. Hence, the reagent immobilized layer may be formed in an appropriate manner depending on the intended purpose. Here, a method for thermally transferring the reagent immobilized layer onto the flow path member will be described.
As illustrated in FIG. 7, a transfer medium 100 includes a support 101, a release layer 102, and a reagent immobilized layer 103 in a layered state in the order of reciting, and further includes a back layer 104 over a surface of the support 101 opposite to the surface over which the release layer 102 is layered.
Examples of the method for thermally transferring the reagent immobilized layer 103 onto the flow path member 30 include a method including a step of bringing the reagent immobilized layer 103 of the transfer medium 100 and the flow path member 30 into contact with each other to transfer the reagent immobilized layer 103 onto the flow path member 30. A printer used for thermal transfer is not particularly limited and may be appropriately selected depending on the intended purpose. Examples of the printer include thermal printers including, for example, a serial thermal head and a line thermal head. The energy applied for thermal transfer is not particularly limited, may be appropriately selected depending on the intended purpose, and is preferably 0.05 mJ/dot or higher but 0.5 mJ/dot or lower. When the applied energy is 0.05 mJ/dot or higher, the reagent immobilized layer 103 is fused sufficiently. When the applied energy is 0.5 mJ/dot or lower, there is no risk of the reagent being denatured by heat, and there is no risk of the other portions of the transfer medium 100 than the reagent immobilized layer 103 being fused. This prevents the thermal head from being contaminated.

<Applications of testing device>

Applications of the testing device are not particularly limited and may be appropriately selected depending on the intended purpose. Examples of the applications of the testing device include lateral flow chromatographic devices for nucleic acid detection, biochemical sensors (sensing chips) for blood testing and DNA testing, and small-size analytical devices (chemical sensors) for, for example, quality control of foods and beverages. Among these applications, the testing device is preferable as a lateral flow chromatographic device for nucleic acid detection.
The lateral flow chromatographic device for nucleic acid detection can accurately diagnose, for example, infectious diseases, hereditary diseases such as tumors, and predispositions by checking presence or absence of nucleic acids (DNAs and RNAs) attributable to viruses or bacteria or presence or absence of nucleic acids attributable to mutant genes related to specific diseases or predispositions. The lateral flow chromatographic device for nucleic acid detection is also used for food inspections for detecting allergens or specific foods and environmental surveys for detecting microorganisms existing in the environment.
Samples (analytes) used in biochemical testings are not particularly limited and may be appropriately selected depending on the intended purpose, so long as the samples contain a single-stranded nucleic acid that is the target. Examples of the samples include pathogens such as bacteria and viruses, blood, saliva, lesional tissues, etc. separated from living organisms, and excretion such as enteruria. Further, for performing a prenatal diagnosis, the sample may be a part of a fetus cell in an amniotic fluid or a part of a dividing egg cell in a test tube.
Furthermore, these samples may be condensed to a sediment directly or by, for example, centrifugation as needed, and then subjected to a pretreatment such as nucleic acid extraction treatment, nucleic acid amplification, and modification or any combinations of these treatments.
The testing device also has a function of chromatographing (separating or refining) a testing target liquid because the flow path member functions as a static bed. In this case, the flow path member including the continuous cells of which internal wall has hydrophilicity functions as the static bed (or a support). Different components in the testing target liquid flow through the flow path at different speeds because of the difference in the interaction with the static bed during the process of permeating the flow path, i.e., the difference in whether the components are hydrophilic or hydrophobic.
A component having a higher hydrophilicity adsorbs to the porous portion functioning as the static bed more

easily, and repeats adsorbing and desorbing more times, resulting in a lower permeating speed through the flow path. In contrast, a component having a higher hydrophobicity permeates the flow path without adsorbing to the static bed, and hence moves through the flow path more quickly. By extracting the target component in the testing target liquid selectively based on the difference in the moving speed in the testing target liquid and letting the target component undergo a reaction, it is possible to use the testing device of the present invention as a highly functional chemical or biochemical sensor.

(Testing method)

A testing method of the present invention is not particularly limited so long as the testing method is a testing method for performing testing using the testing device of the present invention, and may include an analyte supplying step of supplying an analyte to the flow path member and a step of capturing a part of the analyte by the capture nucleic acid immobilized to the shaped body.

In a specific operation, the testing target liquid 12 is dropped and supplied onto the dropping portion 80 (see FIG. 1 and FIG. 2) provided on the flow path member 30 of the testing device 10. Subsequently, the supplied testing target liquid 12 and the label body (nucleic acid) 16 supported by the label body (nucleic acid) supplying portion 40 are brought into contact with each other, to release the label body (nucleic acid) 16 from the label body (nucleic acid) supplying portion 40. When any target nucleic acid 14 is contained in the testing target liquid 12, the label body (nucleic acid) 16 released from the label body (nucleic acid) supplying portion 40 reacts and binds with the target nucleic acid 14.

Subsequently, the testing target liquid 12 containing the label body (nucleic acid) 16 and the target nucleic acid 14 is spread along the flow path member 30 to arrive at the region at which the first detecting portion 50a is disposed. The first nucleic acid 17 immobilized to the surface of the first detecting portion 50a facing the flow path member 30 binds with and captures the target nucleic acid 14 that is in the state of being bound with the label body (nucleic acid) 16. As described above, the first capture nucleic acid 17 is immobilized to the shaped body by the covalent bond 52. Therefore, even when the first capture nucleic acid 17 contacts the testing target liquid 12, the first capture nucleic acid 17 does not come to have affinity with the testing target liquid 12 and is not easily released into the testing target liquid 12. Even if some part of the first capture nucleic acid 17 is released into the testing target liquid 12, the released part gets bound with the fiber constituting the flow path member 30 soon. This facilitates immobilization of the label body (nucleic acid) 16 to about the first detecting portion 50a. As a result, the first detecting portion 50a functioning as the test line develops a color clearly.

Any label body (nucleic acid) 16 that passes by the first detecting portion 50a without being captured is spread along the flow path member 30 to arrive at the region at which the second detecting portion 50b is disposed. At the second detecting portion 50b, the label body (nucleic acid) 16 is captured by being bound with the second capture nucleic acid 18. Because the second capture nucleic acid 18 is immobilized, the second detecting portion 50b functioning as the control line develops a color clearly.

(Testing kit)

A testing kit of the present invention includes the testing device of the present invention and an analyte collecting unit configured to collect an analyte, and further includes other members as needed.

For performing testing according to the testing method of the present invention described above, it is possible to use a testing kit including the testing device 10, and at least one of a tool configured to collect an analyte (an example of the analyte collecting unit) and a liquid for treating the analyte as illustrated in FIG. 8.

Examples of the tool configured to collect an analyte include known tools such as a sterilized cotton swab 201 for collecting an analyte from, for example, pharynx and nasal cavity. Examples of the liquid for treating the analyte include known liquids such as a diluting fluid 202 for diluting the analyte and an extraction liquid for extracting the analyte.

In the embodiment described above, a case where the reagents supplied from the label body (nucleic acid) supplying portion and the detecting portions are nucleic acids is described. The present invention is not limited to this embodiment. An indicator used in a chemical assay refers to a reagent for indicating a chemical property of a solution.

The indicator is not particularly limited and may be appropriately selected depending on the intended purpose. Examples of the indicator include pH indicators, various ionophores that discolor by reacting with various ions such as a lead ion, a copper ion, and a nitrite ion, and reagents that discolor by reacting with various agricultural chemicals.

In the embodiment described above, a case where the support 101 and the reagent immobilized layer 103 of the transfer medium 100 are separated from each other by heat during transfer is described. The present invention is not limited to this embodiment. For example, the support 101 and the reagent immobilized layer 103 may be separated from each other by light. In this case, the release layer 102 may contain a light absorber such as carbon black and may make the light absorber absorb light and generate heat, so the release layer 102 fuses and releases the reagent immobilized layer 103. Alternatively, the release layer 102 may contain a material that changes properties in response to light

irradiation and may make the material absorb light, so the release layer 102 becomes fragile to release the reagent immobilized layer 103.

In the embodiment described above, a case where the flow path is formed throughout the flow path member 30 is described. The present invention is not limited to this embodiment. Examples of the method for forming a flow path in a partial region of the flow path member 30 include a method of forming a flow path wall defining the external edge of the flow path by filling the voids of a hydrophilic porous material with a hydrophobic material according to a known method.

The testing device 10 of the present embodiment may be provided with an arbitrary protective member configured to prevent a hand from being contaminated by touching the flow path member 30. Examples of the protective member include a housing configured to cover the whole of the testing device 10, and a film provided over the flow path member 30. When providing the protective member, it is preferable to provide an opening at a position above the dropping portion 80 on the flow path member 30. It is also preferable to provide an opening in the protective member in order to dissipate the internal pressure in the flow path.

In the embodiment described above, a case where the testing target liquid is hydrophilic is described. The present invention is not limited to this embodiment. For example, the testing target liquid may be lipophilic or solvophilic. Examples of solvophilic testing target liquids include solvophilic liquids containing organic solvents such as alcohols such as methyl alcohol, ethyl alcohol, 1-propyl alcohol, and 2-propyl alcohol, and ketones such as acetone and methyl ethyl ketone (MEK). When the testing target liquid is solvophilic, the term "hydrophilic" in the embodiment described above is replaced by "hydrophobic", and the term "hydrophobic" is replaced by "hydrophilic".

[Examples]

The present invention will be described below by way of Examples. However, the present invention should not be construed as being limited to these Examples.

(Preparation example 1)

-Preparation of back layer coating liquid-

A silicone-based rubber emulsion (available from Shin-Etsu Chemical Co., Ltd., KS779H, with a solid concentration of 30% by mass) (16.8 parts by mass), a chloroplatinic acid catalyst (0.2 parts by mass), and toluene (83 parts by mass) were mixed, to obtain a back layer coating liquid.

(Preparation example 2)

-Preparation of release layer coating liquid-

A polyethylene wax (available from Toyo ADL Corporation, POLYWAX 1000, with a melting point of 99 degrees C and a penetration of 2 at 25 degrees C) (14 parts by mass), an ethylene-vinyl acetate copolymer (available from Du Pont-Mitsui Polychemicals Co., Ltd., EV-150, with a weight average molecular weight of 2, 100, and VAc of 21% by mass) (6 parts by mass), toluene (60 parts by mass), and methyl ethyl ketone (20 parts by mass) were subjected to dispersion treatment until the average particle diameter became 2.5 micrometers, to obtain a release layer coating liquid.

(Preparation example 3)

-Preparation of reagent immobilized layer coating liquid-

An aminoethylated acrylic polymer (POLYMENT NK-380, available from Nippon Shokubai Co., Ltd.) was diluted to 15% by mass by addition of toluene as a solvent, to obtain a reagent immobilized layer coating liquid.

(Preparation example 4)

-Preparation of test line reagent coating liquid-

A DNA fragment that continuously contained 20 bases of thymine (T) and to which a thiol group was introduced at a 5' end was prepared to have a final concentration of 2.5 $\mu$M with a sodium phosphate buffer having a final concentration of 100 mM (pH=7.2) and EDTA having a final concentration of 5 mM. To the resultant, SULFO-GMBS (available from Thermo Fisher Scientific Inc.) having a final concentration of 1 mM was added as a linker, to obtain a test line

reagent coating liquid.

(Preparation example 5)

-Preparation of control line reagent coating liquid-

A DNA fragment that continuously contained 20 bases of cytosine (C) and to which a thiol group was introduced at a 3' end was prepared to have a final concentration of 2.5 $\mu$ M with a sodium phosphate buffer having a final concentration of 100 mM (pH=7.2) and EDTA having a final concentration of 5 mM. To the resultant, SULFO-GMBS (available from Thermo Fisher Scientific Inc.) having a final concentration of 1 mM was added as a linker, to obtain a control line reagent coating liquid.

(Preparation example 6)

-Preparation of label body (nucleic acid) reagent coating liquid-

Gold colloid modified with a carboxyl group was bound by amide binding with a DNA fragment that continuously contained 20 bases of guanine (G) and to which an amino group was introduced at a 3' end using 1-ethyl-3-(3-dimethylaminopropyl)carbodiimide (EDC) (available from Thermo Fisher Scientific Inc.). The resultant was washed with a 50mM Tris-HCl buffer (pH=8.2), suspended in a label body diluting fluid (a 20mM Tris-HCl buffer (pH=8.2), 0.05% by mass polyethylene glycol, 5% by mass sucrose, and purified water), and adjusted to OD=2, to obtain a label body reagent coating liquid.

(Preparation example 7)

-Preparation of test line reagent coating liquid-

A DNA fragment that continuously contained 20 bases of thymine (T) and to which a thiol group was introduced at a 5' end was prepared to have a final concentration of 2.5 $\mu$ M with a sodium phosphate buffer having a final concentration of 100 mM (pH=7.2) and EDTA having a final concentration of 5 mM. To the resultant, SM (PEG) 4 (available from Thermo Fisher Scientific Inc.) having a final concentration of 1 mM was added as a linker, to obtain a test line reagent coating liquid.

(Preparation example 8)

-Preparation of control line reagent coating liquid-

A DNA fragment that continuously contained 20 bases of cytosine (C) and to which a thiol group was introduced at a 3' end was prepared to have a final concentration of 2.5 $\mu$M with a sodium phosphate buffer having a final concentration of 100 mM (pH=7.2) and EDTA having a final concentration of 5 mM. To the resultant, SM (PEG) 4 (available from Thermo Fisher Scientific Inc.) having a final concentration of 1 mM was added as a linker, to obtain a control line reagent coating liquid.

(Preparation example 9)

-Preparation of test line reagent coating liquid-

A DNA fragment that continuously contained 20 bases of thymine (T) and to which a thiol group was introduced at a 5' end was prepared to be 25 $\mu$ M with a TE buffer (10 mM Tris-HCl and 1 mM EDTA, pH=7.4, available from Takara Bio Inc.), to obtain a test line reagent coating liquid.

(Preparation example 10)

-Preparation of control line reagent coating liquid-

A DNA fragment that continuously contained 20 bases of cytosine (C) and to which a thiol group was introduced at a 3' end was prepared to be 25 $\mu$ M with a TE buffer (10 mM Tris-HCl and 1 mM EDTA, pH=7.4, available from Takara Bio Inc.), to obtain a control line reagent coating liquid.

(Preparation example 11)

-Preparation of label body (antibody) reagent coating liquid-

To a gold colloid solution (available from BBI Solutions, EMGC50) (9 mL), a $KH_2PO_4$ buffer (pH=7.0) (1 mL) prepared to 50 mM and then an anti-biotin monoclonal antibody (available from Bethyl Laboratories, Inc., ANTI-BIOTIN, GOAT-POLY A150-111A) (1 mL) prepared to 50 micrograms/mL were added and stirred. The resultant was left to stand still for 10 minutes. To the resultant, a 1% by mass polyethylene glycol aqueous solution (available from Wako Pure Chemical Industries, Ltd., 168-11285) (550 microliters) was added and stirred. To the resultant, a 10% by mass BSA aqueous solution (available from Sigma-Aldrich Co., LLC, A-7906) (1.1 mL) was further added and stirred.

Next, this solution was subjected to centrifugation for 30 minutes, and then, after the supernatant being removed, subjected to re-dispersion of the gold colloid using an ultrasonic cleaner. The centrifugation was performed with a centrifuge (available from Hitachi Koki Co., Ltd., HIMAC CF16RN) at a centrifugal acceleration of 8,000 × g at 4 degrees C. Subsequently, the resultant was dispersed in a gold colloid preservative solution [a 20 mM Tris-HCl buffer (pH=8.2), 0.05% by mass polyethylene glycol (with a weight average molecular weight of 2,000), 150 mM NaCl, a 1% by mass BSA aqueous solution, and a 0.1% by mass $NaN_3$ aqueous solution] (20 mL), subjected again to centrifugation under the same conditions as described above, and after the supernatant being removed except about 1 mL, subjected to re-dispersion of the gold colloid using an ultrasonic cleaner. These operations were repeated to prepare the solution to be OD=15 in the gold colloid preservative solution, to obtain a label body reagent coating liquid.

(Preparation example 12)

-Preparation of test line reagent coating liquid-

A DNA fragment that continuously contained 20 bases of thymine (T) and to which an N-hydroxysuccinimide ester group was introduced at a 5' end was prepared to have a final concentration of 2.5 $\mu$M with a sodium phosphate buffer having a final concentration of 100 mM (pH=7.2) and EDTA having a final concentration of 5 mM, to obtain a test line reagent coating liquid.

(Preparation example 13)

-Preparation of control line reagent coating liquid-

A DNA fragment that continuously contained 20 bases of cytosine (C) and to which an N-hydroxysuccinimide ester group was introduced at a 3' end was prepared to have a final concentration of 2.5 $\mu$M with a sodium phosphate buffer having a final concentration of 100 mM (pH=7.2) and EDTA having a final concentration of 5 mM, to obtain a control line reagent coating liquid.

(Preparation example 14)

-Preparation of test line reagent coating liquid-

A DNA fragment that continuously contained 20 bases of thymine (T) and to which a thiol group was introduced at a 5' end was prepared to have a final concentration of 2.5 $\mu$M with a sodium phosphate buffer having a final concentration of 100 mM (pH=7.2) and EDTA having a final concentration of 5 mM, to obtain a test line reagent coating liquid.

(Preparation example 15)

-Preparation of control line reagent coating liquid-

A DNA fragment that continuously contained 20 bases of cytosine (C) and to which a thiol group was introduced at a 3' end was prepared to have a final concentration of 2.5 $\mu$M with a sodium phosphate buffer having a final concentration of 100 mM (pH=7.2) and EDTA having a final concentration of 5 mM, to obtain a control line reagent coating liquid.

(Preparation example 16)

-Preparation of reagent immobilized layer coating liquid-

An aziridine derivative (CL2502, available from Arakawa Chemical Industries, Ltd.), which was an amino group-containing ethyleneimine resin, was mixed with a curing catalyst (ACS-164, available from Arakawa Chemical Industries, Ltd.) at a ratio of 100:4 (as a mass ratio), to obtain a reagent immobilized layer coating liquid.

(Preparation example 17)

-Preparation of reagent immobilized layer coating liquid-

A carboxyl group-containing ester resin (AP2510, available from Arakawa Chemical Industries, Ltd.) was diluted to 15% by mass by addition of a solvent, which was a methyl ethyl ketone/toluene mixed liquid, (at a ratio by volume of 6:4), to obtain a reagent immobilized layer coating liquid.

(Preparation example 18)

-Preparation of reagent immobilized layer coating liquid-

A hydroxyl group-containing acrylic resin (DA105, available from Arakawa Chemical Industries, Ltd.) was mixed with a curing agent (CL102H, available from Arakawa Chemical Industries, Ltd.) at a ratio of 10:3.8 (as a mass ratio), to obtain a reagent immobilized layer coating liquid.

(Preparation example 19)

-Preparation of test line reagent coating liquid-

A DNA fragment that continuously contained 20 bases of thymine (T) and to which an amino group was introduced at a 5' end was prepared to have a final concentration of 2.5 μ M with a sodium phosphate buffer having a final concentration of 100 mM (pH=7.2) and EDTA having a final concentration of 5 mM. To the resultant, DSG (available from Thermo Fisher Scientific Inc.) having a final concentration of 1 mM was added as a linker, to obtain a test line reagent coating liquid.

(Preparation example 20)

-Preparation of control line reagent coating liquid-

A DNA fragment that continuously contained 20 bases of cytosine (C) and to which an amino group was introduced at a 3' end was prepared to have a final concentration of 2.5 μ M with a sodium phosphate buffer having a final concentration of 100 mM (pH=7.2) and EDTA having a final concentration of 5 mM. To the resultant, DSG (available from Thermo Fisher Scientific Inc.) having a final concentration of 1 mM was added as a linker, to obtain a control line reagent coating liquid.

(Preparation example 21)

-Preparation of test line reagent coating liquid-

A DNA fragment that continuously contained 20 bases of thymine (T) and to which a carboxyl group was introduced at a 5' end was prepared to have a final concentration of 2.5 μ M with a MES buffer (available from Dojindo Laboratories) having a final concentration of 100 mM. To the resultant, EDC (available from Thermo Fisher Scientific Inc.) having a final concentration of 1.25 mg/mL was added as a linker, to obtain a test line reagent coating liquid.

(Preparation example 22)

-Preparation of control line reagent coating liquid-

A DNA fragment that continuously contained 20 bases of cytosine (C) and to which a carboxyl group was

introduced at a 3' end was prepared to have a final concentration of 2.5 $\mu$M with a MES buffer (available from Dojindo Laboratories) having a final concentration of 100 mM. To the resultant, EDC (available from Thermo Fisher Scientific Inc.) having a final concentration of 1.25 mg/mL was added as a linker, to obtain a control line reagent coating liquid.

(Preparation example 23)

-Preparation of test line reagent coating liquid-

A DNA fragment that continuously contained 20 bases of thymine (T) and to which an amino group was introduced at a 5' end was prepared to have a final concentration of 2.5 $\mu$M with a MES buffer (available from Dojindo Laboratories) having a final concentration of 100 mM. To the resultant, EDC (available from Thermo Fisher Scientific Inc.) having a final concentration of 1.25 mg/mL was added as a linker, to obtain a test line reagent coating liquid.

(Preparation example 24)

-Preparation of control line reagent coating liquid-

A DNA fragment that continuously contained 20 bases of cytosine (C) and to which an amino group was introduced at a 3' end was prepared to have a final concentration of 2.5 $\mu$M with a MES buffer (available from Dojindo Laboratories) having a final concentration of 100 mM. To the resultant, EDC (available from Thermo Fisher Scientific Inc.) having a final concentration of 1.25 mg/mL was added as a linker, to obtain a control line reagent coating liquid.

(Preparation example 25)

-Preparation of test line reagent coating liquid-

A DNA fragment that continuously contained 20 bases of thymine (T) and to which a thiol group was introduced at a 5' end was prepared to have a final concentration of 2.5 $\mu$M with a boric acid buffer having a final concentration of 100 mM (pH=8.5). To the resultant, PMPI (available from Thermo Fisher Scientific Inc.) having a final concentration of 1 mM was added as a linker, to obtain a test line reagent coating liquid.

(Preparation example 26)

-Preparation of control line reagent coating liquid-

A DNA fragment that continuously contained 20 bases of cytosine (C) and to which a thiol group was introduced at a 3' end was prepared to have a final concentration of 2.5 $\mu$M with a boric acid buffer having a final concentration of 100 mM (pH=8.5). To the resultant, PMPI (available from Thermo Fisher Scientific Inc.) having a final concentration of 1 mM was added as a linker, to obtain a control line reagent coating liquid.

(Example 1)

<Production of transfer medium for test line>

-Formation of back layer-

The back layer coating liquid of Preparation example 1 was coated over one surface of a support, which was a polyethylene terephthalate (PET) film having an average thickness of 4.5 micrometers (available from Toray Industries, Inc. LUMIRROR F57) and dried at 80 degrees C for 10 seconds, to form a back layer having an average thickness of 0.02 micrometers.

-Formation of release layer-

Next, the release layer coating liquid of Preparation example 2 was coated over a surface of the PET film opposite to the surface over which the back layer was formed and dried at 25 degrees C for 30 minutes, to form a release layer having an average thickness of 30 micrometers.

-Formation of reagent immobilized layer-

Next, the reagent immobilized layer coating liquid of Preparation example 3 was coated over the surface of the release layer and dried at 50 degrees C for 30 minutes, to form a reagent immobilized layer having an average thickness of 3 micrometers. In this way, a transfer medium was produced.

<Immobilization of capture nucleic acids>

-Test line (immobilization of first capture nucleic acid)-

Next, the test line reagent coating liquid of Preparation example 4 was poured into a shallow square vat, and the transfer medium was floated over the test line reagent coating liquid such that only the reagent immobilized layer surface contacted the test line reagent coating liquid. In this state, the shallow square vat was covered with a lid and left to stand still at 25 degrees C for 30 minutes. Subsequently, the reagent immobilized layer surface was washed with a 50 mM Tris-HCl buffer (pH=9.0) and subjected to vacuum drying at 25 degrees C for 30 minutes, to immobilize the reagent to the reagent immobilized layer. In this way, a transfer medium for a test line of Example 1 was produced.

It was confirmed that the first capture nucleic acid and the reagent immobilized layer were covalently bound with each other by a FT-IR ATR method (FT-IR6800, available from JASCO Corporation) based on presence or absence of a spectrum attributable to a thioether bond produced by the thiol group in the first capture nucleic acid newly forming a thioether bond (covalent bond) with the maleimide group at one end of the linker and presence or absence of a spectrum attributable to an amide bond produced by the amino group in the reagent immobilized layer newly forming an amide bond (covalent bond) with the N-hydroxysulfosuccinimide ester group at another end of the linker.

<Production of transfer medium for control line>

-Control line (immobilization of second capture nucleic acid)-

A transfer medium for a control line of Example 1 was produced in the same manner as in <Production of transfer medium for test line> described above, except that unlike in <Production of transfer medium for test line>, the control line reagent coating liquid of Preparation example 5 was used instead of the test line reagent coating liquid.

It was confirmed that the second capture nucleic acid and the reagent immobilized layer were covalently bound with each other by a FT-IR ATR method (FT-IR6800, available from JASCO Corporation) based on presence or absence of a spectrum attributable to a thioether bond produced by the thiol group in the second capture nucleic acid newly forming a thioether bond (covalent bond) with the maleimide group at one end of the linker and presence or absence of a spectrum attributable to an amide bond produced by the amino group in the reagent immobilized layer newly forming an amide bond (covalent bond) with the N-hydroxysulfosuccinimide ester group at another end of the linker.

<Production of testing device>

The testing device 10 illustrated in FIG. 1 and FIG. 2 was produced in the manner described below. FIG. 1 is a top view of the testing device of Example. FIG. 2 is a schematic cross-sectional view of the testing device of FIG. 1 taken along a line A-A.

-Production of paper substrate (substrate+flow path member)-

As a thermoplastic resin, a polyester-based hot-melt adhesive (available from Toagosei Co., Ltd., ARONMELT PES375S40) was heated to 190 degrees C, and then with a roll coater, coated over a PET film (available from Toray Industries, Inc., LUMIRROR S10, with an average thickness of 50 micrometers) 20 cut into a size of 40 mm in width and 80 mm in length to have an average thickness of 50 micrometers over the PET film, to form an adhesive layer.

The PET film 20 over which the adhesive layer was formed was left to stand still for 2 hours or longer. Subsequently, a nitrocellulose membrane (available from Merck Millipore Corporation, HF180) cut into a size of 40 mm in width and 35 mm in length was overlapped with the surface of the adhesive layer at a position that was 33 mm from one end of the surface of the adhesive layer in the longer direction (this end being an upstream end, the opposite end being a downstream end) in a state that the nitrocellulose membrane and the surface of the adhesive layer coincided widthwise, and a load of 1 $kgf/cm^2$ was imposed on the overlapped product at a temperature of 150 degrees C for 10 seconds, to form a flow path member 30. Finally, the obtained product was cut along the longer direction of the product into a size of 4 mm in width and 80 mm in length, to obtain a paper substrate.

The voidage of the nitrocellulose membrane as the flow path member 30 of the paper substrate was calculated

according to a calculation formula 1 below based on a basis weight (g/m$^2$) and an average thickness (micrometer) of the flow path member and the specific gravity of the component of the flow path member. As a result, the voidage of the nitrocellulose membrane was 70%.

<Calculation formula 1>

$$\text{Voidage (\%)} = \{1 - [\text{basis weight (g/m}^2\text{)/average thickness (micrometer)/specific gravity of the component}]\} \times 100$$

When the voidage of the flow path member is 40% or greater but 90% or less, the flow path member can be said to be porous.

-Transfer of test line-

The flow path member 30 of the paper substrate and the reagent-immobilized side of the transfer medium for a test line were faced and overlapped with each other. Subsequently, with a thermal transfer printer, as illustrated in FIG. 1 and FIG. 2, the transfer medium for test a line was transferred onto a position that was apart by 9 mm from the upstream end of the flow path member 30 in a line shape having a width of 4 mm and a length of 0.7 mm (first detecting portion 50a).

As the thermal transfer printer, an evaluation system having a printing speed of 42 mm/sec and an applied energy of 0.17 mJ/dot was constructed with a thermal head having a dot density of 300 dpi (available from TDK Corporation).

-Transfer of control line-

Next, the transfer medium for a control line was transferred onto a position that was apart by 5 mm from the position onto which the transfer medium for a test line was transferred in a line shape having a width of 4 mm and a length of 0.7 mm (second detecting portion 50b).

-Formation of label body supplying portion-

Next, the label body reagent coating liquid of Preparation example 6 was coated in an amount of 60 microliters/cm$^2$ over a glass-fiber pad (available from Merck Millipore Corporation, GFCP203000) cut into a size of 4 mm in width and 18 mm in length, and dried overnight at a reduced pressure to produce a label body supporting pad.

As illustrated in FIG. 1 and FIG. 2, the label body supporting pad was disposed at a position that was apart by 17 mm from the upstream end of the paper substrate, and overlapped with and pasted over the adhesive layer provided over the paper substrate (label body supplying portion 40).

-Formation of dropping portion-

As illustrated in FIG. 1 and FIG. 2, a sample pad (available from Merck Millipore Corporation, CFSP223000) having a width of 4 mm and a length of 35 mm was disposed and pasted in a manner to overlap with the upper surface of the label body supplying portion 40 by 18 mm (dropping portion 80).

-Absorbing member-

An absorbing member 70 (available from Merck Millipore Corporation, CFSP223000) was provided as illustrated in FIG. 1 and FIG. 2. In this way, a lateral flow chromatographic device for nucleic acid detection (testing device 10) of Example 1 was obtained.

<Evaluation of lines>

-Preparation of testing target liquid-

A DNA fragment incluing a base sequence that contained from a 5' end, 20 bases of cytosine (C) continuously,

a sequence containing 10 bases of thymine (T) and guanine (G) repeatedly, and 20 bases of adenine (A) continuously was prepared to have a final concentration of 1.0 $\mu$M with a 5 × SSC buffer (75 mM sodium citrate and 750 mM sodium chloride available from Nacalai Tesque, Inc.), to obtain a testing target liquid.

- Reaction-

The testing target liquid was dropped in an amount of 100 microliters onto the upstream end portion of the lateral flow chromatographic device 10 for nucleic acid detection illustrated in FIG. 1 and FIG. 2. Thirty minutes later, the lines were evaluated according to the criteria described below based on visual observation. The result is presented in Table 2.

<Evaluation criteria>

A: A clear color development was recognized at the positions of the test line and the control line at a uniform color optical density throughout the lines without discontinuation of the lines.
B: The lines were not discontinuous and enabled judgement but were slightly non-uniform in the color optical density from place to place.
C: Color development was barely recognized as line shapes but with a partial discontinuation in the lines.
D: No color development was recognized or color development was not in line shapes such as when the lines flowed to the downstream side.

Examples of the evaluation criteria are presented in Table 1. The photographs in Table 1 are each a photograph of the test line after testing.

[Table 1]

| Evaluation criteria | A | B | C | D |
|---|---|---|---|---|
| Photograph | 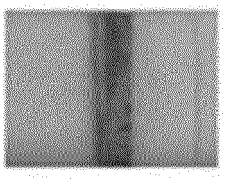 | 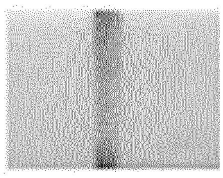 | 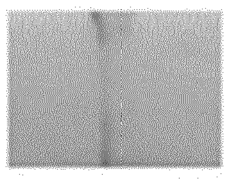 | 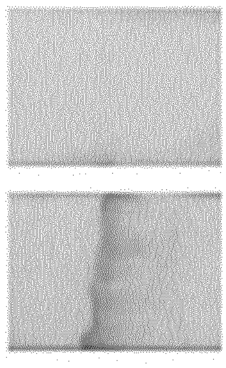 |

<Measurement of density of lines>

The lateral flow chromatographic device for nucleic acid detection after having developed colors and used in the evaluation of lines above was measured with a chromatoreader (available from Hamamatsu Photonics K.K., C 10066), to obtain the optical density of the lines and S/N ratio, which were evaluated according to the criteria described below. The results are presented in Table 3. A greater optical density of the lines is more preferable, and a greater S/N ratio is more preferable because the contours of the lines were clearer. When no line optical density was detected by the chromatoreader, the result is presented as "-".

<Evaluation criteria for optical density of lines>

A: The optical density was 200 or greater.
B: The optical density was 100 or greater but less than 200.

C: The optical density was 30 or greater but less than 100.
D: The optical density was less than 30.
E: The optical density was unmeasurable because no lines were recognized.

<Evaluation criteria for S/N ratio>

A: The S/N ratio was 30 or greater.
B: The S/N ratio was 10 or greater but less than 30.
C: The S/N ratio was 3 or greater but less than 10.
D: The S/N ratio was less than 3.
E: It was impossible to calculate the S/N ratio.

<Criteria for total evaluation>

The achieved grades were converted into numbers in a manner that A was 5 points, B was 4 points, C was 3 points, D was 2 points, and E was 1 point, to perform total evaluation based on the total of the grades achieved in the evaluation f lines, the evaluation of optical density, and the evaluation of S/N ratio.

S: Extraordinarily excellent: 15 points or greater
A: Excellent: 14 points or greater but less than 15 points
B: Good: 13 points or greater but less than 14 points
C: Ordinary: 12 points or greater but less than 13 points
D: Slightly bad but passable: 11 points or greater but less than 12 points
E: Unpassable with no visibility: less than 11 points

(Example 2)

A lateral flow chromatographic device for nucleic acid detection (testing device 10) of Example 2 was produced in the same manner as in Example 1, except that unlike in Example 1, the test line reagent coating liquid of Preparation example 7 was used in <Production of transfer medium for test line> and the control line reagent coating liquid of Preparation example 8 was used in <Production of transfer medium for control line>, and was evaluated in the same manners as in Example 1. The results are presented in Table 2 and Table 3.

In Example 2, it was confirmed that the first capture nucleic acid and the reagent immobilized layer were covalently bound with each other by a FT-IR ATR method in the same manner as in Example 1 based on presence or absence of a spectrum attributable to a thioether bond produced by the thiol group in the first capture nucleic acid newly forming a thioether bond (covalent bond) with the maleimide group at one end of the linker and presence or absence of a spectrum attributable to an amide bond produced by the amino group in the reagent immobilized layer newly forming an amide bond (covalent bond) with the N-hydroxysulfosuccinimide ester group at another end of the linker.

In Example 2, it was confirmed that the second capture nucleic acid and the reagent immobilized layer were covalently bound with each other by a FT-IR ATR method in the same manner as in Example 1 based on presence or absence of a spectrum attributable to a thioether bond produced by the thiol group in the second capture nucleic acid newly forming a thioether bond (covalent bond) with the maleimide group at one end of the linker and presence or absence of a spectrum attributable to an amide bond produced by the amino group in the reagent immobilized layer newly forming an amide bond (covalent bond) with the N-hydroxysulfosuccinimide ester group at another end of the linker.

(Example 3)

A lateral flow chromatographic device for nucleic acid detection (testing device) 10 of Example 3 onto which only a test line was transferred was produced in the same manner as in Example 1 except that unlike in Example 1, the label body reagent coating liquid of Preparation example 11 was used in <Formation of label body supplying portion>, and evaluated in the same manners as in Example 1 except that in <Evaluation of lines>, a testing target liquid was prepared using a DNA fragment that was labeled with biotin at a 5' end and included a base sequence that contained from the 5' end, a sequence containing 10 bases of thymine (T) and guanine (G) repeatedly, and 20 bases of adenine (A) continuously. The results are presented in Table 2 and Table 3.

In Example, 3, it was confirmed that the first capture nucleic acid and the reagent immobilized layer were covalently bound with each other by a FT-IR ATR method in the same manner as in Example 1 based on presence or

absence of a spectrum attributable to a thioether bond produced by the thiol group in the first capture nucleic acid newly forming a thioether bond (covalent bond) with the maleimide group at one end of the linker and presence or absence of a spectrum attributable to an amide bond produced by the amino group in the reagent immobilized layer newly forming an amide bond (covalent bond) with the N-hydroxysulfosuccinimide ester group at another end of the linker.

In Example 3, it was confirmed that the second capture nucleic acid and the reagent immobilized layer were covalently bound with each other by a FT-IR ATR method in the same manner as in Example 1 based on presence or absence of a spectrum attributable to a thioether bond produced by the thiol group in the second capture nucleic acid newly forming a thioether bond (covalent bond) with the maleimide group at one end of the linker and presence or absence of a spectrum attributable to an amide bond produced by the amino group in the reagent immobilized layer newly forming an amide bond (covalent bond) with the N-hydroxysulfosuccinimide ester group at another end of the linker.

(Example 4)

A lateral flow chromatographic device for nucleic acid detection (testing device 10) of Example 4 onto which only a test line was transferred was produced in the same manner as in Example 2 except that unlike in Example 2, the label body reagent coating liquid of Preparation example 11 was used in <Formation of label body supplying portion>, and evaluated in the same manners as in Example 1 except that in <Evaluation of lines>, a testing target liquid was prepared using a DNA fragment that was labeled with biotin at a 5' end and included a base sequence that contained from the 5' end, a sequence containing 10 bases of thymine (T) and guanine (G) repeatedly, and 20 bases of adenine (A) continuously. The results are presented in Table 2 and Table 3.

In Example 4, it was confirmed that the first capture nucleic acid and the reagent immobilized layer were covalently bound with each other by a FT-IR ATR method in the same manner as in Example 1 based on presence or absence of a spectrum attributable to a thioether bond produced by the thiol group in the first capture nucleic acid newly forming a thioether bond (covalent bond) with the maleimide group at one end of the linker and presence or absence of a spectrum attributable to an amide bond produced by the amino group in the reagent immobilized layer newly forming an amide bond (covalent bond) with the N-hydroxysulfosuccinimide ester group at another end of the linker.

In Example 4, it was confirmed that the second capture nucleic acid and the reagent immobilized layer were covalently bound with each other by a FT-IR ATR method in the same manner as in Example 1 based on presence or absence of a spectrum attributable to a thioether bond produced by the thiol group in the second capture nucleic acid newly forming a thioether bond (covalent bond) with the maleimide group at one end of the linker and presence or absence of a spectrum attributable to an amide bond produced by the amino group in the reagent immobilized layer newly forming an amide bond (covalent bond) with the N-hydroxysulfosuccinimide ester group at another end of the linker.

(Example 5)

A lateral flow chromatographic device for nucleic acid detection (testing device 10) of Example 5 was produced in the same manner as in Example 1 except that unlike in Example 1, the test line reagent coating liquid of Preparation example 12 was used in <Production of transfer medium for test line> and the control line reagent coating liquid of Preparation example 13 was used in <Production of transfer medium for control line>, and evaluated in the same manners as in Example 1. The results are presented in Table 2 and Table 3.

In Example 5, it was confirmed that the first capture nucleic acid and the reagent immobilized layer were covalently bound with each other by a FT-IR ATR method in the same manner as in Example 1 based on presence or absence of a spectrum attributable to a thioether bond produced by the thiol group in the first capture nucleic acid newly forming a thioether bond (covalent bond) with the maleimide group at one end of the linker and presence or absence of a spectrum attributable to an amide bond produced by the amino group in the reagent immobilized layer newly forming an amide bond (covalent bond) with the N-hydroxysulfosuccinimide ester group at another end of the linker.

In Example 5, it was confirmed that the second capture nucleic acid and the reagent immobilized layer were covalently bound with each other by a FT-IR ATR method in the same manner as in Example 1 based on presence or absence of a spectrum attributable to a thioether bond produced by the thiol group in the second capture nucleic acid newly forming a thioether bond (covalent bond) with the maleimide group at one end of the linker and presence or absence of a spectrum attributable to an amide bond produced by the amino group in the reagent immobilized layer newly forming an amide bond (covalent bond) with the N-hydroxysulfosuccinimide ester group at another end of the linker.

(Example 6)

A lateral flow chromatographic device for nucleic acid detection (testing device 10) of Example 6 was produced in the same manner as in Example 1 except that unlike in Example 1, the reagent immobilized layer coating liquid of Preparation example 16 was used in -Formation of reagent immobilized layer-, and evaluated in the same manners as

in Example 1. The results are presented in Table 2 and Table 3.

In Example 6, it was confirmed that the first capture nucleic acid and the reagent immobilized layer were covalently bound with each other by a FT-IR ATR method in the same manner as in Example 1 based on presence or absence of a spectrum attributable to a thioether bond produced by the thiol group in the first capture nucleic acid newly forming a thioether bond (covalent bond) with the maleimide group at one end of the linker and presence or absence of a spectrum attributable to an amide bond produced by the amino group in the reagent immobilized layer newly forming an amide bond (covalent bond) with the N-hydroxysulfosuccinimide ester group at another end of the linker.

In Example 6, it was confirmed that the second capture nucleic acid and the reagent immobilized layer were covalently bound with each other by a FT-IR ATR method in the same manner as in Example 1 based on presence or absence of a spectrum attributable to a thioether bond produced by the thiol group in the second capture nucleic acid newly forming a thioether bond (covalent bond) with the maleimide group at one end of the linker and presence or absence of a spectrum attributable to an amide bond produced by the amino group in the reagent immobilized layer newly forming an amide bond (covalent bond) with the N-hydroxysulfosuccinimide ester group at another end of the linker.

(Example 7)

A lateral flow chromatographic device for nucleic acid detection (testing device 10) of Example 7 was produced in the same manner as in Example 1 except that unlike in Example 1, the reagent immobilized layer coating liquid of Preparation example 16 was used in -Formation of reagent immobilized layer-, the test line reagent coating liquid of Preparation example 19 was used in <Production of transfer medium for test line>, and the control line reagent coating liquid of Preparation example 20 was used in <Production of transfer medium for control line>, and evaluated in the same manners as in Example 1. The results are presented in Table 2 and Table 3.

In Example 7, it was confirmed that the first capture nucleic acid and the reagent immobilized layer were covalently bound with each other by a FT-IR ATR method in the same manner as in Example 1 based on presence or absence of a spectrum attributable to an amide bond produced by the amino group in the first capture nucleic acid newly forming an amide bond (covalent bond) with the N-hydroxysulfosuccinimide ester group at one end of the linker and presence or absence of a spectrum attributable to an amide bond produced by the amino group in the reagent immobilized layer newly forming an amide bond (covalent bond) with the N-hydroxysulfosuccinimide ester group at another end of the linker.

In Example 7, it was confirmed that the second capture nucleic acid and the reagent immobilized layer were covalently bound with each other by a FT-IR ATR method in the same manner as in Example 1 based on presence or absence of a spectrum attributable to an amide bond produced by the amino group in the second capture nucleic acid newly forming an amide bond (covalent bond) with the N-hydroxysulfosuccinimide ester group at one end of the linker and presence or absence of a spectrum attributable to an amide bond produced by the amino group in the reagent immobilized layer newly forming an amide bond (covalent bond) with the N-hydroxysulfosuccinimide ester group at another end of the linker.

(Example 8)

A lateral flow chromatographic device for nucleic acid detection (testing device 10) of Example 8 was produced in the same manner as in Example 1 except that unlike in Example 1, the reagent immobilized layer coating liquid of Preparation example 16 was used in -Formation of reagent immobilized layer-, the test line reagent coating liquid of Preparation example 21 was used in <Production of transfer medium for test line>, and the control line reagent coating liquid of Preparation example 22 was used in <Production of transfer medium for control line>, and evaluated in the same manners as in Example 1. The results are presented in Table 2 and Table 3.

In Example 8, it was confirmed that the first capture nucleic acid and the reagent immobilized layer were covalently bound with each other by a FT-IR ATR method in the same manner as in Example 1 based on presence or absence of a spectrum attributable to an amide bond produced by the carboxyl group in the first capture nucleic acid newly forming an amide bond (covalent bond) with the amino group in the reagent immobilized layer.

In Example 8, it was confirmed that the second capture nucleic acid and the reagent immobilized layer were covalently bound with each other by a FT-IR ATR method in the same manner as in Example 1 based on presence or absence of a spectrum attributable to an amide bond produced by the carboxyl group in the second capture nucleic acid newly forming an amide bond (covalent bond) with the amino group in the reagent immobilized layer.

(Example 9)

A lateral flow chromatographic device for nucleic acid detection (testing device 10) of Example 9 was produced in the same manner as in Example 1 except that unlike in Example 1, the reagent immobilized layer coating liquid of

Preparation example 17 was used in -Formation of reagent immobilized layer-, the test line reagent coating liquid of Preparation example 23 was used in <Production of transfer medium for test line>, and the control line reagent coating liquid of Preparation example 24 was used in <Production of transfer medium for control line>, and evaluated in the same manners as in Example 1. The results are presented in Table 2 and Table 3.

In Example 9, it was confirmed that the first capture nucleic acid and the reagent immobilized layer were covalently bound with each other by a FT-IR ATR method in the same manner as in Example 1 based on presence or absence of a spectrum attributable to an amide bond produced by the amino group in the first capture nucleic acid newly forming an amide bond (covalent bond) with the carboxyl group in the reagent immobilized layer.

In Example 9, it was confirmed that the second capture nucleic acid and the reagent immobilized layer were covalently bound with each other by a FT-IR ATR method in the same manner as in Example 1 based on presence or absence of a spectrum attributable to an amide bond produced by the amino group in the second capture nucleic acid newly forming an amide bond (covalent bond) with the carboxyl group in the reagent immobilized layer.

(Example 10)

A lateral flow chromatographic device for nucleic acid detection (testing device 10) of Example 10 was produced in the same manner as in Example 1 except that unlike in Example 1, the reagent immobilized layer coating liquid of Preparation example 18 was used in -Formation of reagent immobilized layer-, the test line reagent coating liquid of Preparation example 25 was used in <Production of transfer medium for test line>, and the control line reagent coating liquid of Preparation example 26 was used in <Production of transfer medium for control line>, and evaluated in the same manners as in Example 1. The results are presented in Table 2 and Table 3.

In Example 10, it was confirmed that the first capture nucleic acid and the reagent immobilized layer were covalently bound with each other by a FT-IR ATR method in the same manner as in Example 1 based on presence or absence of a spectrum attributable to a thioether bond produced by the thiol group in the first capture nucleic acid newly forming a thioether bond (covalent bond) with the maleimide group at one end of the linker and presence or absence of a spectrum attributable to an ether bond and an amide bond produced by the hydroxyl group in the reagent immobilized layer newly forming an ether bond (covalent bond) and an amide bond (covalent bond) with the isocyanate group at another end of the linker.

In Example 10, it was confirmed that the second capture nucleic acid and the reagent immobilized layer were covalently bound with each other by a FT-IR ATR method in the same manner as in Example 1 based on presence or absence of a spectrum attributable to a thioether bond produced by the thiol group in the second capture nucleic acid newly forming a thioether bond (covalent bond) with the maleimide group at one end of the linker and presence or absence of a spectrum attributable to an ether bond and an amide bond produced by the hydroxyl group in the reagent immobilized layer newly forming an ether bond (covalent bond) and an amide bond (covalent bond) with the isocyanate group at another end of the cross-inker.

(Comparative Example 1)

<Production of testing device>

A testing device 10 illustrated in FIG. 9 and FIG. 10 was produced in the manner described below. FIG. 9 is a top view of a testing device of Comparative Example. FIG. 10 is a schematic cross-sectional view of the testing device of FIG. 9 taken along a line B-B.

-Production of paper substrate-

As a thermoplastic resin, a polyester-based hot-melt adhesive (available from Toagosei Co., Ltd., ARONMELT PES375S40) was heated to 190 degrees C, and then with a roll coater, coated over a PET film (available from Toray Industries, Inc., LUMIRROR S10, with an average thickness of 50 micrometers) cut into a size of 40 mm in width and 35 mm in length to have an average thickness of 50 micrometers over the PET film, to form an adhesive layer.

The PET film over which the adhesive layer was formed was left to stand still for 2 hours or longer. Subsequently, a nitrocellulose membrane (available from Merck Millipore Corporation, HF180) functioning as a flow path member 30 and cut into the same size as the PET film was overlapped with the adhesive layer-formed surface, and a load of 1 $kgf/cm^2$ was imposed on the overlapped product at a temperature of 150 degrees C for 10 seconds, to form a paper substrate.

-Immobilization of capture nucleic acids-

As illustrated in FIG. 9 and FIG. 10, with a positive-pressure spray device (available from BioDot, Inc., BIOJET), the test line reagent coating liquid of Preparation example 9 was coated at a position that was apart by 9 mm from the upstream end of the flow path member 30 of the paper substrate in a line shape having a length of 0.7 mm (test line 90a).

With the positive-pressure spray device, the control line reagent coating liquid of Preparation example 10 was coated at a position that was apart by 5 mm from the test line 90a in a line shape having a length of 0.7 mm (control line 90b).

After coating, the coating liquids were dried at 20 degrees C at 20 RH% for 16 hours.

It was confirmed that the capture nucleic acids were not immobilized to the flow path member by covalent binding by a FT-IR ATR method in the same manner as in Example 1 based on an analysis of the surface of the flow path member, proving that there was no spectrum change between before and after the immobilization.

-Formation of label body supplying portion-

Next, the label body reagent coating liquid of Preparation example 6 was coated in an amount of 60 microliters/$cm^2$ over a glass-fiber pad (available from Merck Millipore Corporation, GFCP203000) cut into a size of 40 mm in width and 18 mm in length, and dried overnight at a reduced pressure, to produce a label body supporting pad.

-Assembly of assay (testing device)-

The flow path member 30 was pasted over a base film, which was a PET film (available from Toray Industries, Inc. LUMIRROR S10, with an average thickness of 100 micrometers) cut into a size of 40 mm in width and 80 mm in length, at a position that was apart by 33 mm from one end of the base film (PET film) in the longer direction of the base film (PET film), in a state that the side of the flow path member opposite to the reagent-coated surface faced the base film (PET film).

Next, the label body supporting pad produced above and having a size of 40 mm in width and 18 mm in length was disposed and pasted over the top surface of the flow path member 30 in a manner to overlap the upstream end of the flow path member 30 by 2 mm (label body supplying portion 40), and a sample pad (available from Merck Millipore Corporation, CFSP223000) having a size of 40 mm in width and 35 mm in length was disposed and pasted in a manner to overlap the top surface of the label body supporting pad by 18 mm to produce a sample dropping pad (dropping portion) 80.

Next, an absorbing pad (available from Merck Millipore Corporation, CFSP223000) having a size of 40 mm in width and 28 mm in length was disposed and pasted over the top surface of the flow path member 30 in a manner to overlap the downstream end of the flow path member 30 by 16 mm to provide an absorbing member 70. Finally, the obtained product was cut along the longer direction of the product into a size of 4 mm in width and 80 mm in length, to obtain a lateral flow chromatographic device for nucleic acid detection (testing device 10) of Comparative Example 1.

The produced lateral flow chromatographic device for nucleic acid detection of Comparative Example 1 was evaluated in the same manners as in Example 1. The results are presented in Table 2 and Table 3.

(Comparative Example 2)

A lateral flow chromatographic device for nucleic acid detection (testing device 10) of Comparative Example 2 was produced in the same manner as in Example 1 except that unlike in Example 1, the test line reagent coating liquid of Preparation example 14 was used in <Production of transfer medium for test line> and the control line reagent coating liquid of Preparation example 15 was used in <Production of transfer medium for control line>, and evaluated in the same manners as in Example 1.

The results are presented in Table 2 and Table 3.

In Comparative Example 2, it was confirmed that the capture nucleic acids were not immobilized to the flow path member by covalent binding by a FT-IR ATR method in the same manner as in Example 1 based on an analysis of the surface of the flow path member, proving that there was no spectrum change between before and after the immobilization.

[Table 2]

| | Immobilization method | Shaped body | | Nucleic acid | Cross-linker | Label body | Photograph (test line) | Evaluation |
|---|---|---|---|---|---|---|---|---|
| | | Functional group | Resin | Functional group | | | | |
| Ex. 1 | Covalent binding | Amino group | Acrylic resin | Thiol group | SULFO-GMBS | Nucleic acid | 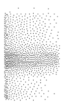 | A |
| Ex. 2 | Covalent binding | Amino group | Acrylic resin | Thiol group | SM(PEG)4 | Nucleic acid | 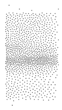 | A |
| Ex. 3 | Covalent binding | Amino group | Acrylic resin | Thiol group | SULFO-GMBS | Antibody | 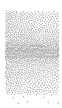 | A |
| Ex. 4 | Covalent binding | Amino group | Acrylic resin | Thiol group | SM(PEG)4 | Antibody | 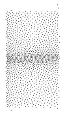 | A |
| Ex. 5 | Covalent binding | Amino group | Acrylic resin | Active ester group | None | Nucleic acid | 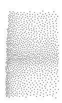 | B |
| Ex. 6 | Covalent | Amino group | Ethyleneimine resin | Thiol group | SULFO-GMBS | Nucleic acid | 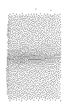 | A |
| Ex. 7 | Covalent binding | Amino group | Ethyleneimine resin | Amino group | DSG | Nucleic acid | 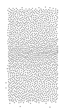 | B |
| Ex. 8 | Covalent binding | Amino group | Ethyleneimine resin | Carboxyl group | EDC | Nuleic acid | 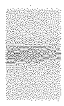 | A |
| Ex. 9 | Covalent binding | Carboxyl group | Ester resin | Amino group | EDC | Nucleic acid | 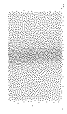 | A |
| Ex. 10 | Covalent binding | Hydroxyl group | Acrylic resin | Thiol group | PMPI | Nucleic acid | 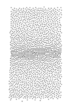 | A |
| Comp. Ex. 1 | Direct coating/drying | None | None | None | None | Nucleic acid | 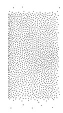 | D |
| Comp. Ex. 2 | No cross-linker | Amino group | Acrylic resin | Thiol group | None | Nucleic acid | 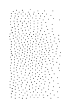 | D |

[Table 3]

| | Optical density of lines (signal: S) | Evaluation result | Background (N) | S/N ratio | Evaluation result | Total evaluation |
|---|---|---|---|---|---|---|
| Ex. 1 | 127 | B | 3.6 | 35 | A | A |
| Ex. 2 | 290 | A | 4.2 | 69 | A | S |
| Ex. 3 | 141 | B | 3.7 | 38 | A | A |
| Ex. 4 | 324 | A | 3.5 | 93 | A | S |
| Ex. 5 | 98 | C | 3.1 | 32 | A | C |
| Ex. 6 | 230 | A | 3.3 | 70 | A | S |
| Ex. 7 | 68 | C | 3.0 | 23 | B | D |
| Ex. 8 | 370 | A | 3.6 | 103 | A | S |
| Ex. 9 | 346 | A | 3.4 | 102 | A | S |
| Ex. 10 | 247 | A | 3.1 | 80 | A | S |
| Comp. Ex. 1 | 63 | C | 51 | 1.2 | D | E |
| Comp. Ex. 2 | - | E | - | - | E | E |

From the results of Table 2 and Table 3, in the evaluation of lines, it was possible to observe lines having a uniform color optical density throughout the lines and having a high visibility in Examples 1 to 10. In the evaluation of optical density, it was possible to observe lines having a high density in all Examples except for Examples 5 and 7. In terms of S/N ratio, a high value higher than or equal to 20 was observed in all Examples. From the total evaluation, it was revealed that the visibility of the lines was extraordinarily excellent when the combination of the functional group possessed by the shaped body and the functional group possessed by the capture nucleic acid was the combination of an amino group and a thiol group, the combination of an amino group and a carboxyl group, the combination of a carboxyl group and an amino group, and the combination of a hydroxyl group and a thiol group.

As compared with this, in Comparative Example 1, in the evaluation of lines, color development was observed, but blurring near the lines was so severe that it was barely possible to observe the color development. In the evaluation of optical density, the labeling particles in the lines were diffused in the paper to blur the color development to have a low density, leading to a significantly low S/N ratio.

In Comparative Example 2, it was impossible to observe color development in the evaluation of lines.

(Example 11)

A lateral flow chromatographic device for nucleic acid detection (testing device 10) of Example 11 was produced in the same manner as in Example 1. In <Evaluation of lines>, the density of lines was measured with a testing target liquid prepared by preparing a DNA fragment including a base sequence that contained from a 5' end, 20 bases of cytosine (C) continuously and 20 bases of adenine (A) continuously to a final concentration of 1.0 $\mu$ M with a 5×SSC buffer (75 mM sodium citrate and 750 mM sodium chloride, available from Nacalai Tesque, Inc.). The result is presented in Table 4.

(Example 12)

A lateral flow chromatographic device for nucleic acid detection (testing device 10) of Example 12 was produced in the same manner as in Example 1. In <Evaluation of lines>, the density of lines was measured with a testing target liquid prepared by preparing a DNA fragment including a base sequence that contained from a 5' end, 20 bases of cytosine (C) continuously, 5 bases of thymine (T) continuously, and 20 bases of adenine (A) continuously to a final concentration of 1.0 $\mu$M with a 5 × SSC buffer (75 mM sodium citrate and 750 mM sodium chloride, available from Nacalai Tesque, Inc.). The result is presented in Table 4.

(Example 13)

A lateral flow chromatographic device for nucleic acid detection (testing device 10) of Example 13 was produced in the same manner as in Example 1. In <Evaluation of lines>, the density of lines was measured with a testing target liquid prepared by preparing a DNA fragment including a base sequence that contained from a 5' end, 20 bases of cytosine (C) continuously, 10 bases of thymine (T) continuously, and 20 bases of adenine (A) continuously to a final concentration of 1.0 $\mu$M with a 5 × SSC buffer (75 mM sodium citrate and 750 mM sodium chloride, available from Nacalai Tesque, Inc.). The result is presented in Table 4.

(Example 14)

A lateral flow chromatographic device for nucleic acid detection (testing device 10) of Example 14 was produced in the same manner as in Example 1. In <Evaluation of lines>, the density of lines was measured with a testing target liquid prepared by preparing a DNA fragment including a base sequence that contained from a 5' end, 20 bases of cytosine (C) continuously, 20 bases of thymine (T) continuously, and 20 bases of adenine (A) continuously to a final concentration of 1.0 $\mu$M with a 5 × SSC buffer (75 mM sodium citrate and 750 mM sodium chloride, available from Nacalai Tesque, Inc.). The result is presented in Table 4.

(Example 15)

A lateral flow chromatographic device for nucleic acid detection (testing device 10) of Example 15 was produced in the same manner as in Example 1. In <Evaluation of lines>, the density of lines was measured with a testing target liquid prepared by preparing a DNA fragment including a base sequence that contained from a 5' end, 20 bases of cytosine (C) continuously, 30 bases of thymine (T) continuously, and 20 bases of adenine (A) continuously to a final concentration of 1.0 $\mu$ M with a 5 × SSC buffer (75 mM sodium citrate and 750 mM sodium chloride, available from Nacalai Tesque, Inc.). The result is presented in Table 4.

(Example 16)

A lateral flow chromatographic device for nucleic acid detection (testing device 10) of Example 16 was produced in the same manner as in Example 1. In <Evaluation of lines>, the density of lines of the lateral flow chromatographic device for nucleic acid detection was measured with a testing target liquid prepared by preparing a DNA fragment including a base sequence that contained from a 5' end, 20 bases of cytosine (C) continuously, 40 bases of thymine (T) continuously, and 20 bases of adenine (A) continuously to a final concentration of 1.0 $\mu$ M with a 5 × SSC buffer (75 mM sodium citrate and 750 mM sodium chloride, available from Nacalai Tesque, Inc.). The result is presented in Table 4.

[Table 4]

| | Distance (bases) between sites to be bound | Optical density of lines | Evaluation result |
|---|---|---|---|
| Ex. 11 | 0 | 209 | A |
| Ex. 12 | 5 | 190 | B |
| Ex. 13 | 10 | 201 | A |
| Ex. 14 | 20 | 157 | B |
| Ex. 15 | 30 | 119 | B |
| Ex. 16 | 40 | 98 | C |

From the results of Table 4, color development of the lines was observed in all of Examples 11 to 16. The S/N values, the actual measurements of which are not presented though, were 20 or higher. A high color development intensity was observed when the distance between one end of the site bound with the capture nucelic acid and one end of the site bound with the label body was 20 bases or less, and particularly 10 bases or less.

(Preparation example 101)

-Preparation of back layer coating liquid-

A silicone-based rubber emulsion (available from Shin-Etsu Chemical Co., Ltd., KS779H, with a solid concentration of 30% by mass) (16.8 parts by mass), a chloroplatinic acid catalyst (0.2 parts by mass), and toluene (83 parts by mass) were mixed, to obtain a back layer coating liquid.

(Preparation example 102)

-Preparation of release layer coating liquid-

A polyethylene wax (available from Toyo ADL Corporation, POLYWAX 1000, with a melting point of 99 degrees C and a penetration of 2 at 25 degrees C) (14 parts by mass), an ethylene-vinyl acetate copolymer (available from Du Pont-Mitsui Polychemicals Co., Ltd., EV-150, with a weight average molecular weight of 2,100, and VAc of 21% by mass) (6 parts by mass), toluene (60 parts by mass), and methyl ethyl ketone (20 parts by mass) were subjected to dispersion treatment until the average particle diameter became 2.5 micrometers, to obtain a release layer coating liquid.

(Preparation example 103)

-Preparation of reagent immobilized layer coating liquid-

An aminoethylated acrylic polymer (POLYMENT NK-380, available from Nippon Shokubai Co., Ltd.) was diluted to 15% by mass by addition of toluene as a solvent, to obtain a reagent immobilized layer coating liquid. The aminoethylated acrylic polymer is known to easily deteriorate and may highly assumedly have impact on immobilization of the reagent. Therefore, the preparation of the aminoethylated acrylic polymer was on an on demand basis.

(Preparation example 104)

-Preparation of test line reagent coating liquid-

A DNA fragment that continuously contained 20 bases of thymine (T) and to which a thiol group was introduced at a 5' end was prepared to have a final concentration of 2.5 $\mu$M with a sodium phosphate buffer having a final concentration of 100 mM (pH=7.2) and EDTA having a final concentration of 5 mM. To the resultant, GMBS (available from Thermo Fisher Scientific Inc.) having a final concentration of 1 mM was added as a linker, to obtain a test line reagent coating liquid.

The structural formula of GMBS is presented below. The spacer length of the linker is the distance from the carbon atom in the N-hydroxysuccinic acid imide ester group indicated by an arrow to the carbon atom in the maleimide group indicated by an arrow.

[Chem. 8]

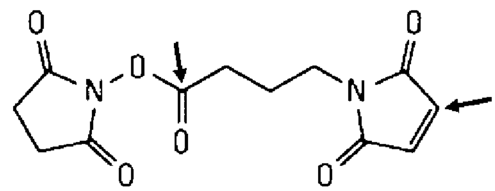

(Preparation example 105)

-Preparation of control line reagent coating liquid-

A DNA fragment that continuously contained 20 bases of cytosine (C) and to which a thiol group was introduced at a 3' end was prepared to have a final concentration of 2.5 $\mu$M with a sodium phosphate buffer having a final concentration of 100 mM (pH=7.2) and EDTA having a final concentration of 5 mM. To the resultant, GMBS (available from Thermo Fisher Scientific Inc.) having a final concentration of 1 mM was added as a linker, to obtain a control line reagent coating

liquid.

(Preparation example 106)

-Preparation of label body (nucleic acid) reagent coating liquid-

Gold colloid modified with a carboxyl group was bound by amide binding with a DNA fragment that continuously contained 20 bases of guanine (G) and to which an amino group was introduced at a 3' end using 1-ethyl-3-(3-dimethylaminopropyl)carbodiimide (EDC) (available from Thermo Fisher Scientific Inc.). The resultant was washed with a 50mM Tris-HCl buffer (pH=8.2), suspended in a label body diluting fluid (a 20mM Tris-HCl buffer (pH=8.2), 0.05% by mass polyethylene glycol, 5% by mass sucrose, and purified water), and adjusted to OD=2, to obtain a label body reagent coating liquid.

(Preparation example 107)

-Preparation of test line reagent coating liquid-

A DNA fragment that continuously contained 20 bases of thymine (T) and to which a thiol group was introduced at a 5' end was prepared to have a final concentration of 2.5 $\mu$M with a sodium phosphate buffer having a final concentration of 100 mM (pH=7.2) and EDTA having a final concentration of 5 mM. To the resultant, SM (PEG) 2 (available from Thermo Fisher Scientific Inc.) having a final concentration of 1 mM was added as a linker, to obtain a test line reagent coating liquid.

(Preparation example 108)

-Preparation of control line reagent coating liquid-

A DNA fragment that continuously contained 20 bases of cytosine (C) and to which a thiol group was introduced at a 3' end was prepared to have a final concentration of 2.5 $\mu$M with a sodium phosphate buffer having a final concentration of 100 mM (pH=7.2) and EDTA having a final concentration of 5 mM. To the resultant, SM (PEG) 2 (available from Thermo Fisher Scientific Inc.) having a final concentration of 1 mM was added as a linker, to obtain a control line reagent coating liquid.

(Preparation example 109)

-Preparation of test line reagent coating liquid-

A DNA fragment that continuously contained 20 bases of thymine (T) and to which a thiol group was introduced at a 5' end was prepared to have a final concentration of 2.5 $\mu$M with a sodium phosphate buffer having a final concentration of 100 mM (pH=7.2) and EDTA having a final concentration of 5 mM. To the resultant, SM (PEG) 4 (available from Thermo Fisher Scientific Inc.) having a final concentration of 1 mM was added as a linker, to obtain a test line reagent coating liquid.

(Preparation example 110)

-Preparation of control line reagent coating liquid-

A DNA fragment that continuously contained 20 bases of cytosine (C) and to which a thiol group was introduced at a 3' end was prepared to have a final concentration of 2.5 $\mu$M with a sodium phosphate buffer having a final concentration of 100 mM (pH=7.2) and EDTA having a final concentration of 5 mM. To the resultant, SM (PEG) 4 (available from Thermo Fisher Scientific Inc.) having a final concentration of 1 mM was added as a linker, to obtain a control line reagent coating liquid.

(Preparation example 111)

-Preparation of test line reagent coating liquid-

A DNA fragment that continuously contained 20 bases of thymine (T) and to which a thiol group was introduced

at a 5' end was prepared to have a final concentration of 2.5 $\mu$M with a sodium phosphate buffer having a final concentration of 100 mM (pH=7.2) and EDTA having a final concentration of 5 mM. To the resultant, SM (PEG) 6 (available from Thermo Fisher Scientific Inc.) having a final concentration of 1 mM was added as a linker, to obtain a test line reagent coating liquid.

(Preparation example 112)

-Preparation of control line reagent coating liquid-

A DNA fragment that continuously contained 20 bases of cytosine (C) and to which a thiol group was introduced at a 3' end was prepared to have a final concentration of 2.5 $\mu$M with a sodium phosphate buffer having a final concentration of 100 mM (pH=7.2) and EDTA having a final concentration of 5 mM. To the resultant, SM (PEG) 6 (available from Thermo Fisher Scientific Inc.) having a final concentration of 1 mM was added as a linker, to obtain a control line reagent coating liquid.

(Preparation example 113)

-Preparation of test line reagent coating liquid-

A DNA fragment that continuously contained 20 bases of thymine (T) and to which a thiol group was introduced at a 5' end was prepared to have a final concentration of 2.5 $\mu$M with a sodium phosphate buffer having a final concentration of 100 mM (pH=7.2) and EDTA having a final concentration of 5 mM. To the resultant, SM (PEG) 8 (available from Thermo Fisher Scientific Inc.) having a final concentration of 1 mM was added as a linker, to obtain a test line reagent coating liquid.

(Preparation example 114)

-Preparation of control line reagent coating liquid-

A DNA fragment that continuously contained 20 bases of cytosine (C) and to which a thiol group was introduced at a 3' end was prepared to have a final concentration of 2.5 $\mu$M with a sodium phosphate buffer having a final concentration of 100 mM (pH=7.2) and EDTA having a final concentration of 5 mM. To the resultant, SM (PEG) 8 (available from Thermo Fisher Scientific Inc.) having a final concentration of 1 mM was added as a linker, to obtain a control line reagent coating liquid.

(Preparation example 115)

-Preparation of test line reagent coating liquid-

A DNA fragment that continuously contained 20 bases of thymine (T) and to which a thiol group was introduced at a 5' end was prepared to have a final concentration of 2.5 $\mu$M with a sodium phosphate buffer having a final concentration of 100 mM (pH=7.2) and EDTA having a final concentration of 5 mM. To the resultant, SM (PEG) 12 (available from Thermo Fisher Scientific Inc.) having a final concentration of 1 mM was added as a linker, to obtain a test line reagent coating liquid.

(Preparation example 116)

-Preparation of control line reagent coating liquid-

A DNA fragment that continuously contained 20 bases of cytosine (C) and to which a thiol group was introduced at a 3' end was prepared to have a final concentration of 2.5 $\mu$M with a sodium phosphate buffer having a final concentration of 100 mM (pH=7.2) and EDTA having a final concentration of 5 mM. To the resultant, SM (PEG) 12 (available from Thermo Fisher Scientific Inc.) having a final concentration of 1 mM was added as a linker, to obtain a control line reagent coating liquid.

(Preparation example 117)

-Preparation of test line reagent coating liquid-

A DNA fragment that continuously contained 20 bases of thymine (T) and to which a thiol group was introduced at a 5' end was prepared to have a final concentration of 2.5 $\mu$M with a sodium phosphate buffer having a final concentration of 100 mM (pH=7.2) and EDTA having a final concentration of 5 mM. To the resultant, SM (PEG) 24 (available from Thermo Fisher Scientific Inc.) having a final concentration of 1 mM was added as a linker, to obtain a test line reagent coating liquid.

(Preparation example 118)

-Preparation of control line reagent coating liquid-

A DNA fragment that continuously contained 20 bases of cytosine (C) and to which a thiol group was introduced at a 3' end was prepared to have a final concentration of 2.5 $\mu$M with a sodium phosphate buffer having a final concentration of 100 mM (pH=7.2) and EDTA having a final concentration of 5 mM. To the resultant, SM (PEG) 24 (available from Thermo Fisher Scientific Inc.) having a final concentration of 1 mM was added as a linker, to obtain a control line reagent coating liquid.

(Preparation example 119)

-Preparation of label body (antibody) reagent coating liquid-

To a gold colloid solution (available from BBI Solutions, EMGC50) (9 mL), a $KH_2PO_4$ buffer (pH=7.0) prepared to 50 mM (1 mL) and then an anti-biotin monoclonal antibody (available from Bethyl Laboratories, Inc., ANTI-BIOTIN, GOAT-POLY A150-111A) prepared to 50 micrograms/mL (1 mL) were added and stirred. The resultant was left to stand still for 10 minutes. To the resultant, a 1% by mass polyethylene glycol aqueous solution (available from Wako Pure Chemical Industries, Ltd., 168-11285) (550 microliters) was added and stirred. To the resultant, a 10% by mass BSA aqueous solution (available from Sigma-Aldrich Co., LLC, A-7906) (1.1 mL) was further added and stirred.

Next, this solution was subjected to centrifugation for 30 minutes, and then, after the supernatant being removed, subjected to re-dispersion of the gold colloid using an ultrasonic cleaner. The centrifugation was performed with a centrifuge (available from Hitachi Koki Co., Ltd., HIMAC CF16RN) at a centrifugal acceleration of 8,000 $\times$ g at 4 degrees C. Subsequently, the resultant was dispersed in a gold colloid preservative solution [a 20 mM Tris-HCl buffer (pH=8.2), 0.05% by mass polyethylene glycol (with a weight average molecular weight of 2,000), 150 mM NaCl, a 1% by mass BSA aqueous solution, and a 0.1% by mass $NaN_3$ aqueous solution] (20 mL), subjected again to centrifugation under the same conditions as described above, and after the supernatant being removed except about 1 mL, subjected to re-dispersion of the gold colloid using an ultrasonic cleaner. These operations were repeated to prepare the solution to be OD=15 in the gold colloid preservative solution, to obtain a label body reagent coating liquid.

(Preparation example 120)

-Preparation of test line reagent coating liquid-

A DNA fragment that continuously contained 20 bases of thymine (T) and to which a thiol group was introduced at a 5' end was prepared to be 25 $\mu$M with a TE buffer (10 mM Tris-HCl and 1 mM EDTA, pH=7.4, available from Takara Bio Inc.), to obtain a test line reagent coating liquid.

(Preparation example 121)

-Preparation of control line reagent coating liquid-

A DNA fragment that continuously contained 20 bases of cytosine (C) and to which a thiol group was introduced at a 3' end was prepared to be 25 $\mu$M with a TE buffer (10 mM Tris-HCl and 1 mM EDTA, pH=7.4, available from Takara Bio Inc.), to obtain a control line reagent coating liquid.

(Preparation example 122)

-Preparation of test line reagent coating liquid-

A DNA fragment that continuously contained 20 bases of thymine (T) and to which a thiol group was introduced at a 5' end was prepared to have a final concentration of 2.5 $\mu$M with a sodium phosphate buffer having a final concentration of 100 mM (pH=7.2) and EDTA having a final concentration of 5 mM, to obtain a test line reagent coating liquid.

(Preparation example 123)

-Preparation of control line reagent coating liquid-

A DNA fragment that continuously contained 20 bases of cytosine (C) and to which a thiol group was introduced at a 3' end was prepared to have a final concentration of 2.5 $\mu$M with a sodium phosphate buffer having a final concentration of 100 mM (pH=7.2) and EDTA having a final concentration of 5 mM, to obtain a control line reagent coating liquid.

(Example 101)

<Production of transfer medium for test line>

-Formation of back layer-

The back layer coating liquid of Preparation example 101 was coated over one surface of a support, which was a polyethylene terephthalate (PET) film having an average thickness of 4.5 micrometers (available from Toray Industries, Inc. LUMIRROR F57) and dried at 80 degrees C for 10 seconds, to form a back layer having an average thickness of 0.02 micrometers.

-Formation of release layer-

Next, the release layer coating liquid of Preparation example 102 was coated over a surface of the PET film opposite to the surface over which the back layer was formed and dried at 25 degrees C for 30 minutes, to form a release layer having an average thickness of 30 micrometers.

-Formation of reagent immobilized layer-

Next, the reagent immobilized layer coating liquid of Preparation example 103 was coated over the surface of the release layer and dried at 50 degrees C for 30 minutes, to form a reagent immobilized layer having an average thickness of 3 micrometers. In this way, a transfer medium was produced.

<Immobilization of capture nucleic acids>

-Test line (immobilization of first capture nucleic acid)-

Next, the test line reagent coating liquid of Preparation example 104 was poured into a shallow square vat, and the transfer medium was floated over the test line reagent coating liquid such that only the reagent immobilized layer surface contacted the test line reagent coating liquid. In this state, the shallow square vat was covered with a lid and left to stand still at 25 degrees C for 30 minutes. Subsequently, the reagent immobilized layer surface was washed with a 50 mM Tris-HCl buffer (pH=9.0) and subjected to vacuum drying at 25 degrees C for 30 minutes, to immobilize the reagent to the reagent immobilized layer. In this way, a transfer medium for a test line of Example 101 was produced. The transfer medium for a test line was used for production of a lateral flow chromatographic device for nucleic acid detection immediately after the transfer medium was produced.

It was confirmed that the first capture nucleic acid and the reagent immobilized layer were bound with each other by a linker by a FT-IR ATR method (FT-IR6800, available from JASCO Corporation) based on presence or absence of a spectrum attributable to a thioether bond produced by the thiol group in the first capture nucleic acid newly forming a thioether bond (covalent bond) with the maleimide group at one end of the linker and presence or absence of a spectrum attributable to an amide bond produced by the amino group in the reagent immobilized layer newly forming an amide bond (covalent bond) with the N-hydroxysulfosuccinimide ester group at another end of the linker.

<Production of transfer medium for control line>

-Control line (immobilization of second capture nucleic acid)-

A transfer medium for a control line of Example 101 was produced in the same manner as in <Production of transfer medium for test line> described above, except that unlike in <Production of transfer medium for test line>, the control line reagent coating liquid of Preparation example 105 was used instead of the test line reagent coating liquid. The transfer medium for a control line was used for production of a lateral flow chromatographic device for nucleic acid detection immediately after the transfer medium was produced.

It was confirmed that the second capture nucleic acid and the reagent immobilized layer were bound with each other by a linker by a FT-IR ATR method (FT-IR6800, available from JASCO Corporation) based on presence or absence of a spectrum attributable to a thioether bond produced by the thiol group in the second capture nucleic acid newly forming a thioether bond (covalent bond) with the maleimide group at one end of the linker and presence or absence of a spectrum attributable to an amide bond produced by the amino group in the reagent immobilized layer newly forming an amide bond (covalent bond) with the N-hydroxysulfosuccinimide ester group at another end of the linker.

<Production of testing device>

The testing device 10 illustrated in FIG. 1 and FIG. 2 was produced in the manner described below. FIG. 1 is a top view of the testing device of Example. FIG. 2 is a schematic cross-sectional view of the testing device of FIG. 1 taken along a line A-A. Immediately after the testing device was produced, reactions and evaluations were performed.

-Production of paper substrate (substrate+flow path member)-

As a thermoplastic resin, a polyester-based hot-melt adhesive (available from Toagosei Co., Ltd., ARONMELT PES375S40) was heated to 190 degrees C, and then with a roll coater, coated over a PET film (available from Toray Industries, Inc., LUMIRROR S10, with an average thickness of 50 micrometers) 20 cut into a size of 40 mm in width and 80 mm in length to have an average thickness of 50 micrometers over the PET film, to form an adhesive layer.

The PET film 20 over which the adhesive layer was formed was left to stand still for 2 hours or longer. Subsequently, a nitrocellulose membrane (available from Merck Millipore Corporation, HF180) cut into a size of 40 mm in width and 35 mm in length was overlapped with the surface of the adhesive layer at a position that was 33 mm from one end of the surface of the adhesive layer in the longer direction (this end being an upstream end, the opposite end being a downstream end) in a state that the nitrocellulose membrane and the surface of the adhesive layer coincided widthwise, and a load of 1 $kgf/cm^2$ was imposed on the overlapped product at a temperature of 150 degrees C for 10 seconds, to form a flow path member 30. Finally, the obtained product was cut along the longer direction of the product into a size of 4 mm in width and 80 mm in length, to obtain a paper substrate.

The voidage of the nitrocellulose membrane as the flow path member 30 of the paper substrate was calculated according to a calculation formula 1 below based on a basis weight ($g/m^2$) and an average thickness (micrometer) of the flow path member and the specific gravity of the component of the flow path member. As a result, the voidage of the nitrocellulose membrane was 70%.

<Calculation formula 1>

$$\text{Voidage (\%)} = \{1 - [\text{basis weight } (g/m^2)/\text{average thickness (micrometer)}/\text{specific gravity of the component}]\} \times 100$$

When the voidage of the flow path member is 40% or greater but 90% or less, the flow path member can be said to be porous.

-Transfer of test line-

The flow path member 30 of the paper substrate and the reagent-immobilized side of the transfer medium for a test line were faced and overlapped with each other. Subsequently, with a thermal transfer printer, as illustrated in FIG. 1 and FIG. 2, the transfer medium for a test line was transferred onto a position that was apart by 9 mm from the upstream end of the flow path member 30 in a line shape having a width of 4 mm and a length of 0.7 mm (first detecting

portion 50a).

As the thermal transfer printer, an evaluation system having a printing speed of 42 mm/sec and an applied energy of 0.17 mJ/dot was constructed with a thermal head having a dot density of 300 dpi (available from TDK Corporation).

-Transfer of control line-

Next, the transfer medium for a control line was transferred onto a position that was apart by 5 mm from the position onto which the transfer medium for a test line was transferred in a line shape having a width of 4 mm and a length of 0.7 mm (second detecting portion 50b).

-Formation of label body supplying portion-

Next, the label body reagent coating liquid of Preparation example 106 was coated in an amount of 60 microliters/$cm^2$ over a glass-fiber pad (available from Merck Millipore Corporation, GFCP203000) cut into a size of 4 mm in width and 18 mm in length, and dried overnight at a reduced pressure to produce a label body supporting pad.

As illustrated in FIG. 1 and FIG. 2, the label body supporting pad was disposed at a position that was apart by 17 mm from the upstream end of the paper substrate, and overlapped with and pasted over the adhesive layer provided over the paper substrate (label body supplying portion 40).

-Formation of dropping portion-

As illustrated in FIG. 1 and FIG. 2, a sample pad (available from Merck Millipore Corporation, CFSP223000) having a width of 4 mm and a length of 35 mm was disposed and pasted in a manner to overlap with the upper surface of the label body supplying portion 40 by 18 mm (dropping portion 80).

-Absorbing member-

An absorbing member 70 (available from Merck Millipore Corporation, CFSP223000) was provided as illustrated in FIG. 1 and FIG. 2. In this way, a lateral flow chromatographic device for nucleic acid detection (testing device 10) of Example 101 was obtained.

<Evaluation of lines>

-Preparation of testing target liquid-

A DNA fragment including a base sequence that contained from a 5' end, 20 bases of cytosine (C) continuously, a sequence containing 10 bases of thymine (T) and guanine (G) repeatedly, and 20 bases of adenine (A) continuously was prepared to have a final concentration of 1.0 nM with a 5 × SSC buffer (75 mM sodium citrate and 750 mM sodium chloride available from Nacalai Tesque, Inc.), to obtain a testing target liquid.

-Reaction-

The testing target liquid was dropped in an amount of 100 microliters onto the upstream end portion of the lateral flow chromatographic device 10 for nucleic acid detection illustrated in FIG. 1 and FIG. 2. Thirty minutes later, the lines were evaluated according to the criteria described below based on visual observation. The result is presented in Table 6.

<Evaluation criteria>

A: A clear color development was recognized at the positions of the test line and the control line at a uniform color optical density throughout the lines without discontinuation of the lines.
B: The lines were not discontinuous and enabled judgement but were slightly non-uniform in the color optical density from place to place.
C: Color development was barely recognized as line shapes but with a partial discontinuation in the lines.
D: No color development was recognized or color development was not in line shapes such as when the lines flowed

to the downstream side. Examples of the evaluation criteria are presented in Table 5. The photographs in Table 5 are each a photograph of the test line after testing.

[Table 5]

| Evaluation criteria | A | B | C | D |
|---|---|---|---|---|
| Photograph | 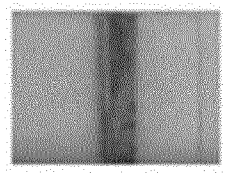 | 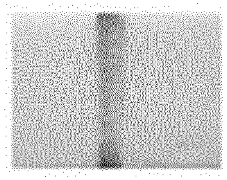 | 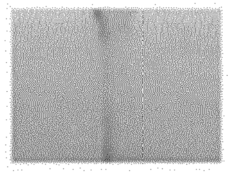 | 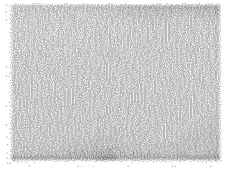 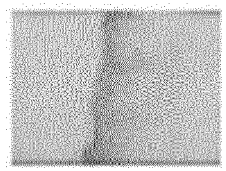 |

<Measurement of optical density of lines>

The lateral flow chromatographic device for nucleic acid detection after having developed colors and used in the evaluation of lines above was measured with a chromatoreader (available from Hamamatsu Photonics K.K., C 10066), to obtain the optical density of the lines. The optical density was evaluated according to the criteria described below. The results are presented in Table 7. A greater optical density of the lines is more preferable. When no line optical density was detected by the chromatoreader, the result is presented as "-". When an optical density by this chromatoreader is 20 or greater, the color development can be recognized visually.

<Evaluation criteria for optical density of lines>

A: The optical density was 250 or greater.
B: The optical density was 150 or greater but less than 250.
C: The optical density was 20 or greater but less than 150.
D: The optical density was less than 20, or was unmeasurable because no lines were recognized.

(Example 102)

A lateral flow chromatographic device for nucleic acid detection (testing device 10) of Example 102 was produced in the same manner as in Example 101 except that unlike in Example 101, the test line reagent coating liquid of Preparation example 107 was used in <Production of transfer medium for test line> and the control line reagent coating liquid of Preparation example 108 was used in <Production of transfer medium for control line>, and evaluated in the same manners as in Example 101. The results are presented in Table 6 and Table 7.

In Example 102, it was confirmed that the first capture nucleic acid and the reagent immobilized layer were bound with each other by a linker by a FT-IR ATR method in the same manner as in Example 101 based on presence or absence of a spectrum attributable to a thioether bond produced by the thiol group in the first capture nucleic acid newly forming a thioether bond (covalent bond) with the maleimide group at one end of the linker and presence or absence of a spectrum attributable to an amide bond produced by the amino group in the reagent immobilized layer newly forming an amide bond (covalent bond) with the N-hydroxysulfosuccinimide ester group at another end of the linker.

In Example 102, it was confirmed that the second capture nucleic acid and the reagent immobilized layer were bound with each other by a linker by a FT-IR ATR method in the same manner as in Example 101 based on presence or absence of a spectrum attributable to a thioether bond produced by the thiol group in the second capture nucleic acid newly forming a thioether bond (covalent bond) with the maleimide group at one end of the linker and presence or absence of a spectrum attributable to an amide bond produced by the amino group in the reagent immobilized layer newly forming an amide bond (covalent bond) with the N-hydroxysulfosuccinimide ester group at another end of the linker.

(Example 103)

A lateral flow chromatographic device for nucleic acid detection (testing device 10) of Example 103 was produced in the same manner as in Example 101 except that unlike in Example 101, the test line reagent coating liquid of Preparation example 109 was used in <Production of transfer medium for test line> and the control line reagent coating liquid of Preparation example 110 was used in <Production of transfer medium for control line>, and evaluated in the same manners as in Example 101. The results are presented in Table 6 and Table 7.

In Example 103, it was confirmed that the first capture nucleic acid and the reagent immobilized layer were bound with each other by a linker by a FT-IR ATR method in the same manner as in Example 101 based on presence or absence of a spectrum attributable to a thioether bond produced by the thiol group in the first capture nucleic acid newly forming a thioether bond (covalent bond) with the maleimide group at one end of the linker and presence or absence of a spectrum attributable to an amide bond produced by the amino group in the reagent immobilized layer newly forming an amide bond (covalent bond) with the N-hydroxysulfosuccinimide ester group at another end of the linker.

In Example 103, it was confirmed that the second capture nucleic acid and the reagent immobilized layer were bound with each other by a linker by a FT-IR ATR method in the same manner as in Example 101 based on presence or absence of a spectrum attributable to a thioether bond produced by the thiol group in the second capture nucleic acid newly forming a thioether bond (covalent bond) with the maleimide group at one end of the linker and presence or absence of a spectrum attributable to an amide bond produced by the amino group in the reagent immobilized layer newly forming an amide bond (covalent bond) with the N-hydroxysulfosuccinimide ester group at another end of the linker.

(Example 104)

A lateral flow chromatographic device for nucleic acid detection (testing device 10) of Example 104 was produced in the same manner as in Example 101 except that unlike in Example 101, the test line reagent coating liquid of Preparation example 111 was used in <Production of transfer medium for test line> and the control line reagent coating liquid of Preparation example 112 was used in <Production of transfer medium for control line>, and evaluated in the same manners as in Example 101. The results are presented in Table 6 and Table 7.

In Example 104, it was confirmed that the first capture nucleic acid and the reagent immobilized layer were bound with each other by a linker by a FT-IR ATR method in the same manner as in Example 101 based on presence or absence of a spectrum attributable to a thioether bond produced by the thiol group in the first capture nucleic acid newly forming a thioether bond (covalent bond) with the maleimide group at one end of the linker and presence or absence of a spectrum attributable to an amide bond produced by the amino group in the reagent immobilized layer newly forming an amide bond (covalent bond) with the N-hydroxysulfosuccinimide ester group at another end of the linker.

In Example 104, it was confirmed that the second capture nucleic acid and the reagent immobilized layer were bound with each other by a linker by a FT-IR ATR method in the same manner as in Example 101 based on presence or absence of a spectrum attributable to a thioether bond produced by the thiol group in the second capture nucleic acid newly forming a thioether bond (covalent bond) with the maleimide group at one end of the linker and presence or absence of a spectrum attributable to an amide bond produced by the amino group in the reagent immobilized layer newly forming an amide bond (covalent bond) with the N-hydroxysulfosuccinimide ester group at another end of the linker.

(Example 105)

A lateral flow chromatographic device for nucleic acid detection (testing device 10) of Example 105 was produced in the same manner as in Example 101 except that unlike in Example 101, the test line reagent coating liquid of Preparation example 113 was used in <Production of transfer medium for test line> and the control line reagent coating liquid of Preparation example 114 was used in <Production of transfer medium for control line>, and evaluated in the same manners as in Example 101. The results are presented in Table 6 and Table 7.

In Example 105, it was confirmed that the first capture nucleic acid and the reagent immobilized layer were bound with each other by a linker by a FT-IR ATR method in the same manner as in Example 101 based on presence or absence of a spectrum attributable to a thioether bond produced by the thiol group in the first capture nucleic acid newly forming a thioether bond (covalent bond) with the maleimide group at one end of the linker and presence or absence of a spectrum attributable to an amide bond produced by the amino group in the reagent immobilized layer newly forming an amide bond (covalent bond) with the N-hydroxysulfosuccinimide ester group at another end of the linker.

In Example 105, it was confirmed that the second capture nucleic acid and the reagent immobilized layer were bound with each other by a linker by a FT-IR ATR method in the same manner as in Example 101 based on presence or absence of a spectrum attributable to a thioether bond produced by the thiol group in the second capture nucleic acid newly forming a thioether bond (covalent bond) with the maleimide group at one end of the linker and presence or absence of a spectrum attributable to an amide bond produced by the amino group in the reagent immobilized layer newly forming an amide bond (covalent bond) with the N-hydroxysulfosuccinimide ester group at another end of the linker.

(Example 106)

A lateral flow chromatographic device for nucleic acid detection (testing device 10) of Example 106 was produced in the same manner as in Example 101 except that unlike in Example 101, the test line reagent coating liquid of Preparation example 115 was used in <Production of transfer medium for test line> and the control line reagent coating liquid of Preparation example 116 was used in <Production of transfer medium for control line>, and evaluated in the same manners as in Example 101. The results are presented in Table 6 and Table 7.

In Example 106, it was confirmed that the first capture nucleic acid and the reagent immobilized layer were bound with each other by a linker by a FT-IR ATR method in the same manner as in Example 101 based on presence or absence of a spectrum attributable to a thioether bond produced by the thiol group in the first capture nucleic acid newly forming a thioether bond (covalent bond) with the maleimide group at one end of the linker and presence or absence of a spectrum attributable to an amide bond produced by the amino group in the reagent immobilized layer newly forming an amide bond (covalent bond) with the N-hydroxysulfosuccinimide ester group at another end of the linker.

In Example 106, it was confirmed that the second capture nucleic acid and the reagent immobilized layer were bound with each other by a linker by a FT-IR ATR method in the same manner as in Example 101 based on presence or absence of a spectrum attributable to a thioether bond produced by the thiol group in the second capture nucleic acid newly forming a thioether bond (covalent bond) with the maleimide group at one end of the linker and presence or absence of a spectrum attributable to an amide bond produced by the amino group in the reagent immobilized layer

newly forming an amide bond (covalent bond) with the N-hydroxysulfosuccinimide ester group at another end of the linker.

(Example 107)

A lateral flow chromatographic device for nucleic acid detection (testing device 10) of Example 107 was produced in the same manner as in Example 101 except that unlike in Example 101, the test line reagent coating liquid of Preparation example 117 was used in <Production of transfer medium for test line> and the control line reagent coating liquid of Preparation example 118 was used in <Production of transfer medium for control line>, and evaluated in the same manners as in Example 101. The results are presented in Table 6 and Table 7.

In Example 107, it was confirmed that the first capture nucleic acid and the reagent immobilized layer were bound with each other by a linker by a FT-IR ATR method in the same manner as in Example 101 based on presence or absence of a spectrum attributable to a thioether bond produced by the thiol group in the first capture nucleic acid newly forming a thioether bond (covalent bond) with the maleimide group at one end of the linker and presence or absence of a spectrum attributable to an amide bond produced by the amino group in the reagent immobilized layer newly forming an amide bond (covalent bond) with the N-hydroxysulfosuccinimide ester group at another end of the linker.

In Example 107, it was confirmed that the second capture nucleic acid and the reagent immobilized layer were bound with each other by a linker by a FT-IR ATR method in the same manner as in Example 101 based on presence or absence of a spectrum attributable to a thioether bond produced by the thiol group in the second capture nucleic acid newly forming a thioether bond (covalent bond) with the maleimide group at one end of the linker and presence or absence of a spectrum attributable to an amide bond produced by the amino group in the reagent immobilized layer newly forming an amide bond (covalent bond) with the N-hydroxysulfosuccinimide ester group at another end of the linker.

(Example 108)

A lateral flow chromatographic device for nucleic acid detection (testing device 10) of Example 108 onto which only a test line was transferred was produced in the same manner as in Example 103 except that unlike in Example 103, the label body reagent coating liquid of Preparation example 113 was used in <Formation of label body supplying portion>, and evaluated in the same manners as in Example 101 except that in <Evaluation of lines>, a testing target liquid was prepared using a DNA fragment that was labeled with biotin at a 5’ end and included a base sequence that contained from the 5’ end, a sequence containing 10 bases of thymine (T) and guanine (G) repeatedly, and 20 bases of adenine (A) continuously.

In Example 108, it was confirmed that the first capture nucleic acid and the reagent immobilized layer were bound with each other by a linker by a FT-IR ATR method in the same manner as in Example 101 based on presence or absence of a spectrum attributable to a thioether bond produced by the thiol group in the first capture nucleic acid newly forming a thioether bond (covalent bond) with the maleimide group at one end of the linker and presence or absence of a spectrum attributable to an amide bond produced by the amino group in the reagent immobilized layer newly forming an amide bond (covalent bond) with the N-hydroxysulfosuccinimide ester group at another end of the linker.

In Example 108, it was confirmed that the second capture nucleic acid and the reagent immobilized layer were bound with each other by a linker by a FT-IR ATR method in the same manner as in Example 101 based on presence or absence of a spectrum attributable to a thioether bond produced by the thiol group in the second capture nucleic acid newly forming a thioether bond (covalent bond) with the maleimide group at one end of the linker and presence or absence of a spectrum attributable to an amide bond produced by the amino group in the reagent immobilized layer newly forming an amide bond (covalent bond) with the N-hydroxysulfosuccinimide ester group at another end of the linker.

(Comparative Example 101)

<Production of testing device>

A testing device 10 illustrated in FIG. 9 and FIG. 10 was produced in the manner described below. FIG. 9 is a top view of the testing device.

FIG. 10 is a schematic cross-sectional view of the testing device of FIG. 9 taken along a line B-B.

-Production of paper substrate-

As a thermoplastic resin, a polyester-based hot-melt adhesive (available from Toagosei Co., Ltd., ARONMELT PES375S40) was heated to 190 degrees C, and then with a roll coater, coated over a PET film (available from Toray Industries, Inc., LUMIRROR S10, with an average thickness of 50 micrometers) cut into a size of 40 mm in width and 35 mm in length to have an average thickness of 50 micrometers over the PET film, to form an adhesive layer.

The PET film over which the adhesive layer was formed was left to stand still for 2 hours or longer. Subsequently, a nitrocellulose membrane (available from Merck Millipore Corporation, HF180) functioning as a flow path member 30 and cut into the same size as the PET film was overlapped with the adhesive layer-formed surface, and a load of 1 kgf/cm$^2$ was imposed on the overlapped product at a temperature of 150 degrees C for 10 seconds, to form a paper substrate.

-Immobilization of capture nucleic acids-

As illustrated in FIG. 9 and FIG. 10, with a positive-pressure spray device (available from BioDot, Inc., BIOJET), the test line reagent coating liquid of Preparation example 120 was coated at a position that was apart by 9 mm from the upstream end of the flow path member 30 of the paper substrate in a line shape having a length of 0.7 mm (test line 90a).

With the positive-pressure spray device, the control line reagent coating liquid of Preparation example 121 was coated at a position that was apart by 5 mm from the test line 90a in a line shape having a length of 0.7 mm (control line 90b). After coating, the coating liquids were dried at 20 degrees C at 20 RH% for 16 hours.

It was confirmed that the capture nucleic acids were not immobilized to the flow path member by a linker by a FT-IR ATR method in the same manner as in Example 101 based on an analysis of the surface of the flow path member, proving that there was no spectrum change between before and after the immobilization.

-Formation of label body supplying portion-

Next, the label body reagent coating liquid of Preparation example 106 was coated in an amount of 60 microliters/cm$^2$ over a glass-fiber pad (available from Merck Millipore Corporation, GFCP203000) cut into a size of 40 mm in width and 18 mm in length, and dried overnight at a reduced pressure, to produce a label body supporting pad.

-Assembly of assay (testing device)-

The flow path member 30 was pasted over a base film, which was a PET film (available from Toray Industries, Inc. LUMIRROR S10, with an average thickness of 100 micrometers) cut into a size of 40 mm in width and 80 mm in length, at a position that was apart by 33 mm from one end of the base film (PET film) in the longer direction of the base film (PET film), in a state that the side of the flow path member opposite to the reagent-coated surface faced the base film (PET film).

Next, the label body supporting pad produced above and having a size of 40 mm in width and 18 mm in length was disposed and pasted over the top surface of the flow path member 30 in a manner to overlap the upstream end of the flow path member 30 by 2 mm (label body supplying portion 40), and a sample pad (available from Merck Millipore Corporation, CFSP223000) having a size of 40 mm in width and 35 mm in length was disposed and pasted in a manner to overlap the top surface of the label body supporting pad by 18 mm to produce a sample dropping pad (dropping portion) 80.

Next, an absorbing pad (available from Merck Millipore Corporation, CFSP223000) having a size of 40 mm in width and 28 mm in length was disposed and pasted over the top surface of the flow path member 30 in a manner to overlap the downstream end of the flow path member 30 by 16 mm to provide an absorbing member 70. Finally, the obtained product was cut along the longer direction of the product into a size of 4 mm in width and 80 mm in length, to obtain a lateral flow chromatographic device for nucleic acid detection (testing device 10) of Comparative Example 101.

The produced lateral flow chromatographic device for nucleic acid detection of Comparative Example 101 was evaluated in the same manners as in Example 101. The results are presented in Table 6 and Table 7.

(Comparative Example 102)

A lateral flow chromatographic device for nucleic acid detection (testing device 10) of Comparative Example 102 was produced in the same manner as in Example 101 except that unlike in Example 101, the test line reagent coating liquid of Preparation example 122 was used in <Production of transfer medium for test line> and the control line reagent coating liquid of Preparation example 123 was used in <Production of transfer medium for control line>, and evaluated in the same manners as in Example 101. The results are presented in Table 6 and Table 7.

In Comparative Examples 101 and 102, it was confirmed that the capture nucleic acids were not bound with the flow path member by a FT-IR ATR method in the same manner as in Example 101 based on an analysis of the surface of the flow path member, proving that there was no spectrum change between before and after the immobilization.

[Table 6]

| | Flow path member | Linker | Label body | Photograph (test line) | Evaluation result |
|---|---|---|---|---|---|
| Ex. 101 | Nitrocellulose | GMBS | Nucleic acid | 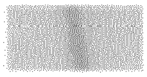 | **A** |
| Ex. 102 | Nitrocellulose | SM(PEG)2 | Nucleic acid | 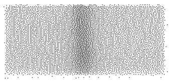 | **A** |
| Ex. 103 | Nitrocellulose | SM(PEG)4 | Nucleic acid | 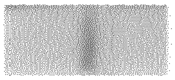 | **A** |
| Ex. 104 | Nitrocellulose | SM(PEG)6 | Nucleic acid | 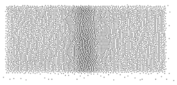 | **A** |
| Ex. 105 | Nitrocellulose | SM(PEG)8 | Nucleic acid | 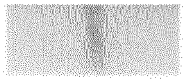 | **A** |
| Ex. 106 | Nitrocellulose | SM(PEG)12 | Nucleic acid | 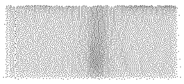 | **A** |
| Ex. 107 | Nitrocellulose | SM(PEG)24 | Nucleic acid | 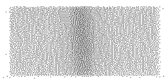 | **A** |
| Ex. 108 | Nitrocellulose | SM(PEG)4 | Antibody | 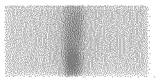 | **A** |
| Comp. Ex. 101 | Nitrocellulose | None (direct coating/drying | Nucleic acid | 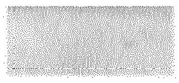 | **D** |
| Comp. Ex. 102 | Nitrocellulose | None | Nucleic acid | 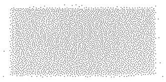 | **D** |

[Table 7]

| | Linker | Spacer length | Optical density of lines | Evaluation result |
|---|---|---|---|---|
| Ex. 101 | GMBS | 7.3 angstroms | 262 | B |
| Ex. 102 | SM (PEG) 2 | 17.6 angstroms | 389 | A |
| Ex. 103 | SM (PEG) 4 | 24.6 angstroms | 348 | A |
| Ex. 104 | SM (PEG) 6 | 32.5 angstroms | 395 | A |
| Ex. 105 | SM (PEG) 8 | 39.25 angstroms | 259 | A |
| Ex. 106 | SM (PEG) 12 | 53.4 angstroms | 246 | B |
| Ex. 107 | SM (PEG) 24 | 95.2 angstroms | 339 | A |
| Ex. 108 | SM (PEG) 4 | 24.6 angstroms | 366 | A |
| Comp. Ex. 101 | None (direct coating/drying) | None | 64 | C |
| Comp. Ex. 102 | None | None | - | D |

From the results of Table 6 and Table 7, in Examples 101 to 108, in the evaluation of visibility, it was possible to observe lines having a uniform color optical density throught the lines and having a high visibility. In the evaluation of

optical density, it was possible to observe lines having a high density, and all of the linkers used resulted in good results.

As compared with this, in Comparative Examle 101 in which the capture nucleic acids were not covalently bound with the reagent immobilized layers, in the evaluation of visibility, color development was observed but blurring near the lines was so severe that it was barely possible to observe the color development.

Likewise, in Comparative Example 102, it was impossible to observe color development on the lines.

(Example 109)

A lateral flow chromatographic device for nucleic acid detection (testing device 10) of Example 109 was produced in the same manner as in Example 101.

<Evaluation of lines>

-Preparation of testing target liquid-

A DNA fragment including a base sequence that contained from a 5' end, 20 bases of cytosine (C) continuously, a sequence containing 10 bases of thymine (T) and guanine (G) repeatedly, and 20 bases of adenine (A) continuously was prepared with a 5 × SSC buffer (75 mM sodium citrate and 750 mM sodium chloride, available from Nacalai Tesque, Inc.) to have final concentrations of from $1 \times 10^{-13}$ M through $1 \times 10^{-7}$ M (from 0.1 pM through 100 nM) at 10-fold intervals, to obtain testing target liquids having 7 different concentrations.

-Reaction-

Each of the testing target liquids was dropped in an amount of 100 microliters onto the upstream end portion of the lateral flow chromatographic device 10 for nucleic acid detection illustrated in FIG. 1 and FIG. 2. Thirty minutes later, the lateral flow chromatographic device for nucleic acid detection after having developed colors was measured with a chromatoreader (available from Hamamatsu Photonics K.K., C10066), to obtain the optical density of the lines. The optical density was evaluated according to the criteria described below. The testing target liquids having the respective concentrations were each used for reaction and measurement 3 times with the lateral flow chromatographic device for nucleic acid detection produced, and the average value was used for evaluation. A greater optical density of the lines is more preferable. When no line optical density was detected by the chromatoreader, the result is presented as "-". When an optical density by this chromatoreader is 20 or greater, the color development can be recognized visually.

Variation of optical density was evaluated according to the criteria described below based on standard deviation. A detectable concentration range was also evaluated according to the criteria described below. For detection of nucleic acids, a wider detectable concentration range is more preferable. The results are presented in Table 8-1 and Table 8-2.

<Evaluation criteria for optical density of lines>

+ + + +: The optical density was 250 or greater.
+ + +: The optical density was 150 or greater but less than 250.
+ +: The optical density was 50 or greater but less than 150.
+: The optical density was 20 or greater but less than 50.
-: The optical density was less than 20, or was unmeasurable because no lines were recognized.

<Evaluation criteria for variation of optical density of lines>

A: The standard deviation was within 20%.
B: The standard deviation was beyond 20% but within 40%.
C: The standard deviation was beyond 40% but within 60%.
D: The standard deviation was beyond 60%.

(Example 110)

A lateral flow chromatographic device for nucleic acid detection of Example 110 was produced in the same

manner as in Example 102 and evaluated in the same manners as in Example 109. The results are presented in Table 8-1 and Table 8-2.

(Example 111)

A lateral flow chromatographic device for nucleic acid detection of Example 111 was produced in the same manner as in Example 103 and evaluated in the same manners as in Example 109. The results are presented in Table 8-1 and Table 8-2.

(Example 112)

A lateral flow chromatographic device for nucleic acid detection of Example 112 was produced in the same manner as in Example 104 and evaluated in the same manners as in Example 109. The results are presented in Table 8-1 and Table 8-2.

(Example 113)

A lateral flow chromatographic device for nucleic acid detection of Example 113 was produced in the same manner as in Example 105 and evaluated in the same manners as in Example 109. The results are presented in Table 8-1 and Table 8-2.

(Example 114)

A lateral flow chromatographic device for nucleic acid detection of Example 114 was produced in the same manner as in Example 106 and evaluated in the same manners as in Example 109. The results are presented in Table 8-1 and Table 8-2.

(Example 115)

A lateral flow chromatographic device for nucleic acid detection of Example 115 was produced in the same manner as in Example 107 and evaluated in the same manners as in Example 109. The results are presented in Table 8-1 and Table 8-2.

[Table 8-1]

| | Linker | Testing target liquid concentration (M) | | | | | | |
|---|---|---|---|---|---|---|---|---|
| | | $1\times10^{-13}$ | $1\times10^{-12}$ | $1\times10^{-11}$ | $1\times10^{-11}$ | $1\times10^{-9}$ | $1\times10^{-11}$ | $1\times10^{-7}$ |
| Ex. 109 | GMBS | - | ± | ++ | +++ | ++++ | ++++ | ++++ |
| Ex. 110 | SM(PEG)2 | - | - | + | ++ | ++++ | ++++ | ++++ |
| Ex. 111 | SM(PEG)4 | - | ± | ± | ++ | +++ | ++++ | +++ |
| Ex. 112 | SM(PEG)6 | - | - | ++ | ++ | ++++ | ++++ | + |
| Ex. 113 | SM(PEG)8 | - | - | ± | - | +++ | +++ | ++ |
| Ex. 114 | SM(PEG)12 | - | - | + | ± | + | +++ | - |
| Ex. 115 | SM(PEG)24 | - | - | - | ++ | +++ | ++++ | - |

[Table 8-2]

| | Variation | Detectable concentration range (M) |
|---|---|---|
| Ex. 109 | A | $1\times10^{5}$ |
| Ex. 110 | A | $1\times10^{5}$ |
| Ex. 111 | A | $1\times 10^{4}$ |
| Ex. 112 | B | $1\times10^{4}$ |

(continued)

| | Variation | Detectable concentration range (M) |
|---|---|---|
| Ex. 113 | C | $1 \times 10^3$ |
| Ex. 114 | C | $1\times 10^2$ |
| Ex. 115 | C | $1 \times 10^3$ |

From the results of Table 8-1 and Table 8-2, Examples 109 to 111 had a small variation in optical density.

Examples 109 to 112 had a detectable centration range of $1 \times 10^4$ M or greater, which was a broad detectable concentration range. Particularly, Examples 109 and 110 exhibited a broad detectable concentration range of $1 \times 10^5$ M or greater.

(Preparation example 201)

-Preparation of back layer coating liquid-

A silicone-based rubber emulstion (available from Shin-Etsu Chemical Co., Ltd., KS779H, with a solid concentration of 30% by mass) (16.8 parts by mass), a chloroplatinic acid catalyst (0.2 parts by mass), and toluene (83 parts by nass) were mixed, to obtain a back layer coating liquid.

(Preparation example 202)

-Preparation of release layer coating liquid-

A polyethylene wax (available from Toyo ADL Corporation, POLYWAX 1000, with a melting point of 99 degrees C and a penetration of 2 at 25 degrees C) (14 parts by mass), an ethylene-vinyl acetate copolymer (available from Du Pont-Mitsui Polychemicals Co., Ltd., EV-150, with a weight average molecular weight of 2,100 and VAc of 21% by mass) (6 parts by mass), toluene (60 parts by mass), and methyl ethyl ketone (20 parts by mass) were subjected to dispersion treatment until the average particle diameter became 2.5 micrometers, to obtain a release layer coating liquid.

(Preparation example 203)

-Preparation of reagent immobilized layer coating liquid-

An aminoethylated acrylic polymer (POLYMENT NK-380, available from Nippon Shokubai Co., Ltd.) was diluted to 15% by mass by addition of toluene as a solvent, to obtain a reagent immobilized layer coating liquid. The aminoethylated acrylic polymer is known to easily deteriorate and may highly assumedly have impact on immobilization of the reagent. Therefore, the preparation of the aminoethylated acrylic polymer was on an on demand basis.

(Preparation example 204)

-Preparation of test line reagent coating liquid-

A DNA fragment that continuously contained 20 bases of thymine (T) and to which a carboxyl group was introduced at a 5' end was prepared to have a final concentration of 2.5 $\mu$M with a MES buffer (available from Dojindo Laboratories) having a final concentration of 100 mM. To the resultant, EDC (available from Thermo Fisher Scientific Inc.) having a final concentration of 1.25 mg/mL was added as a linker, to obtain a test line reagent coating liquid.

(Preparation example 205)

-Preparation of control line reagent coating liquid-

A DNA fragment that continuously contained 20 bases of cytosine (C) and to which a carboxyl group was introduced at a 3' end was prepared to have a final concentration of 2.5 $\mu$M with a MES buffer (available from Dojindo Laboratories) having a final concentration of 100 mM. To the resultant, EDC (available from Thermo Fisher Scientific Inc.) having a final concentration of 1.25 mg/mL was added as a linker, to obtain a control line reagent coating liquid.

(Preparation example 206)

-Preparation of label body (nucleic acid) reagent coating liquid-

Gold colloid modified with a carboxyl group was bound by amide binding with a DNA fragment that continuously contained 20 bases of guanine (G) and to which an amino group was introduced at a 3' end using 1-ethyl-3-(3-dimethylaminopropyl)carbodiimide (EDC) (available from Thermo Fisher Scientific Inc.). The resultant was washed with a 50mM Tris-HCl buffer (pH=8.2), suspended in a label body diluting fluid (a 20mM Tris-HCl buffer (pH=8.2), 0.05% by mass polyethylene glycol, 5% by mass sucrose, and purified water), and adjusted to OD=2, to obtain a label body reagent coating liquid.

(Preparation example 207)

-Preparation of test line reagent coating liquid-

A DNA fragment that continuously contained 20 bases of thymine (T) and to which a carboxyl group and 1 molecule of a C3 linker were introduced at a 5' end was prepared to have a final concentration of 2.5 $\mu$M with a MES buffer (available from Dojindo Laboratories) having a final concentration of 100 mM. To the resultant, EDC (available from Thermo Fisher Scientific Inc.) having a final concentration of 1.25 mg/mL was added as a linker, to obtain a test line reagent coating liquid. The DNA fragment is represented by a general formula A below where n=1.

<General formula A>

[Chem. 9]

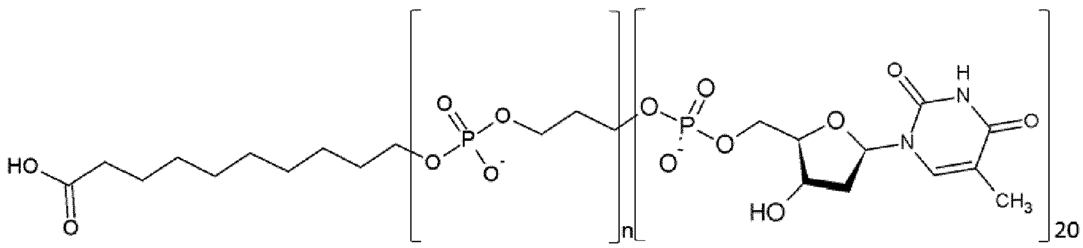

In general formula A, n represents an integer of preferably 20 or lower and more preferably from 0 through 5.

(Preparation example 208)

-Preparation of test line reagent coating liquid-

A DNA fragment that continuously contained 20 bases of thymine (T) and to which a carboxyl group and 2 molecules of a C3 linker were introduced at a 5' end was prepared to have a final concentration of 2.5 $\mu$M with a MES buffer (available from Dojindo Laboratories) having a final concentration of 100 mM. To the resultant, EDC (available from Thermo Fisher Scientific Inc.) having a final concentration of 1.25 mg/mL was added as a linker, to obtain a test line reagent coating liquid. The DNA fragment of Preparation example 208 is represented by the general formula A above where n=2.

(Preparation example 209)

-Preparation of test line reagent coating liquid-

A DNA fragment that continuously contained 20 bases of thymine (T) and to which a carboxyl group and 3 molecules of a C3 linker were introduced at a 5' end was prepared to have a final concentration of 2.5 $\mu$M with a MES buffer (available from Dojindo Laboratories) having a final concentration of 100 mM. To the resultant, EDC (available from Thermo Fisher Scientific Inc.) having a final concentration of 1.25 mg/mL was added as a linker, to obtain a test

line reagent coating liquid. The DNA fragment of Preparation example 209 is represented by the general formula A above where n=3.

(Preparation example 210)

-Preparation of test line reagent coating liquid-

A DNA fragment that continuously contained 20 bases of thymine (T) and to which a carboxyl group and 4 molecules of a C3 linker were introduced at a 5' end was prepared to have a final concentration of 2.5 $\mu$M with a MES buffer (available from Dojindo Laboratories) having a final concentration of 100 mM. To the resultant, EDC (available from Thermo Fisher Scientific Inc.) having a final concentration of 1.25 mg/mL was added as a linker, to obtain a test line reagent coating liquid. The DNA fragment of Preparation example 210 is represented by the general formula A above where n=4.

(Preparation example 211)

-Preparation of test line reagent coating liquid-

A DNA fragment that continuously contained 20 bases of thymine (T) and to which a carboxyl group and 5 molecules of a C3 linker were introduced at a 5' end was prepared to have a final concentration of 2.5 $\mu$M with a MES buffer (available from Dojindo Laboratories) having a final concentration of 100 mM. To the resultant, EDC (available from Thermo Fisher Scientific Inc.) having a final concentration of 1.25 mg/mL was added as a linker, to obtain a test line reagent coating liquid. The DNA fragment of Preparation example 211 is represented by the general formula A above where n=5.

(Preparation example 212)

-Preparation of reagent immobilized layer coating liquid-

A carboxyl group-containing ester resin (AP2510, available from Arakawa Chemical Industries, Ltd.) was diluted to 15% by mass by addition of a methyl ethyl ketone/toluene mixed liquid (at a volume ratio of 6:4) as a solvent, to obtain a reagent immobilized layer coating liquid.

(Preparation example 213)

-Preparation of test line reagent coating liquid-

A DNA fragment that continuously contained 20 bases of thymine (T) and to which an amino group was introduced at a 5' end was prepared to have a final concentration of 2.5 $\mu$M with a MES buffer (available from Dojindo Laboratories) having a final concentration of 100 mM. To the resultant, EDC (available from Thermo Fisher Scientific Inc.) having a final concentration of 1.25 mg/mL was added as a linker, to obtain a test line reagent coating liquid.

(Preparation example 214)

-Preparation of control line reagent coating liquid-

A DNA fragment that continuously contained 20 bases of cytosine (C) and to which an amino group was introduced at a 3' end was prepared to have a final concentration of 2.5 $\mu$M with a MES buffer (available from Dojindo Laboratories) having a final concentration of 100 mM. To the resultant, EDC (available from Thermo Fisher Scientific Inc.) having a final concentration of 1.25 mg/mL was added as a linker, to obtain a control line reagent coating liquid.

(Preparation example 215)

-Preparation of test line reagent coating liquid-

A DNA fragment that continuously contained 20 bases of thymine (T) and to which an amino group and 5 molecules of a C3 linker were introduced at a 5' end was prepared to have a final concentration of 2.5 $\mu$M with a MES buffer (available from Dojindo Laboratories) having a final concentration of 100 mM. To the resultant, EDC (available

from Thermo Fisher Scientific Inc.) having a final concentration of 1.25 mg/mL was added as a linker, to obtain a test line reagent coating liquid.

(Preparation example 216)

-Preparation of label body (antibody) reagent coating liquid-

To a gold colloid solution (available from BBI Solutions, EMGC50) (9 mL), a $KH_2PO_4$ buffer (pH=7.0) (1 mL) prepared to 50 mM and then an anti-biotin monoclonal antibody (available from Bethyl Laboratories, Inc., ANTI-BIOTIN, GOAT-POLY A150-111A) (1 mL) prepared to 50 micrograms/mL were added and stirred. The resultant was left to stand still for 10 minutes. To the resultant, a 1% by mass polyethylene glycol aqueous solution (available from Wako Pure Chemical Industries, Ltd., 168-11285) (550 microliters) was added and stirred. To the resultant, a 10% by mass BSA aqueous solution (available from Sigma-Aldrich Co., LLC, A-7906) (1.1 mL) was further added and stirred.

Next, this solution was subjected to centrifugation for 30 minutes, and then, after the supernatant being removed, subjected to re-dispersion of the gold colloid using an ultrasonic cleaner. The centrifugation was performed with a centrifuge (available from Hitachi Koki Co., Ltd., HIMAC CF16RN) at a centrifugal acceleration of 8,000 $\times$ g at 4 degrees C. Subsequently, the resultant was dispersed in a gold colloid preservative solution [a 20 mM Tris-HCl buffer (pH=8.2), 0.05% by mass polyethylene glycol (with a weight average molecular weight of 2,000), 150 mM NaCl, a 1% by mass BSA aqueous solution, and a 0.1% by mass $NaN_3$ aqueous solution] (20 mL), subjected again to centrifugation under the same conditions as described above, and after the supernatant being removed except about 1 mL, subjected to re-dispersion of the gold colloid using an ultrasonic cleaner. These operations were repeated to prepare the solution to be OD=15 in the gold colloid preservative solution, to obtain a label body reagent coating liquid.

(Preparation example 217)

-Preparation of test line reagent coating liquid-

A DNA fragment that continuously contained 20 bases of thymine (T) and to which a carboxyl group was introduced at a 5' end was prepared to be 25 $\mu$M with a TE buffer (10 mM Tris-HCl and 1 mM EDTA, pH=7.4, available from Takara Bio Inc.), to obtain a test line reagent coating liquid.

(Preparation example 218)

-Preparation of control line reagent coating liquid-

A DNA fragment that continuously contained 20 bases of cytosine (C) and to which a carboxyl group was introduced at a 3' end was prepared to be 25 $\mu$M with a TE buffer (10 mM Tris-HCl and 1 mM EDTA, pH=7.4, available from Takara Bio Inc.), to obtain a control line reagent coating liquid.

(Preparation example 219)

-Preparation of test line reagent coating liquid-

A DNA fragment that continuously contained 20 bases of thymine (T) and to which a carboxyl group was introduced at a 5' end was prepared to have a final concentration of 2.5 $\mu$M with a MES buffer (available from Dojindo Laboratories) having a final concentration of 100 mM, to obtain a test line reagent coating liquid.

(Preparation example 220)

-Preparation of control line reagent coating liquid-

A DNA fragment that continuously contained 20 bases of cytosine (C) and to which a carboxyl group was introduced at a 3' end was prepared to have a final concentration of 2.5 $\mu$M with a MES buffer (available from Dojindo Laboratories) having a final concentration of 100 mM, to obtain a control line reagent coating liquid.

(Example 201)

<Production of transfer medium for test line>

-Formatin of back layer-

The back layer coating liquid of Preparation example 201 was coated over one surface of a support, which was a polyethylene terephthalate (PET) film having an average thickness of 4.5 micrometers (available from Toray Industries, Inc. LUMIRROR F57) and dried at 80 degrees C for 10 seconds, to form a back layer having an average thickness of 0.02 micrometers.

-Formation of release layer-

Next, the release layer coating liquid of Preparation example 202 was coated over a surface of the PET film opposite to the surface over which the back layer was formed and dried at 25 degrees C for 30 minutes, to form a release layer having an average thickness of 30 micrometers.

-Formation of reagent immobilized layer-

Next, the reagent immobilized layer coating liquid of Preparation example 203 was coated over the surface of the release layer and dried in vacuum at room temperature for 30 minutes, to form a reagent immobilized layer having an average thickness of 6 micrometers. In this way, a transfer medium was produced.

<Immobilization of capture nucleic acids>

-Test line (immobilization of first capture nucleic acid)-

Next, the test line reagent coating liquid of Preparation example 204 was poured into a shallow square vat, and the transfer medium was floated over the test line reagent coating liquid such that only the reagent immobilized layer surface contacted the test line reagent coating liquid. In this state, the shallow square vat was covered with a lid and left to stand still at 25 degrees C for 2 hours. Subsequently, the reagent immobilized layer surface was washed with a 50 mM Tris-HCl buffer (pH=9.0) and subjected to air drying, to produce a transfer medium for a test line of Example 201. The transfer medium for a test line was used for production of a lateral flow chromatographic device for nucleic acid detection immediately after the transfer medium was produced.

It was confirmed that the first capture nucleic acid and the reagent immobilized layer were bound with each other by a FT-IR ATR method (FT-IR6800, available from JASCO Corporation) based on presence or absence of a spectrum attributable to an amide bond produced by the carboxyl group in the first capture nucleic acid newly forming an amide bond (covalent bond) with the amino group in the reagent immobilized layer.

<Production of transfer medium for control line>

-Control line (immobilization of second capture nucleic acid)-

A transfer medium for a control line of Example 201 was produced in the same manner as in <Production of transfer medium for test line> described above, except that unlike in <Production of transfer medium for test line>, the control line reagent coating liquid of Preparation example 205 was used instead of the test line reagent coating liquid. The transfer medium for a control line was used for production of a lateral flow chromatographic device for nucleic acid detection immediately after the transfer medium was produced.

It was confirmed that the second capture nucleic acid and the reagent immobilized layer were bound with each other by a FT-IR ATR method (FT-IR6800, available from JASCO Corporation) based on presence or absence of a spectrum attributable to an amide bond produced by the carboxyl group in the second capture nucleic acid newly forming an amide bond (covalent bond) with the amino group in the reagent immobilized layer.

<Production of testing device>

The testing device 10 illustrated in FIG. 1 and FIG. 2 was produced in the manner described below. FIG. 1 is a top view of the testing device of Example. FIG. 2 is a schematic cross-sectional view of the testing device of FIG. 1 taken along a line A-A. Immediately after the testing device was produced, reactions and evaluations were performed.

-Production of paper substrate (substrate+flow path member)-

As a thermoplastic resin, a polyester-based hot-melt adhesive (available from Toagosei Co., Ltd., ARONMELT PES375S40) was heated to 190 degrees C, and then with a roll coater, coated over a PET film (available from Toray Industries, Inc., LUMIRROR S10, with an average thickness of 50 micrometers) 20 cut into a size of 40 mm in width and 80 mm in length to have an average thickness of 50 micrometers over the PET film, to form an adhesive layer.

The PET film 20 over which the adhesive layer was formed was left to stand still for 2 hours or longer. Subsequently, a nitrocellulose membrane (available from Merck Millipore Corporation, HF180) cut into a size of 40 mm in width and 35 mm in length was overlapped with the surface of the adhesive layer at a position that was 33 mm from one end of the surface of the adhesive layer in the longer direction (this end being an upstream end, the opposite end being a downstream end) in a state that the nitrocellulose membrane and the surface of the adhesive layer coincided widthwise, and a load of 1 $kgf/cm^2$ was imposed on the overlapped product at a temperature of 150 degrees C for 10 seconds, to form a flow path member 30. Finally, the obtained product was cut along the longer direction of the product into a size of 4 mm in width and 80 mm in length, to obtain a paper substrate.

The voidage of the nitrocellulose membrane as the flow path member 30 of the paper substrate was calculated according to a calculation formula 1 below based on a basis weight ($g/m^2$) and an average thickness (micrometer) of the flow path member and the specific gravity of the component of the flow path member. As a result, the voidage of the nitrocellulose membrane was 70%.

<Calculation formula 1>

$$\text{Voidage (\%)} = \{1 - [\text{basis weight } (g/m^2)/\text{average thickness (micrometer)}/\text{specific gravity of the component}]\} \times 100$$

When the voidage of the flow path member is 40% or greater but 90% or less, the flow path member can be said to be porous.

-Transfer of test line-

The flow path member 30 of the paper substrate and the reagent-immobilized side of the transfer medium for a test line were faced and overlapped with each other. Subsequently, with a thermal transfer printer, as illustrated in FIG. 1 and FIG. 2, the transfer medium for a test line was transferred onto a position that was apart by 9 mm from the upstream end of the flow path member 30 in a line shape having a width of 4 mm and a length of 0.7 mm (first detecting portion 50a).

As the thermal transfer printer, an evaluation system having a printing speed of 42 mm/sec and an applied energy of 0.17 mJ/dot was constructed with a thermal head having a dot density of 300 dpi (available from TDK Corporation).

-Transfer of control line-

Next, the transfer medium for a control line was transferred onto a position that was apart by 5 mm from the position onto which the transfer medium for a test line was transferred in a line shape having a width of 4 mm and a length of 0.7 mm (second detecting portion 50b).

-Formation of label body supplying portion-

Next, the label body reagent coating liquid of Preparation example 206 was coated in an amount of 60 microliters/$cm^2$ over a glass-fiber pad (available from Merck Millipore Corporation, GFCP203000) cut into a size of 4 mm in width and 18 mm in length, and dried overnight at a reduced pressure to produce a label body supporting pad.

As illustrated in FIG. 1 and FIG. 2, the label body supporting pad was disposed at a position that was apart by 17 mm from the upstream end of the paper substrate, and overlapped with and pasted over the adhesive layer provided over the paper substrate (label body supplying portion 40).

-Formation of dropping portion-

As illustrated in FIG. 1 and FIG. 2, a sample pad (available from Merck Millipore Corporation, CFSP223000) having a width of 4 mm and a length of 35 mm was disposed and pasted in a manner to overlap with the upper surface of the label body supplying portion 40 by 18 mm (dropping portion 80).

-Absorbing member-

An absorbing member 70 (available from Merck Millipore Corporation, CFSP223000) was provided as illustrated in FIG. 1 and FIG. 2. In this way, a lateral flow chromatographic device for nucleic acid detection (testing device 10) of Example 201 was obtained.

<Evaluation of lines>

-Preparation of testing target liquid-

A DNA fragment incluing a base sequence that contained from a 5' end, 20 bases of cytosine (C) continuously, a sequence containing 10 bases of thymine (T) and guanine (G) repeatedly, and 20 bases of adenine (A) continuously was prepared to have a final concentration of 10 pM, a final concentration of 1 nM, and a final concentration of 100 nM with a 5 × SSC buffer (75 mM sodium citrate and 750 mM sodium chloride available from Nacalai Tesque, Inc.), to obtain testing target liquids.

- Reaction-

The testing target liquid having a final concentration of 1 nM was dropped in an amount of 100 microliters onto the upstream end portion of the lateral flow chromatographic device 10 for nucleic acid detection illustrated in FIG. 1 and FIG. 2. Thirty minutes later, the lines were evaluated according to the criteria described below based on visual observation. The result is presented in Table 10.

<Evaluation criteria>

A: A clear color development was recognized at the positions of the test line and the control line at a uniform color optical density throughout the lines without discontinuation of the lines.
B: The lines were not discontinuous and enabled judgement but were slightly non-uniform in the color optical density from place to place.
C: Color development was barely recognized as line shapes but with a partial discontinuation in the lines.
D: No color development was recognized or color development was not in line shapes such as when the lines flowed to the downstream side.

Examples of the evaluation criteria are presented in Table 9. The photographs in Table 9 are each a photograph of the test line after testing.

[Table 9]

| Evaluation criteria | A | B | C | D |
|---|---|---|---|---|
| Photograph | 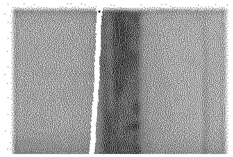 | 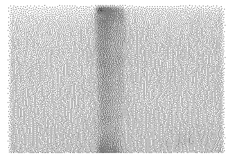 | 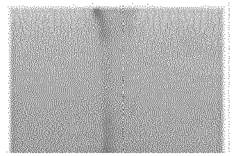 | 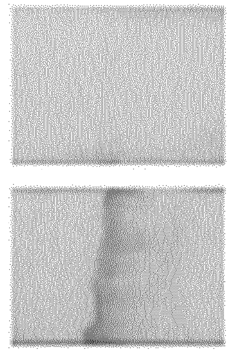 |

<Measurement of optical density of lines>

The lateral flow chromatographic device for nucleic acid detection after having developed colors and used in the evaluation of lines above was measured with a chromatoreader (available from Hamamatsu Photonics K.K., C10066), to obtain the optical density of the lines. The optical density of the lines was evaluated according to the criteria described below. The results are presented in Table 11. A greater optical density of the lines is more preferable. When no line optical density was detected by the chromatoreader, the result is presented as "-". When an optical density by this chromatoreader is 20 or greater, the color development can be recognized visually.

<Evaluation criteria for optical density of lines>

+ + + +: The optical density was 250 or greater.
+ + +: The optical density was 150 or greater but less than 250.
+ +: The optical density was 50 or greater but less than 150.
+: The optical density was 20 or greater but less than 50.
-: The optical density was less than 20, or was unmeasurable because no lines were recognized.

(Example 202)

A lateral flow chromatographic device for nucleic acid detection (testing device 10) of Example 202 was produced in the same manner as in Example 201, except that unlike in Example 201, the test line reagent coating liquid of Preparation example 207 was used in <Production of transfer medium for test line>, and was evaluated in the same manners as in Example 201. The results are presented in Table 10 and Table 11.

In Example 202, it was confirmed that the first capture nucleic acid and the reagent immobilized layer were bound with each other by a FT-IR ATR method in the same manner as in Example 201 based on presence or absence of a spectrum attributable to an amide bond produced by the carboxyl group in the first capture nucleic acid newly forming an amide bond (covalent bond) with the amino group in the reagent immobilized layer.

(Example 203)

A lateral flow chromatographic device for nucleic acid detection (testing device 10) of Example 203 was produced in the same manner as in Example 201, except that unlike in Example 201, the test line reagent coating liquid of Preparation example 208 was used in <Production of transfer medium for test line>, and was evaluated in the same manners as in Example 201. The restuls are presented in Table 10 and Table 11.

In Example 203, it was confirmed that the first capture nucleic acid and the reagent immobilized layer were bound with each other by a FT-IR ATR method in the same manner as in Example 201 based on presence or absence of a spectrum attributable to an amide bond produced by the carboxyl group in the first capture nucleic acid newly forming an amide bond (covalent bond) with the amino group in the reagent immobilized layer.

(Example 204)

A lateral flow chromatographic device for nucleic acid detection (testing device 10) of Example 204 was produced in the same manner as in Example 201, except that unlike in Example 201, the test line reagent coating liquid of Preparation example 209 was used in <Production of transfer medium for test line>, and was evaluated in the same manners as in Example 201. The restuls are presented in Table 10 and Table 11.

In Example 204, it was confirmed that the first capture nucleic acid and the reagent immobilized layer were bound with each other by a FT-IR ATR method in the same manner as in Example 201 based on presence or absence of a spectrum attributable to an amide bond produced by the carboxyl group in the first capture nucleic acid newly forming an amide bond (covalent bond) with the amino group in the reagent immobilized layer.

(Example 205)

A lateral flow chromatographic device for nucleic acid detection (testing device 10) of Example 205 was produced in the same manner as in Example 201, except that unlike in Example 201, the test line reagent coating liquid of Preparation example 210 was used in <Production of transfer medium for test line>, and was evaluated in the same manners as in Example 201. The restuls are presented in Table 10 and Table 11.

In Example 205, it was confirmed that the first capture nucleic acid and the reagent immobilized layer were bound with each other by a FT-IR ATR method in the same manner as in Example 201 based on presence or absence of a spectrum attributable to an amide bond produced by the carboxyl group in the first capture nucleic acid newly forming an amide bond (covalent bond) with the amino group in the reagent immobilized layer.

(Example 206)

A lateral flow chromatographic device for nucleic acid detection (testing device 10) of Example 206 was produced in the same manner as in Example 201, except that unlike in Example 201, the test line reagent coating liquid of Preparation example 211 was used in <Production of transfer medium for test line>, and was evaluated in the same manners as in Example 201. The restuls are presented in Table 10 and Table 11.

In Example 206, it was confirmed that the first capture nucleic acid and the reagent immobilized layer were bound with each other by a FT-IR ATR method in the same manner as in Example 201 based on presence or absence of a spectrum attributable to an amide bond produced by the carboxyl group in the first capture nucleic acid newly forming an amide bond (covalent bond) with the amino group in the reagent immobilized layer.

(Example 207)

A lateral flow chromatographic device for nucleic acid detection (testing device 10) of Example 207 was produced in the same manner as in Example 201, except that unlike in Example 201, the reagent immobilized layer coating liquid of Preparation example 212 was used in - Formation of reagent immobilized layer-, the test line reagent coating liquid of Preparation example 213 was used in <Production of transfer medium for test line>, and the control line reagent coating liquid of Preparation example 214 was used in <Production of transfer medium for control line>, and was evaluated in the same manners as in Example 201. The restuls are presented in Table 10 and Table 11.

In Example 207, it was confirmed that the first capture nucleic acid and the reagent immobilized layer were bound with each other by a FT-IR ATR method (FT-IR6800, available from JASCO Corporation) based on presence or absence of a spectrum attributable to an amide bond produced by the amino group in the first capture nucleic acid newly forming an amide bond (covalent bond) with the carboxyl group in the reagent immobilized layer. In Example 207, it was confirmed that the second capture nucleic acid and the reagent immobilized layer were bound with each other by a FT-IR ATF method (FT-IR6800, available from JASCO Corporation) based on presence or absence of a spectrum attributable to an amide bond produced by the amino group in the second capture nucleic acid newly forming an amide bond (covalent bond) with the carboxyl group in the reagent immobilized layer.

(Example 208)

A lateral flow chromatographic device for nucleic acid detection (testing deivce 10) of Example 208 was produced in the same manner as in Example 207, except that unlike in Example 207, the test line reagent coating liquid of Preparation example 215 was used in <Production of transfer medium for test line>, and was evaluated in the same manners as in Example 201. The results are presented in Table 10 and Table 11.

In Example 208, it was confirmed that the first capture nucleic acid and the reagent immobilized layer were bound with each other by a FT-IR ATR method (FT-IR6800, available from JASCO Corporation) based on presence or absence of a spectrum attributable to an amide bond produced by the amino group in the first capture nucleic acid newly forming an amide bond (covalent bond) with the carboxyl group in the reagent immobilized layer.

(Example 209)

A lateral flow chromatographic device for nucleic acid detection (testing device 10) of Example 209 onto which only a test line was transferred was produced in the same manner as in Example 201 except that unlike in Example 201, the label body reagent coating liquid of Preparation example 216 was used in -Formation of label body supplying portion-, and was evaluated in the same manners as in Example 201 except that in <Evaluation of lines>, a testing target liquid was prepared using a DNA fragment that was labeled with biotin at a 5' end and included a base sequence that contained from the 5' end, a sequence containing 10 bases of thymine (T) and guanine (G) repeatedly, and 20 bases of adenine (A) continuously.

(Example 210)

A lateral flow chromatographic device for nucleic acid detection (testing device 10) of Example 210 onto which only a test line was transferred was produced in the same manner as in Example 206 except that unlike in Example

206, the label body reagent coating liquid of Preparation example 216 was used in -Formation of label body supplying portion-, and was evaluated in the same manners as in Example 201 except that in <Evaluation of lines>, a testing target liquid was prepared using a DNA fragment that was labeled with biotin at a 5' end and included a base sequence that contained from the 5' end, a sequence containing 10 bases of thymine (T) and guanine (G) repeatedly, and 20 bases of adenine (A) continuously.

(Comparative Example 201)

<Production of testing device>

A testing device 10 illustrated in FIG. 9 and FIG. 10 was produced in the manner described below. FIG. 9 is a top view of a testing device of Comparative Example. FIG. 10 is a schematic cross-sectional view of the testing device of FIG. 9 taken along a line B-B.

-Production of paper substrate-

As a thermoplastic resin, a polyester-based hot-melt adhesive (available from Toagosei Co., Ltd., ARONMELT PES375S40) was heated to 190 degrees C, and then with a roll coater, coated over a PET film (available from Toray Industries, Inc., LUMIRROR S10, with an average thickness of 50 micrometers) cut into a size of 40 mm in width and 35 mm in length to have an average thickness of 50 micrometers over the PET film, to form an adhesive layer.

The PET film over which the adhesive layer was formed was left to stand still for 2 hours or longer. Subsequently, a nitrocellulose membrane (available from Merck Millipore Corporation, HF180) functioning as a flow path member 30 and cut into the same size as the PET film was overlapped with the adhesive layer-formed surface, and a load of 1 kgf/cm$^2$ was imposed on the overlapped product at a temperature of 150 degrees C for 10 seconds, to form a paper substrate.

-Immobilization of capture nucleic acids-

As illustrated in FIG. 9 and FIG. 10, with a positive-pressure spray device (available from BioDot, Inc., BIOJET), the test line reagent coating liquid of Preparation example 217 was coated at a position that was apart by 9 mm from the upstream end of the flow path member 30 of the paper substrate in a line shape having a length of 0.7 mm (test line 90a).

With the positive-pressure spray device, the control line reagent coating liquid of Preparation example 218 was coated at a position that was apart by 5 mm from the test line 90a in a line shape having a length of 0.7 mm (control line 90b).

After coating, the coating liquids were dried at 20 degrees C at 20 RH% for 16 hours.

It was confirmed that the capture nucleic acids were not bound with the flow path member by a FT-IR ATR method in the same manner as in Example 201 based on an analysis of the surface of the flow path member, proving that there was no spectrum change between before and after the immobilization.

-Formation of label body supplying portion-

Next, the label body reagent coating liquid of Preparation example 206 was coated in an amount of 60 microliters/cm$^2$ over a glass-fiber pad (available from Merck Millipore Corporation, GFCP203000) cut into a size of 40 mm in width and 18 mm in length, and dried overnight at a reduced pressure, to produce a label body supporting pad.

-Assembly of assay (testing device)-

The flow path member 30 was pasted over a base film, which was a PET film (available from Toray Industries, Inc. LUMIRROR S10, with an average thickness of 100 micrometers) cut into a size of 40 mm in width and 80 mm in length, at a position that was apart by 33 mm from one end of the base film (PET film) in the longer direction of the base film (PET film), in a state that the side of the flow path member opposite to the reagent-coated surface faced the base film (PET film).

Next, the label body supporting pad produced above and having a size of 40 mm in width and 18 mm in length was disposed and pasted over the top surface of the flow path member 30 in a manner to overlap the upstream end of the flow path member 30 by 2 mm (label body supplying portion 40), and a sample pad (available from Merck Millipore Corporation, CFSP223000) having a size of 40 mm in width and 35 mm in length was disposed and pasted in a manner to overlap the top surface of the label body supporting pad by 18 mm to produce a sample dropping pad (dropping portion) 80.

Next, an absorbing pad (available from Merck Millipore Corporation, CFSP223000) having a size of 40 mm in

width and 28 mm in length was disposed and pasted over the top surface of the flow path member 30 in a manner to overlap the downstream end of the flow path member 30 by 16 mm to provide an absorbing member 70. Finally, the obtained product was cut along the longer direction of the product into a size of 4 mm in width and 80 mm in length, to obtain a lateral flow chromatographic device for nucleic acid detection (testing device 10) of Comparative Example 201.

The produced lateral flow chromatographic device for nucleic acid detection of Comparative Example 201 was evaluated in the same manners as in Example 201. The results are presented in Table 10 and Table 11.

(Comparative Example 202)

The lateral flow chromatographic device for nucleic acid detection (testing device 10) of Comparative Example 202 was produced in the same manner as in Example 201, except that unlike in Example 201, the test line reagent coating liquid of Preparation example 219 was used in <Production of transfer medium for test line> and the control ine reagent coating liquid of Preparation example 220 was used in <Production of transfer medium for control ine>, and was evaluated in the same manners as in Example 201. The results are presented in Table 10 and Table 11.

In Comparative Example 202, it was confirmed that the capture nucleic acids were not bound with the flow path member by a FT-IR ATR method in the same manner as in Example 201 based on an analysis of the surface of the flow path member, proving that there was no spectrum change between before and after the immobilization.

[Table 10]

| | Reagent immobilized layer | | Capture nucleic acid | | Label body | Photograph (test line) | Evaluation result |
|---|---|---|---|---|---|---|---|
| | Resin | Functional group | Functional group | Spacer length (number of binding) | | | |
| Ex. 201 | Acrylic | Amino group | Carboxyl group | 10 | Nucleic acid | 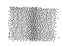 | A |
| Ex. 202 | Acrylic | Amino group | Carboxyl group | 16 | Nucleic eic acid | 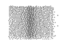 | A |
| Ex. 203 | Acrylic | Amino group | Carboxyl group | 22 | Nucleic acid | 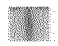 | A |
| Ex. 204 | Acrylic | Amino group | Carboxyl group | 18 | Nucleic acid | 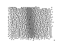 | A |
| Ex. 205 | Acrylic | Amino group | Carboxyl group | 34 | Nucleic acid | 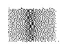 | A |
| Ex. 206 | Acrylic | Amino group | Carboxyl group | 40 | Nucleic acid | 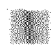 | A |
| Ex. 207 | Ester | Carboxyl group | Amino group | 7 | Nucleic acid | 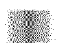 | A |
| Ex. 208 | Ester | Carboxyl group | Amino group | 37 | Nucleic acid | 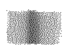 | A |
| Ex. 209 | Acrylic | Amino group | Carboxyl group | to | Antibody | 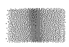 | A |
| Ex. 210 | Acrylic | Amino group | Carboxyl group | 40 | Antibody | 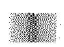 | A |
| Comp. Ex. 201 | None (direct | coating/ drying) | Carboxyl group | 10 | Nucleic acid | 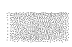 | D |
| Comp. Ex. 202 | Acrylic | Amino group | Carboxyl group | 10 | Nucleic acid | 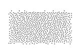 | D |

[Table 11]

| | Concentration of testing target liquid | | |
|---|---|---|---|
| | 10 pM | 1 nM | 100 nM |
| Ex. 201 | + | ++++ | ++++ |
| Ex. 202 | ++ | ++++ | ++++ |
| Ex. 203 | ++ | ++++ | ++++ |
| Ex. 204 | ++ | ++++ | ++++ |
| Ex. 205 | ++ | ++++ | ++++ |
| Ex. 206 | ++ | ++++ | ++++ |
| Ex. 207 | + | ++++ | ++++ |
| Ex. 208 | ++ | ++++ | ++++ |
| Ex. 209 | + | ++++ | ++++ |
| Ex.210 | ++ | ++++ | ++++ |
| Comp. Ex. 201 | + | + | ++ |
| Comp. Ex. 202 | - | - | - |

From the results of Table 10 and Table 11, in Examples 201 to 210, in the evaluation of visibility, it was possible to observe lines having a uniform color optical density throught the lines and having a high visibility. In the evaluation of optical density, it was possible to observe lines having a high density, and good results were obtained when capture nucleic acids having a spacer length corresponding to the number of atoms in the main chain of 16 or greater were used.

As compared with this, in Comparative Examle 201 in which the capture nucleic acids were directly coated over the flow path member and dried, in the evaluation of visibility, color development was observed but blurring near the lines was so severe that it was barely possible to observe the color development.

Likewise, in Comparative Example 202, it was impossible to observe color development on the lines.

[Reference Signs List]

10: testing device
12: testing target liquid
14: target nucleic acid
15: compound or protein bound with target nucleic acid
16: label body (nucleic acid) (an example of a reagent)
17: first capture nucleic acid (an example of a reagent)
18: second capture nucleic acid (an example of a reagent)
19: label body (antibody) (an example of a reagent)
20: base material
30: flow path member
40: label body supplying portion
50a: first detecting portion
50b: second detecting portion
100: transfer medium for producing a testing device
101: support
102: release layer
103: reagent immobilized layer
104: back layer
200: testing kit
201: sterilized cotton swab
202: diluting fluid

## Claims

1. A testing device (10) comprising:

   a porous flow path member (30) constituting a flow path through which a testing target liquid (12) is flowed;
   a testing target liquid dropping portion provided on the flow path member (30);
   a labeling portion configured to apply a label to a target nucleic acid (14) when the target nucleic acid (14) is contained in the testing target liquid (12) dropped onto the testing target liquid dropping portion; and
   a detecting portion configured to detect the target nucleic acid (14) labeled at the labeling portion,
   wherein the testing device (10) comprises a shaped body formed of a resin on the flow path member (30) at the detecting portion, and
   wherein a capture nucleic acid including a sequence bindable and complementary with the target nucleic acid (14) is immobilized by covalent binding to a surface of the shaped body between the shaped body and the flow path member (30).

2. The testing device (10) according to claim 1,
   wherein the covalent binding comprises at least one selected from the group consisting of amide binding, ether binding, and thioether binding.

3. The testing device (10) according to claim 2,
   wherein the capture nucleic acid is single-stranded and hybridizable with the target nucleic acid (14).

4. The testing device (10) according to claim 3,
   wherein the covalent binding is formed by reaction of at least one functional group selected from the group consisting of an amino group, a carboxyl group, a hydroxyl group, and a thiol group on the surface of the shaped body and in the capture nucleic acid.

5. The testing device (10) according to claim 1,
   wherein the capture nucleic acid including a sequence bindable and complementary with the target nucleic acid (14) is bound by a linker with the shaped body.

6. The testing device (10) according to claim 5,
   wherein the shaped body comprises a functional group having reactivity, and
   wherein the capture nucleic acid is bound with the surface of the shaped body via the functional group and the linker.

7. The testing device (10) according to claim 6,
   wherein the shaped body comprises an amino group as the functional group.

8. The testing device (10) according to claim 7,
   wherein the linker comprises an N-hydroxysuccinic acid imide ester group at one end and is bound with the amino group on the surface of the shaped body by amide binding.

9. The testing device (10) according to any one of claims 5 to 8,
   wherein the linker comprises a maleimide group at one end and is bound with a thiol group introduced at a 5’ end or a 3’ end of the capture nucleic acid by thioether binding.

10. The testing device (10) according to claim 1,
    wherein the capture nucleic acid including: a sequence bindable and complementary with the target nucleic acid (14); and a spacer is immobilized to the surface of the shaped body.

11. The testing device (10) according to claim 10,
    wherein the spacer comprises an alkyl group, or an alkyl group and a phosphoric acid group.

12. A transfer medium (100) for producing a testing device (10), the transfer medium (100) being intended for producing the testing device (10) according to any one of claims 1 to 11, the transfer medium (100) comprising:

    a support (101);
    a release layer (102) provided over the support (101); and

a reagent immobilized layer (103) provided over the release layer (102),
wherein the transfer medium (100) has a structure in which a capture nucleic acid reactive with the target nucleic acid (14) is immobilized by covalent binding to a surface of the reagent immobilized layer (103) and wherein the reagent immobilized layer contains the constituent resin of the shaped body forming the detection portion of the testing device.

**13.** A method for producing the testing device (10) according to any one of claims 1 to 11, the method comprising bringing the reagent immobilized layer (103) of the transfer medium (100) for producing a testing device (10) according to claim 12 and the flow path member (30) into contact with each other to transfer the reagent immobilized layer (103) onto the flow path member (30).

**14.** A testing kit (200) comprising:

the testing device (10) according to any one of claims 1 to 11; and
an analyte collecting unit configured to collect an analyte.

**15.** A testing method comprising:

supplying an analyte to the flow path member (30) of the testing device (10) according to any one of claims 1 to 11; and
capturing a part of the analyte by the capture nucleic acid immobilized to the shaped body.

**Patentansprüche**

**1.** Untersuchungsvorrichtung (10), die Folgendes umfasst:

ein poröses Strömungsbahnelement (30), das eine Strömungsbahn darstellt, durch die eine Untersuchungsziel-Flüssigkeit (12) strömen lassen wird,
einen Untersuchungsziel-Flüssigkeitstropfabschnitt, der an dem Strömungsbahnelement (30) bereitgestellt wird,
einen Kennzeichnungsabschnitt, der dafür konfiguriert ist, eine Kennzeichnung an einer Ziel-Nukleinsäure (14) anzubringen, wenn die Ziel-Nukleinsäure (14) in der Untersuchungsziel-Flüssigkeit (12), die auf den Untersuchungsziel-Flüssigkeitstropfabschnitt getropft wird, enthalten ist, und
einen Erkennungsabschnitt, der dafür konfiguriert ist, die an dem Kennzeichnungsabschnitt gekennzeichnete Ziel-Nukleinsäure (14) zu erkennen,
wobei die Untersuchungsvorrichtung (10) einen geformten Körper umfasst, der auf dem Strömungsbahnelement (30) an dem Erkennungsabschnitt aus einem Harz gebildet ist, und,
wobei eine Einfang-Nukleinsäure, die eine Sequenz einschließt, die mit der Ziel-Nukleinsäure (14) zu binden und komplementär ist, durch kovalente Bindung an eine Oberfläche des geformten Körpers zwischen dem geformten Körper und dem Strömungsbahnelement (30) immobilisiert ist.

**2.** Untersuchungsvorrichtung (10) nach Anspruch 1,
wobei die kovalente Bindung mindestens eines umfasst, das ausgewählt ist aus der Gruppe, die aus Amidbindung, Etherbindung und Thioetherbindung besteht.

**3.** Untersuchungsvorrichtung (10) nach Anspruch 2,
wobei die Einfang-Nukleinsäure einzelsträngig und mit der Ziel-Nukleinsäure (14) hybridisierbar ist.

**4.** Untersuchungsvorrichtung (10) nach Anspruch 3,
wobei die kovalente Bindung durch Reaktion mindestens einer funktionalen Gruppe gebildet ist, die ausgewählt ist aus der Gruppe, die aus einer Aminogruppe, einer Carboxylgruppe, einer Hydroxylgruppe und einer Thiolgruppe auf der Oberfläche des geformten Körpers und in der Einfang-Nukleinsäure besteht.

**5.** Untersuchungsvorrichtung (10) nach Anspruch 1,
wobei die Einfang-Nukleinsäure, die eine Sequenz einschließt, die mit der Ziel-Nukleinsäure (14) zu binden und komplementär ist, durch einen Linker mit dem geformten Körper gebunden wird.

**6.** Untersuchungsvorrichtung (10) nach Anspruch 5,
wobei der geformte Körper eine funktionelle Gruppe umfasst, die Reaktionsvermögen aufweist, und
wobei die Einfang-Nukleinsäure über die funktionelle Gruppe und den Linker mit der Oberfläche des geformten Körpers gebunden ist.

**7.** Untersuchungsvorrichtung (10) nach Anspruch 6,
wobei der geformte Körper eine Aminogruppe als die funktionelle Gruppe umfasst.

**8.** Untersuchungsvorrichtung (10) nach Anspruch 7,
wobei der Linker an einem Ende eine N-Hydroxybernsteinsäure-Imidoester-Gruppe umfasst und durch Amidbindung mit der Aminogruppe an der Oberfläche des geformten Körpers gebunden ist.

**9.** Untersuchungsvorrichtung (10) nach einem der Ansprüche 5 bis 8,
wobei der Linker an einem Ende eine Maleimid-Gruppe umfasst und durch Thioetherbindung mit einer an einem 5'-Ende oder einem 3'-Ende der Einfang-Nukleinsäure eingeführten Thiolgruppe gebunden ist.

**10.** Untersuchungsvorrichtung (10) nach Anspruch 1,
wobei die Einfang-Nukleinsäure, die eine Sequenz, die mit der Ziel-Nukleinsäure (14) zu binden und komplementär ist, und ein Spacer einschließt, an der Oberfläche des geformten Körpers immobilisiert ist.

**11.** Untersuchungsvorrichtung (10) nach Anspruch 10,
wobei der Spacer eine Alkylgruppe oder eine Alkylgruppe und eine Phosphorsäuregruppe umfasst.

**12.** Übertragungsmedium (100) zum Herstellen einer Untersuchungsvorrichtung (10), wobei das Übertragungsmedium (100) zum Herstellen der Untersuchungsvorrichtung (10) nach einem der Ansprüche 1 bis 11 vorgesehen ist, wobei das Übertragungsmedium (100) Folgendes umfasst:

einen Träger (101),
eine Freisetzungsschicht (102), die über dem Träger (101) bereitgestellt wird, und
eine Reagenzien-immobilisierte Schicht (103), die über der Freisetzungsschicht (102) bereitgestellt wird,
wobei das Übertragungsmedium (100) eine Struktur aufweist, in der eine Einfang-Nukleinsäure, die mit der Ziel-Nukleinsäure (14) reaktionsfähig ist, durch kovalente Bindung an eine Oberfläche der Reagenzien-immobilisierten Schicht (103) immobilisiert ist und
wobei die Reagenzien-immobilisierte Schicht den Harzbestandteil des geformten Körpers enthält, der den Erkennungsabschnitt der Untersuchungsvorrichtung bildet.

**13.** Verfahren zum Herstellen der Untersuchungsvorrichtung (10) nach einem der Ansprüche 1 bis 11, wobei das Verfahren Folgendes umfasst:

Bringen der Reagenzien-immobilisierten Schicht (103) des Übertragungsmediums (100) zum Herstellen einer Untersuchungsvorrichtung (10) nach Anspruch 12 und des Strömungsbahnelements (30) in Kontakt miteinander, um die Reagenzien-immobilisierte Schicht (103) auf das Strömungsbahnelement (30) zu übertragen.

**14.** Untersuchungsausstattung (200), die Folgendes umfasst:

die Untersuchungsvorrichtung (10) nach einem der Ansprüche 1 bis 11 und
eine Analytsammeleinheit, die dafür konfiguriert ist, einen Analyten zu sammeln.

**15.** Untersuchungsverfahren, das Folgendes umfasst:

Zuführen eines Analyten zu dem Strömungsbahnelement (30) der Untersuchungsvorrichtung (10) nach einem der Ansprüche 1 bis 11 und
Einfangen eines Teils des Analyten durch die an dem geformten Körper immobilisierte Einfang-Nukleinsäure.

## Revendications

**1.** Dispositif d'essai (10) comprenant :

un élément de voie d'écoulement poreux (30) qui constitue une voie d'écoulement au travers de laquelle un liquide cible d'essai (12) s'écoule ;

une section d'acceuil de gouttes de liquide cible d'essai qui est prévue sur l'élément de voie d'écoulement (30) ;

une section d'étiquetage qui est configurée pour appliquer une étiquette à un acide nucléique cible (14) lorsque l'acide nucléique cible (14) est contenu dans le liquide cible d'essai (12) qui est distribué sur la section de d'acceuil de gouttes liquide cible d'essai ; et

une section de détection qui est configurée pour détecter l'acide nucléique cible (14) qui est étiqueté au niveau de la section d'étiquetage ;

dans lequel le dispositif d'essai (10) comprend un corps conformé qui est formé en une résine sur l'élément de voie d'écoulement (30) au niveau de la section de détection ; et

dans lequel un acide nucléique de capture, qui inclut une séquence qui peut être liée avec l'acide nucléique cible (14) et qui est complémentaire à celui-ci, est immobilisé par une liaison covalente sur une surface du corps conformé entre le corps conformé et l'élément de voie d'écoulement (30).

**2.** Dispositif d'essai (10) selon la revendication 1,
dans lequel la liaison covalente comprend au moins l'une sélectionnée parmi le groupe qui est constitué par une liaison amide, une liaison éther et une liaison thioéther.

**3.** Dispositif d'essai (10) selon la revendication 2,
dans lequel l'acide nucléique de capture est à un seul brin et peut être hybridisé avec l'acide nucléique cible (14).

**4.** Dispositif d'essai (10) selon la revendication 3,
dans lequel la liaison covalente est formée par réaction d'au moins un groupe fonctionnel qui est sélectionné parmi le groupe qui est constitué par un groupe amino, un groupe carboxyle, un groupe hydroxyle et un groupe thiol sur la surface du corps conformé et dans l'acide nucléique de capture.

**5.** Dispositif d'essai (10) selon la revendication 1,
dans lequel l'acide nucléique de capture, qui inclut une séquence qui peut être liée avec l'acide nucléique cible (14) et qui est complémentaire à celui-ci, est lié par un coupleur avec le corps conformé.

**6.** Dispositif d'essai (10) selon la revendication 5,
dans lequel le corps conformé comprend un groupe fonctionnel qui présente une réactivité ; et
dans lequel l'acide nucléique de capture est lié avec la surface du corps conformé via le groupe fonctionnel et le coupleur.

**7.** Dispositif d'essai (10) selon la revendication 6,
dans lequel le corps conformé comprend un groupe amino en tant que groupe fonctionnel.

**8.** Dispositif d'essai (10) selon la revendication 7,
dans lequel le coupleur comprend un groupe ester imide d'acide N-hydroxysuccinique au niveau d'une extrémité et est lié, par une liaison amide, au groupe amino sur la surface du corps conformé.

**9.** Dispositif d'essai (10) selon l'une quelconque des revendications 5 à 8,
dans lequel le coupleur comprend un groupe maléimide au niveau d'une extrémité et est lié, par une liaison thioéther, à un groupe thiol qui est introduit au niveau d'une extrémité 5' ou d'une extrémité 3' de l'acide nucléique de capture.

**10.** Dispositif d'essai (10) selon la revendication 1,
dans lequel l'acide nucléique de capture, qui inclut une séquence qui peut être liée avec l'acide nucléique cible (14) et qui est complémentaire à celui-ci et un espaceur, est immobilisé sur la surface du corps conformé.

**11.** Dispositif d'essai (10) selon la revendication 10,
dans lequel l'espaceur comprend un groupe alkyle, ou un groupe alkyle et un groupe acide phosphorique.

**12.** Milieu de transfert (100) pour produire un dispositif d'essai (10), le milieu de transfert (100) étant destiné à la production du dispositif d'essai (10) selon l'une quelconque des revendications 1 à 11, le milieu de transfert (100) comprenant :

un support (101) ;

une couche de libération (102) qui est prévue au-dessus du support (101) ; et
une couche immobilisée de réactif (103) qui est prévue au-dessus de la couche de libération (102) ;
dans lequel le milieu de transfert (100) présente une structure selon laquelle un acide nucléique de capture qui réagit avec l'acide nucléique cible (14) est immobilisé par une liaison covalente sur une surface de la couche immobilisée de réactif (103) et
dans lequel la couche immobilisée de réactif contient la résine constitutive du corps conformé qui forme la section de détection du dispositif d'essai.

**13.** Procédé pour produire le dispositif d'essai (10) selon l'une quelconque des revendications 1 à 11, le procédé comprenant :
le fait d'amener la couche immobilisée de réactif (103) du milieu de transfert (100) pour produire un dispositif d'essai (10) selon la revendication 12 et l'élément de voie d'écoulement (30) en contact mutuel pour transférer la couche immobilisée de réactif (103) sur l'élément de voie d'écoulement (30).

**14.** Kit d'essai (200) comprenant :

le dispositif d'essai (10) selon l'une quelconque des revendications 1 à 11 ; et
une unité de collecte d'analyte qui est configurée pour collecter un analyte.

**15.** Procédé d'essai comprenant :

la fourniture d'un analyte à l'élément de voie d'écoulement (30) du dispositif d'essai (10) selon l'une quelconque des revendications 1 à 11 ; et
la capture d'une partie de l'analyte par l'acide nucléique de capture qui est immobilisé sur le corps conformé.

[Fig. 1]

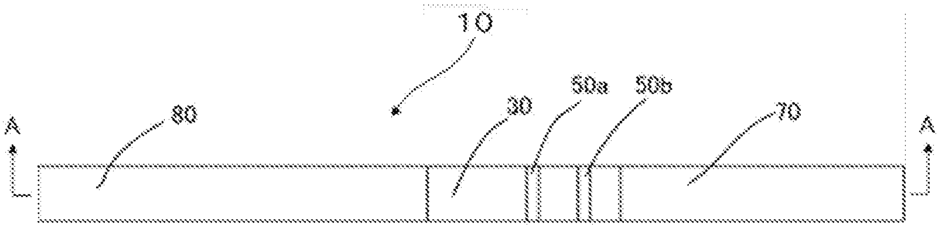


[Fig. 2]

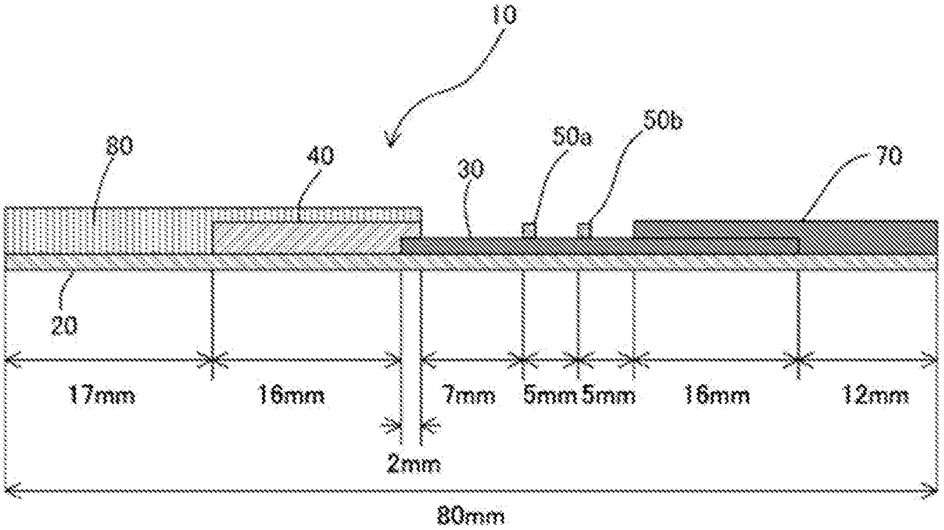


[Fig. 3]

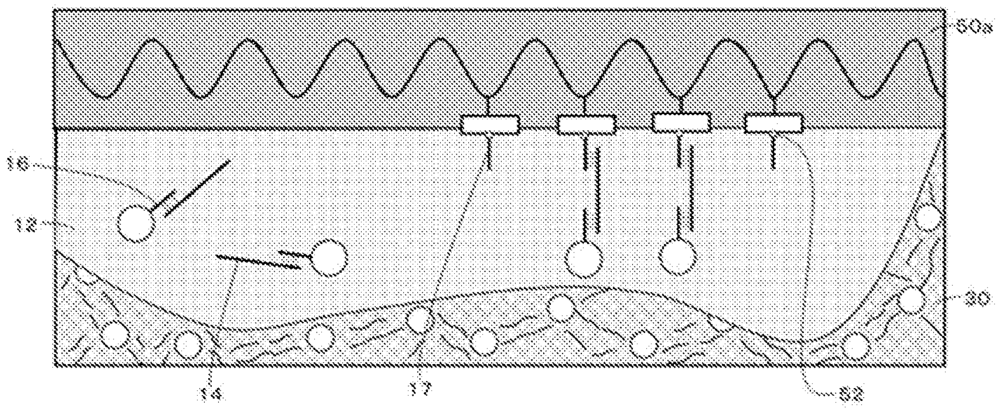


[Fig. 4]

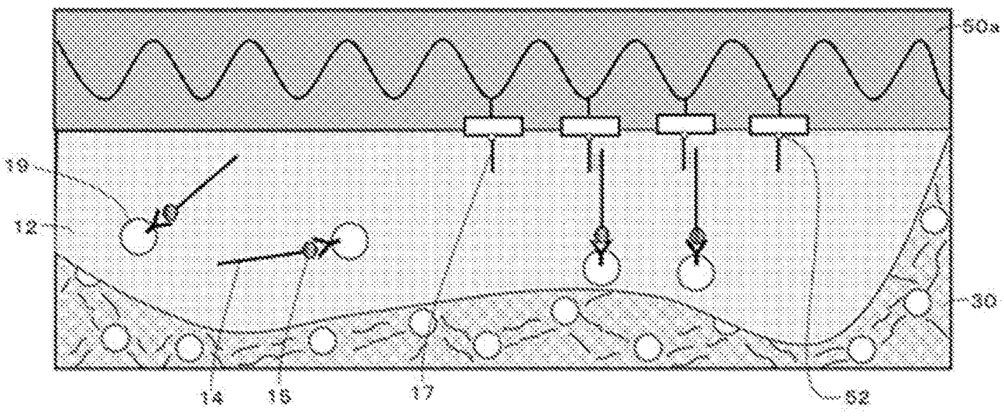

[Fig. 5]

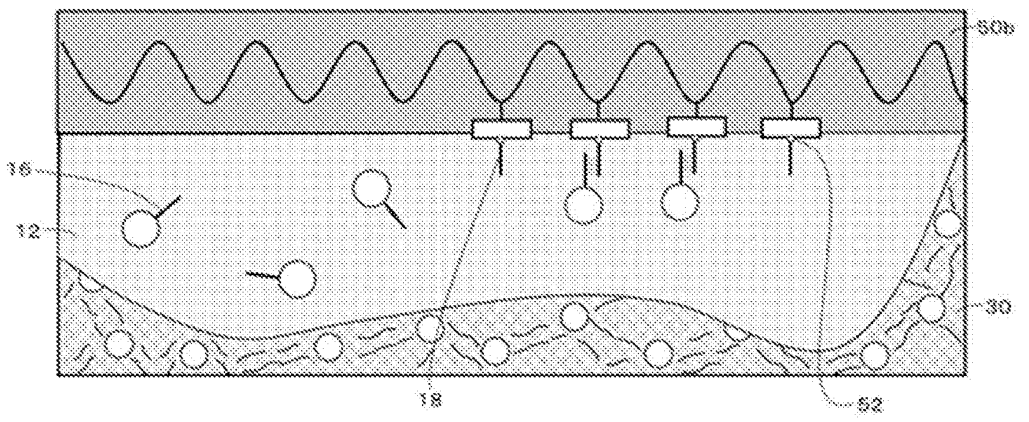


[Fig. 6]

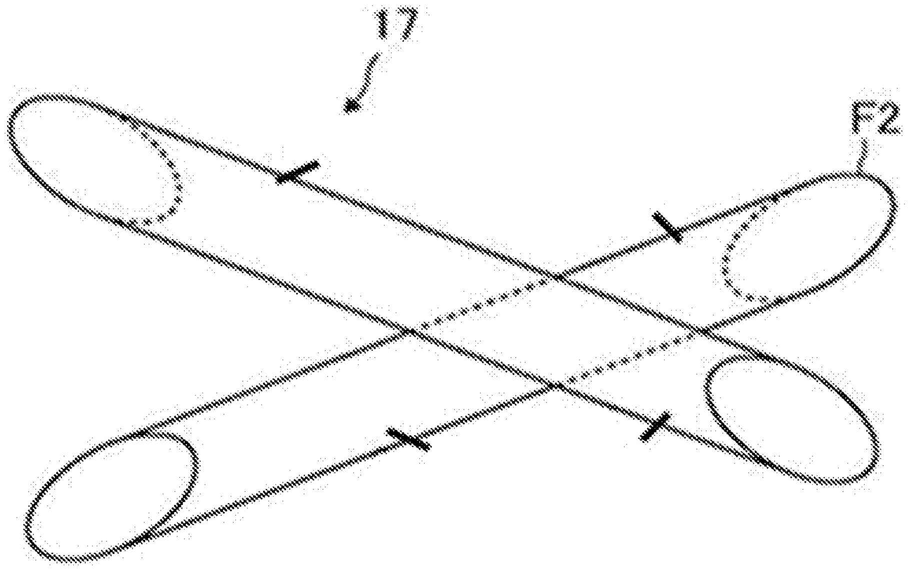


[Fig. 7]

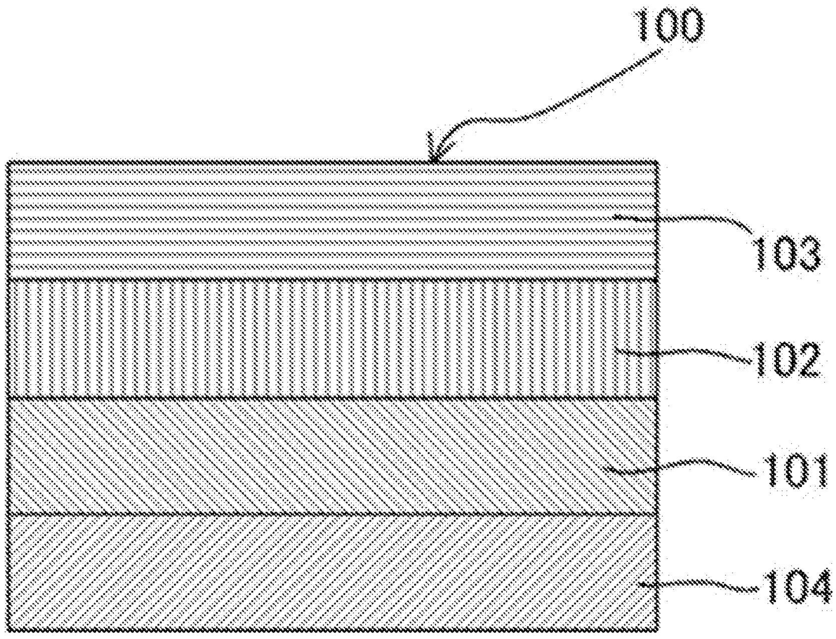

[Fig. 8]

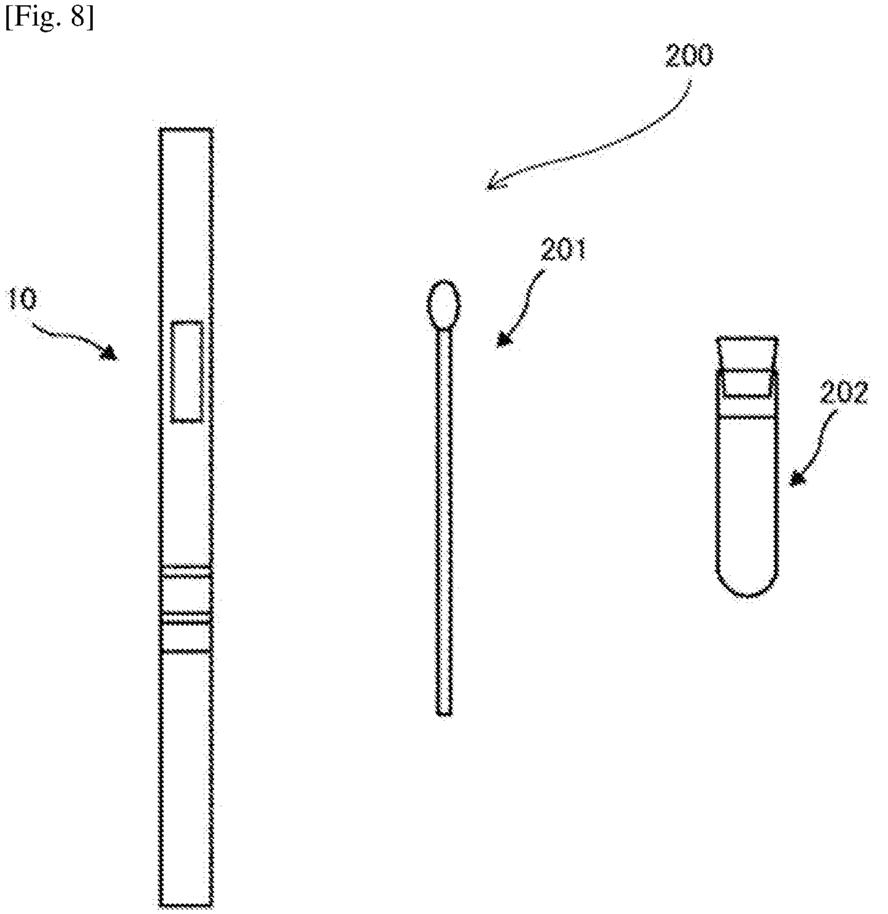


[Fig. 9]

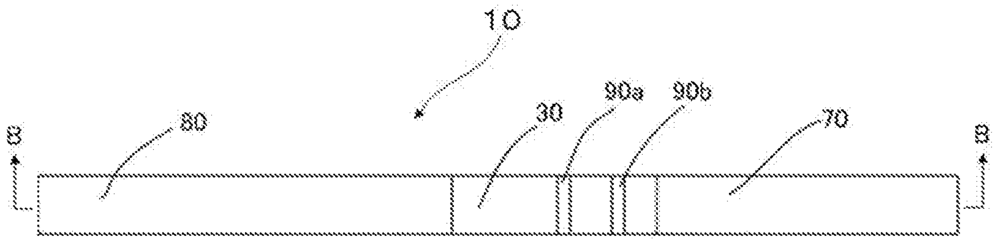


[Fig. 10]

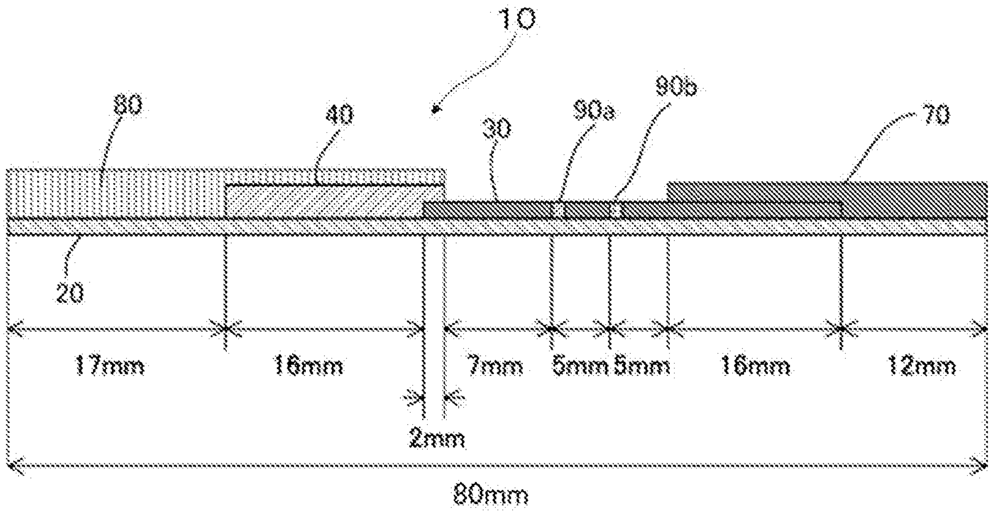

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2001157598 A
- JP T2005503556 PCT
- WO 2015129924 A